# EUROPEAN PATENT APPLICATION

(11) **EP 2 647 626 A1**
(43) Date of publication of application: **09.10.2013**
(21) Application number: 12162982.8
(22) Date of filing: 03.04.2012
(51) Int. Cl.: C07D 209/54, C07D 471/10, A01N 43/38, A01N 43/90

(54) **1-Aza-spiro[4.5]dec-3-ene and 1,8-diaza-spiro[4.5]dec-3-ene derivatives as pesticides**

(71) Applicant: Syngenta Participations AG, 4058 Basel (CH)
(72) Inventor: The designation of the inventor has not yet been filed
(74) Representative: Jantschy, Jasmin

(57) **Abstract**

A compound of the formula I 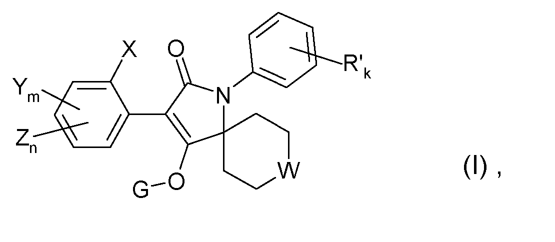 wherein the substituents are as defined in claim 1, are useful as a pesticides.

## Description

The present invention relates to new N-phenyl amide substituted spiroheterocyclic pyrrolidine dione derivatives, to processes for preparing them, to pesticidal, in particular insecticidal, acaricidal, molluscicidal and nematicidal compositions comprising them and to methods of using them to combat and control pests such as insect, acarine, mollusc and nematode pests.

Spiroheterocyclic pyrrolidine dione derivatives are disclosed for example in US 6555567, US 6479489, US 6774133, EP 596298, WO 98/05638 and WO 99/48869. Further, spiroheterocyclic pyrrolidine dione derivatives are known, for example, from WO 09/049851, WO 2010/063670, WO 2010/066780 and WO 11/151199.

It has now surprisingly been found that novel N-phenyl amide substituted spiroheterocyclic pyrrolidine dione derivatives have good insecticidal properties. This is surprising because such a phenyl ring contributes to an overall molecular increase in bulk and lipophilicity, properties which up until now were considered to prevent this class of compounds from having biological activity against pests. The opposite has now been observed.

Such N-phenyl amide substituted spiroheterocyclic pyrrolidine dione derivatives have not been individualised in the prior art.

The present invention therefore provides compounds of the formula I 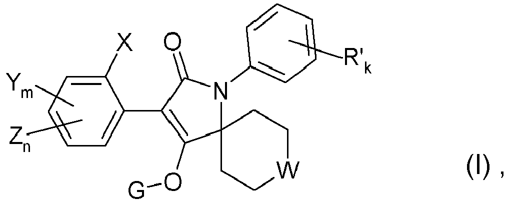 wherein
X , Y and Z independently of each other are C₁₋₄alkyl, C₃₋₆cycloalkyl, C₁₋₄haloalkyl, C₁₋₄alkoxy, C₂₋₆alkenyl, C₂₋₆alkynyl, halogen, phenyl or phenyl substituted by C₁₋₄alkyl, C₁₋₄haloalkyl, halogen or cyano;
m and n, independently of each other, are 0, 1, 2 or 3 and m+n is 0, 1, 2 or 3;
G is hydrogen, a metal, ammonium, sulfonium or a latentiating group;
R' is a C₁₋₄alkyl, C₁₋₄haloalkyl, C₁₋₄alkoxy, halogen or cyano;
k is 0, 1, 2, 3, 4 or 5;
and W is a group selected from W¹ to W¹³_{:} 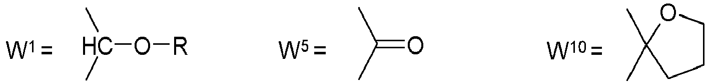 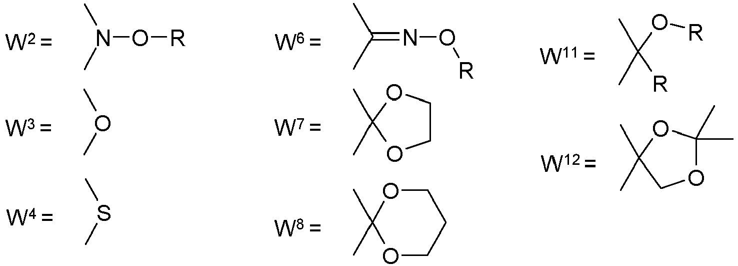 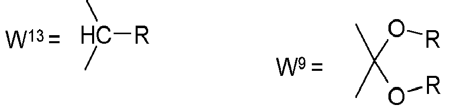 wherein R is hydrogen, C₁₋₆alkyl, C₁₋₆haloalkyl, C₁₋₆cyanoalkyl, C₂₋₆alkenyl, C₂₋₆haloalkenyl, C₃₋₆alkynyl, benzyl, C₁₋₄alkoxy(C₁₋₄)alkyl or C₁₋₄alkoxy(C₁₋₄)alkoxy(C₁₋₄)alkyl; or an agrochemically acceptable salt or an N-oxide thereof.

In the compounds of the formula I, each alkyl moiety either alone or as part of a larger group is a C₁₋₄- or C₁₋₆-straight or branched chain and is, for example, methyl, ethyl, n-propyl, n-butyl, iso-propyl, sec-butyl, iso-butyl, tert-butyl, n-pentyl, iso-pentyl and n-hexyl.

Alkoxy groups have a preferred chain length of from 1 to 4 carbon atoms. Alkoxy is, for example, methoxy, ethoxy, propoxy, i-propoxy, n-butoxy, isobutoxy, sec-butoxy or tert-butoxy. Such groups can be part of a larger group such as alkoxyalkyl and alkoxyalkoxyalkyl. Alkoxyalkyl groups preferably have a chain length of 1 to 4 carbon atoms. Alkoxyalkyl is, for example, methoxymethyl, methoxyethyl, ethoxymethyl, ethoxyethyl, n-propoxymethyl, n-propoxyethyl, isopropoxymethyl. Alkoxyalkoxyalkyl groups preferably have a chain length of 1 to 4 carbon atoms. Alkoxyalkoxyalkyl is, for example, methoxymethoxymethyl, methoxyethoxymethyl, ethoxymethoxyethyl and ethoxyethoxyethyl.

Halogen is generally fluorine, chlorine, bromine or iodine. This also applies, correspondingly, to halogen in combination with other meanings, such as haloalkyl or haloalkenyl.

Haloalkyl groups preferably have a chain length of from 1 to 4 or 1 to 6 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl; preferably trichloromethyl, difluorochloromethyl, difluoromethyl, trifluoromethyl and dichlorofluoromethyl.

The preferred alkenyl and alkynyl radicals having 2 to 6 or 3 to 6 carbon atoms can be straight or branched and can contain more than one double or triple bond respectively. Examples are vinyl, (E)- or (Z)-propenyl, 2-methyl-propenyl, allyl, 3-methyl-but-2-enyl, ethynyl, prop-1-ynyl, propargyl, butenyl, butynyl, pentenyl and pentynyl.

The cycloalkyl groups preferably have from 3 to 6 ring carbon atoms, for example cyclopropyl, cyclobutyl, cyclopentyl and cyclohexyl.

Phenyl, also as part of a substituent such as benzyl, may be substituted, preferably by one or more C₁₋₄alkyl, C₁₋₄haloalkyl, C₁₋₄alkoxy, halogen or cyano groups. The substituents can be in ortho, meta and/or para position.

In the compounds of the formula I, k is preferably 0, 1 or 2. Even more preferred are compounds of the formula I wherein k is 0 or 1. If k =1, 2, 3, 4 or 5, each substituent R' of the phenyl ring can be independently the same or different. In the compounds of formula I, the substituent R' is C₁₋₄alkyl, C₁₋₄haloalkyl, C₁₋₄alkoxy, halogen or cyano. Preferably, R' is selected from methyl, ethyl, n-propyl, iso-propyl, n-butyl, sec-butyl, iso-butyl, tert-butyl, fluoromethyl, difluoromethyl, difluorochloromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, methoxy, ethoxy, propoxy, i-propoxy, n-butoxy, isobutoxy, sec-butoxy and tert-butoxy, fluorine, chlorine, bromine, iodine or cyano. More preferably, R' is selected from methyl, ethyl, n-propyl, iso-propyl, fluoromethyl, difluoromethyl, difluorochloromethyl, trifluoromethyl, chloromethyl, dichloromethyl, trichloromethyl, methoxy, ethoxy, propoxy, i-propoxy, fluorine, chlorine, or cyano. The substituents R' can be in ortho, meta and/or para position.

Preferably, W is a group selected from one of W² to W¹³: 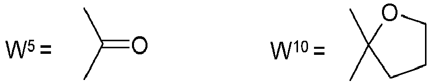 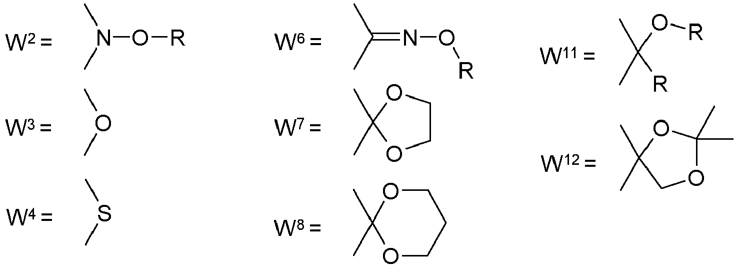 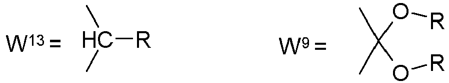 wherein R is defined as above, preferably a hydrogen, methyl, ethyl, n-propyl, iso-propyl, n-butyl, sec-butyl, tert-butyl, trifluoromethyl, allyl, propargyl, benzyl, methoxymethyl, ethoxymethyl or methoxyethyl.

More preferably, W is a group selected from one of W² to W¹²: 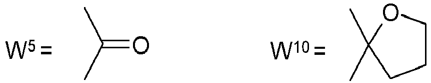 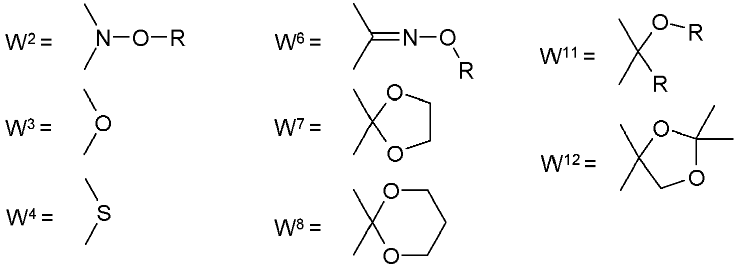 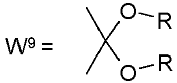 wherein R is defined as above, preferably a hydrogen, methyl, ethyl, n-propyl, iso-propyl, n-butyl, sec-butyl, tert-butyl, trifluoromethyl, allyl, propargyl, benzyl, methoxymethyl, ethoxymethyl or methoxyethyl.

Even more preferably, W is a group selected from one of W² or W⁵ to W¹²: 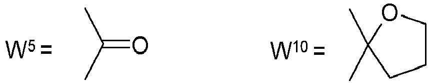 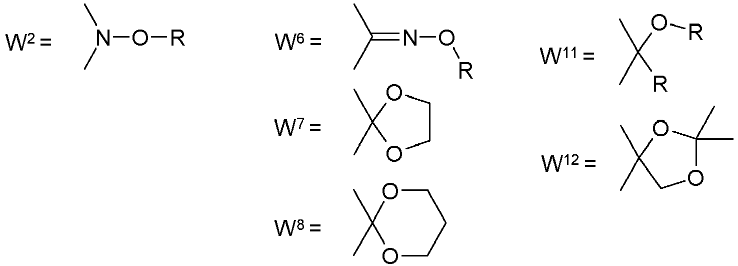 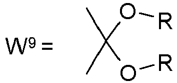 wherein R is defined as above, preferably a hydrogen, methyl, ethyl, n-propyl, iso-propyl, n-butyl, sec-butyl, tert-butyl, trifluoromethyl, allyl, propargyl, benzyl, methoxymethyl, ethoxymethyl or methoxyethyl.

Most preferably, W is a group selected from one of W²: 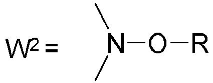 wherein R is defined as above, preferably a hydrogen, methyl, ethyl, n-propyl, iso-propyl, n-butyl, sec-butyl, tert-butyl, trifluoromethyl, allyl, propargyl, benzyl, methoxymethyl, ethoxymethyl or methoxyethyl.

The latentiating groups G (i.e. groups which are metabolizable in a plant; in an insect, acarine, mollusc and/or nematode pest; and/or in the soil) are selected to allow its removal by one or a combination of biochemical, chemical or physical processes to afford compounds of formula I where G is hydrogen before, during or following application to the treated area or plants. Examples of these processes include enzymatic cleavage, chemical hydrolysis and photoloysis. Compounds bearing such groups G may offer certain advantages, such as improved penetration of the cuticula of the plants treated, increased tolerance of crops, improved compatibility or stability in formulated mixtures containing other insecticides, herbicide safeners, plant growth regulators, herbicides or fungicides, or reduced leaching in soils.

The latentiating group G is preferably selected from the groups C₁-C₈alkyl, C₂-C₈haloalkyl, phenylC₁-C₈alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano or by nitro), heteroarylC₁-C₈alkyl (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃ alkylsulfonyl, halogen, cyano or by nitro), C₃-C₈alkenyl, C₃-C₈haloalkenyl, C₃-C₈alkynyl, C(X^{a})-R^{a}, C(X^{b})-X^{c}-R^{b}, C(X^{d})-N(R^{c})-R^{d}, -SO₂-R^{e}, - P(X^{e})(R^{f})-R^{g} and CH₂-X^{f}-R^{h} wherein X^{a}, X^{b}, X^{c}, X^{d}, X^{e} and X^{f} are independently of each other oxygen or sulfur;
R^{a} is H, C₁-C₁₈alkyl, C₂-C₁₈alkenyl, C₂-C₁₈alkynyl, C₁-C₁₀haloalkyl, C₁-C₁₀cyanoalkyl, C₁-C₁₀nitroalkyl, C₁-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynylC₁-C₅oxyalkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₁-C₅alkylaminoC₁-C₅alkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), heteroarylC₁-C₅alkyl, (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), C₂-C₅haloalkenyl, C₃-C₈cycloalkyl; phenyl or phenyl substituted by C₁-C₃alkyl, C₁-C₃haloalkyl, C₁-C₃alkoxy, C₁-C₃haloalkoxy, halogen, cyano or nitro, or heteroaryl or heteroaryl substituted by C₁-C₃ alkyl, C₁-C₃haloalkyl, C₁-C₃alkoxy, C₁-C₃haloalkoxy, halogen, cyano or nitro;
R^{b} is C₁-C₁₈alkyl, C₃-C₁₈alkenyl, C₃-C₁₈alkynyl, C₂-C₁₀haloalkyl, C₁-C₁₀cyanoalkyl, C₁-C₁₀nitroalkyl, C₂-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynyloxyC₁-C₅alkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₁-C₅alkylaminoC₁-C₅alkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), heteroarylC₁-C₅alkyl, (wherein the heteroaryl may optionally be substituted by C₁-C₃alkyl, C₁-C₃haloalkyl, C₁-C₃alkoxy, C₁-C₃haloalkoxy, C₁-C₃alkyl-thio, C₁-C₃alkylsulfinyl, C₁-C₃alkylsulfonyl, halogen, cyano, or by nitro), C₃-C₅haloalkenyl, C₃-C₈cycloalkyl, phenyl or phenyl substituted by C₁-C₃alkyl, C₁-C₃haloalkyl, C₁-C₃alkoxy, C₁-C₃halo-alkoxy, halogen, cyano or nitro; or heteroaryl or heteroaryl substituted by C₁-C₃ alkyl, C₁-₃haloalkyl, C₁-C₃alkoxy, C₁-C₃haloalkoxy, halogen, cyano or nitro; and
R^{c} and R^{d} are each independently of each other hydrogen, C₁-C₁₀alkyl, C₃-C₁₀alkenyl, C₃-C₁₀alkynyl, C₂-C₁₀haloalkyl, C₁-C₁₀cyanoalkyl, C₁-C₁₀nitroalkyl, C₁-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynyloxyC₁-C₅alkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₂-C₅alkylaminoalkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), heteroarylC₁-C₅alkyl, (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), C₂-C₅haloalkenyl, C₃-C₈cycloalkyl; phenyl or phenyl substituted by C₁-C₃alkyl, C₁-C₃haloalkyl, C₁-C₃alkoxy, C₁-C₃haloalkoxy, halogen, cyano or nitro; heteroaryl or heteroaryl substituted by C₁-C₃alkyl, C₁-C₃haloalkyl, C₁-C₃alkoxy, C₁-C₃haloalkoxy, halogen, cyano or nitro; heteroarylamino or heteroarylamino substituted by C₁-C₃alkyl, C₁-C₃haloalkyl, C₁-C₃alkoxy, C₁-C₃haloalkoxy, halogen, cyano or nitro; diheteroarylamino or diheteroarylamino substituted by C₁-C₃alkyl, C₁-C₃haloalkyl, C₁-C₃alkoxy, C₁-C₃haloalkoxy, halogen, cyano or nitro; phenylamino or phenylamino substituted by C₁-C₃alkyl, C₁-C₃haloalkyl, C₁-C₃alkoxy, C₁-C₃haloalkoxy, halogen, cyano or by nitro, diphenylamino or diphenylamino substituted by C₁-C₃alkyl, C₁-C₃haloalkyl, C₁-C₃alkoxy, C₁-C₃haloalkoxy, halogen, cyano or by nitro or C₃-C₇cycloalkylamino, di-C₃-C₇cycloalkylamino or C₃-C₇cycloalkoxy or R^{c} and R^{d} may join together to form a 3-7 membered ring, optionally containing one heteroatom selected from O or S; and
R^{e} is C₁-C₁₀alkyl, C₂-C₁₀alkenyl, C₂-C₁₀alkynyl, C₁-C₁₀haloalkyl, C₁-C₁₀cyanoalkyl, C₁-C₁₀nitroalkyl, C₁-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynyloxyC₁-C₅alkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₁-C₅alkylaminoC₁-C₅alkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), heteroarylC₁-C₅alkyl (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), C₂-C₅haloalkenyl, C₃-C₈cycloalkyl; phenyl or phenyl substituted by C₁-C₃alkyl, C₁-C₃haloalkyl, C₁-C₃alkoxy, C₁-C₃haloalkoxy, halogen, cyano or nitro; heteroaryl or heteroaryl substituted by C₁-C₃ alkyl, C₁-C₃haloalkyl, C₁-C₃alkoxy, C₁-C₃haloalkoxy, halogen, cyano or by nitro; heteroarylamino or heteroarylamino substituted by C₁-C₃ alkyl, C₁-C₃haloalkyl, C₁-C₃alkoxy, C₁-C₃haloalkoxy, halogen, cyano or by nitro; diheteroarylamino or diheteroarylamino substituted by C₁-C₃ alkyl, C₁-C₃haloalkyl, C₁-C₃alkoxy, C₁-C₃haloalkoxy, halogen, cyano or nitro; phenylamino or phenylamino substituted by C₁-C₃alkyl, C₁-C₃haloalkyl, C₁-C₃alkoxy, C₁-C₃haloalkoxy, halogen, cyano or nitro; diphenylamino, or diphenylamino substituted by C₁-C₃alkyl, C₁-C₃haloalkyl, C₁-C₃alkoxy, C₁-C₃haloalkoxy, halogen, cyano or nitro, or C₃-C₇cycloalkylamino, diC₃-C₇cycloalkylamino, C₃-C₇cycloalkoxy, C₁-C₁₀alkoxy, C₁-C₁₀haloalkoxy, C₁-C₅alkylamino or C₂-C₈dialkylamino;
R^{f} and R^{g} are are each independently of each other C₁-C₁₀alkyl, C₂-C₁₀alkenyl, C₂-C₁₀alkynyl, C₁-C₁₀alkoxy, C₁-C₁₀haloalkyl, C₁-C₁₀cyanoalkyl, C₁-C₁₀nitroalkyl, C₁-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynyloxyC₁-C₅alkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₂-C₅alkylaminoalkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), heteroarylC₁-C₅alkyl (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃alkylsulfonyl, halogen, cyano, or by nitro), C₂-C₅haloalkenyl, C₃-C₈cycloalkyl; phenyl or phenyl substituted by C₁-C₃alkyl, C₁-C₃haloalkyl, C₁-C₃alkoxy, C₁-C₃haloalkoxy, halogen, cyano or nitro; heteroaryl or heteroaryl substituted by C₁-C₃ alkyl, C₁-C₃haloalkyl, C₁-C₃alkoxy, C₁-C₃haloalkoxy, halogen, cyano or by nitro, heteroarylamino or heteroarylamino substituted by C₁-C₃ alkyl, C₁-C₃haloalkyl, C₁-C₃alkoxy, C₁-C₃haloalkoxy, halogen, cyano or by nitro; diheteroarylamino or diheteroarylamino substituted by C₁-C₃ alkyl, C₁-C₃haloalkyl, C₁-C₃alkoxy, C₁-C₃haloalkoxy, halogen, cyano or nitro; phenylamino or phenylamino substituted by C₁-C₃alkyl, C₁-C₃haloalkyl, C₁-C₃alkoxy, C₁-C₃haloalkoxy, halogen, cyano or nitro; diphenylamino or diphenylamino substituted by C₁-C₃alkyl, C₁-C₃haloalkyl, C₁-C₃alkoxy, C₁-C₃haloalkoxy, halogen, cyano or nitro, or C₃-C₇cycloalkylamino, diC₃-C₇cycloalkylamino, C₃-C₇cycloalkoxy, C₁-C₁₀haloalkoxy, C₁-C₅alkylamino or C₂-C₈dialkylamino; or benzyloxy or phenoxy, wherein the benzyl and phenyl groups may in turn be substituted by C₁-C₃alkyl, C₁-C₃haloalkyl, C₁-C₃alkoxy, C₁-C₃haloalkoxy, halogen, cyano or nitro; and
R^{h} is C₁-C₁₀alkyl, C₃-C₁₀alkenyl, C₃-C₁₀alkynyl, C₁-C₁₀haloalkyl, C₁-C₁₀cyanoalkyl, C₁-C₁₀nitroalkyl, C₂-C₁₀aminoalkyl, C₁-C₅alkylaminoC₁-C₅alkyl, C₂-C₈dialkylaminoC₁-C₅alkyl, C₃-C₇cycloalkylC₁-C₅alkyl, C₁-C₅alkoxyC₁-C₅alkyl, C₃-C₅alkenyloxyC₁-C₅alkyl, C₃-C₅alkynyloxyC₁-C₅alkyl, C₁-C₅alkylthioC₁-C₅alkyl, C₁-C₅alkylsulfinylC₁-C₅alkyl, C₁-C₅alkylsulfonylC₁-C₅alkyl, C₂-C₈alkylideneaminoxyC₁-C₅alkyl, C₁-C₅alkylcarbonylC₁-C₅alkyl, C₁-C₅alkoxycarbonylC₁-C₅alkyl, aminocarbonylC₁-C₅alkyl, C₁-C₅alkylaminocarbonylC₁-C₅alkyl, C₂-C₈dialkylaminocarbonylC₁-C₅alkyl, C₁-C₅alkylcarbonylaminoC₁-C₅alkyl, *N*-C₁-C₅alkylcarbonyl-*N*-C₁-C₅alkylaminoC₁-C₅alkyl, C₃-C₆trialkylsilylC₁-C₅alkyl, phenylC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃ alkylsulfonyl, halogen, cyano or by nitro), heteroarylC₁-C₅alkyl (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃ alkylsulfonyl, halogen, cyano or by nitro), phenoxyC₁-C₅alkyl (wherein the phenyl may optionally be substituted by C₁-C₃alkyl, C₁-C₃haloalkyl, C₁-C₃alkoxy, C₁-C₃haloalkoxy, C₁-C₃alkylthio, C₁-C₃alkylsulfinyl, C₁-C₃ alkylsulfonyl, halogen, cyano or by nitro), heteroaryloxyC₁-C₅alkyl (wherein the heteroaryl may optionally be substituted by C₁-C₃alkyl, C₁-C₃haloalkyl, C₁-C₃alkoxy, C₁-C₃haloalkoxy, C₁-C₃alkylthio, C₁-C₃akylsulfinyl, C₁-C₃ alkylsulfonyl, halogen, cyano or by nitro), C₃-C₅haloalkenyl, C₃-C₈cycloalkyl, phenyl or phenyl substituted by C₁-C₃alkyl, C₁-C₃haloalkyl, C₁-C₃alkoxy, C₁-C₃haloalkoxy, halogen or by nitro; or heteroaryl, or heteroaryl substituted by C₁-C₃alkyl, C₁-C₃haloalkyl, C₁-C₃alkoxy, C₁-C₃haloalkoxy, halogen, cyano or by nitro.

In particular, the latentiating group G is a group -C(X^{a})-R^{a} or -C(X^{b})-X^{c}-R^{b}, and the meanings of X^{a}, R^{a}, X^{b}, X^{c} and R^{b} are as defined above. Preferably, X^{a}, X^{b} and X^{c} are oxygen and R^{a} and R^{b} are C₁₋₄alkyl.

It is also preferred that G is hydrogen, an alkali metal or alkaline earth metal, or an ammonium or sulfonium group, where hydrogen is especially preferred.

Depending on the nature of the substituents, compounds of formula I may exist in different isomeric forms. When G is hydrogen, for example, compounds of formula I may exist in different tautomeric forms:

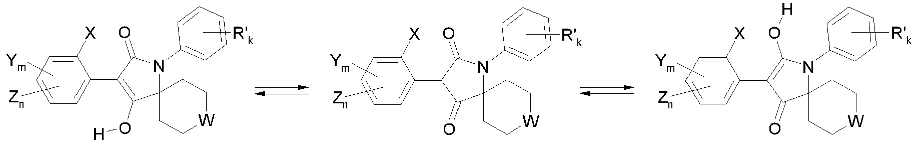

This invention covers all isomers and tautomers and mixtures thereof in all proportions. Also, when substituents contain double bonds, *cis*- and *trans*-isomers can exist. These isomers, too, are within the scope of the claimed compounds of the formula I.

Depending on the nature of the group W, compounds of formula I may exist in different cis-and *trans*-isomeric forms. When W is equal to W¹, for example, compounds of formula I may exist as *trans-* or *cis*-isomers:

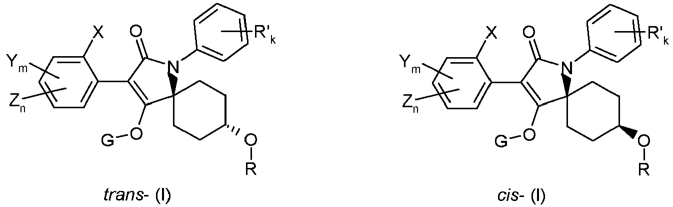

This invention covers all isomers and mixtures thereof in all proportions. Compounds with cis/trans isomers are W¹, W¹⁰, W¹¹, W¹² and W¹³_{.}

The invention relates also to the agriculturally acceptable salts which the compounds of formula I are able to form with transition metal, alkali metal and alkaline earth metal bases, amines, quaternary ammonium bases or tertiary sulfonium bases.

Among the transition metal, alkali metal and alkaline earth metal salt formers, special mention should be made of the hydroxides of copper, iron, lithium, sodium, potassium, magnesium and calcium, and preferably the hydroxides, bicarbonates and carbonates of sodium and potassium.

Examples of amines suitable for ammonium salt formation include ammonia as well as primary, secondary and tertiary C₁-C₁₈alkylamines, C₁-C₄hydroxyalkylamines and C₂-C₄alkoxyalkyl-amines, for example methylamine, ethylamine, *n*-propylamine, *i-*propylamine, the four butylamine isomers, *n*-amylamine, *i*-amylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-*n*-propylamine, di-*i*-propylamine, di-*n*-butylamine, di-*n-*amylamine, di-*i*-amylamine, dihexylamine, diheptylamine, dioctylamine, ethanolamine, *n-*propanolamine, *i*-propanolamine, *N,N*-diethanolamine, *N*-ethylpropanolamine, *N-*butylethanolamine, allylamine, *n*-but-2-enylamine, *n*-pent-2-enylamine, 2,3-dimethylbut-2-enylamine, dibut-2-enylamine, *n*-hex-2-enylamine, propylenediamine, trimethylamine, triethylamine, tri-*n*-propylamine, tri-*i*-opropylamine, tri-*n*-butylamine, tri-*i*-butylamine, tri-*sec-*butylamine, tri-*n*-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, *o*-, *m*- and *p*-toluidines, phenylenediamines, benzidines, naphthylamines and *o*-, *m*- and *p*-chloroanilines; but especially triethylamine, *i*-propylamine and di-*i*-propylamine.

Preferred quaternary ammonium bases suitable for salt formation correspond, for example, to the formula [N(Rₐ₁ R_{b1} R_{c1} R_{d1})]OH, wherein Rₐ₁, R_{b1}, R_{c1} and R_{d1} are each independently of the others hydrogen or C₁-C₄alkyl. Further suitable tetraalkylammonium bases with other anions can be obtained, for example, by anion exchange reactions.

Preferred tertiary sulfonium bases suitable for salt formation correspond, for example, to the formula [SRₑ₁ R_{f1} R_{g1}]OH, wherein Rₑ₁, R_{f1} and R_{g1} are each independently of the others C₁-C₄ alkyl. Trimethylsulfonium hydroxide is especially preferred. Suitable sulfonium bases may be obtained from the reaction of thioethers, in particular dialkylsulfides, with alkylhalides, followed by conversion to a suitable base, for example a hydroxide, by anion exchange reactions.

It should be understood that in those compounds of formula I, where G is a metal, ammonium or sulfonium as mentioned above and as such represents a cation, the corresponding negative charge is largely delocalised across the O-C=C-C=O unit.

The compounds of formula I according to the invention also include hydrates which may be formed during the salt formation.

Preferably, in the compounds of the formula I, the substituent R is hydrogen, C₁-₆alkyl, C₁-₆haloalkyl, C₂-C₆alkenyl, C₃-C₆alkynyl, benzyl or C₁-₄alkoxy(C₁-₄)alkyl, in particular hydrogen, methyl, ethyl, trifluoromethyl, allyl, propargyl, benzyl, methoxymethyl, ethoxymethyl or methoxyethyl.

Preferably, X, Y and Z denote C₁-₄alkyl, C₃₋₆cycloalkyl, C₁-₄alkoxy or halogen, in particular methyl, ethyl, cyclopropyl, methoxy, fluoro, bromo or chloro, when m+n equals 1, 2 or 3, in particular, when m+n equals 1 or 2.

Alternatively, Y and Z, independently of each other, denote C₁-₄alkyl, C₃-₆cycloalkyl, C₁-₄alkoxy, halogen, phenyl or phenyl substituted by C₁-₄alkyl or halogen, in particular methyl, ethyl, cyclopropyl, methoxy, fluoro, chloro, bromo, phenyl or phenyl substituted with halogen, in particular fluoro or chloro, in particular in 4-position, when m+n is 1-3, in particular, when m+n is 1-2.

In another preferred group of compounds of the formula (I), R is hydrogen, methyl, ethyl, trifluoromethyl, allyl, propargyl, benzyl, methoxymethyl, ethoxymethyl or methoxyethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G is hydrogen or ethoxycarbonyl, and W, k and R' have the meanings assigned to them above.

In a particularly preferred group of compounds of the formula (I), R is methyl, ethyl, allyl, propargyl, methoxymethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G is hydrogen or ethoxycarbonyl, and W, k and R' have the meanings assigned to them above.

In a more preferred group of compounds of the formula (I), R is methyl or ethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G is hydrogen or ethoxycarbonyl, and preferably k is equal to 0, 1 or 2. If k is equal to 1 or 2, then each R' is selected from methyl, ethyl, n-propyl, iso-propyl, n-butyl, sec-butyl, iso-butyl, tert-butyl, fluoromethyl, difluoromethyl, difluorochloromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, methoxy, ethoxy, propoxy, i-propoxy, n-butoxy, isobutoxy, sec-butoxy and tert-butoxy, fluorine, chlorine, bromine, iodine or cyano. More preferably, R' is selected from methyl, ethyl, n-propyl, iso-propyl, fluoromethyl, difluoromethyl, difluorochloromethyl, trifluoromethyl, chloromethyl, dichloromethyl, trichloromethyl, methoxy, ethoxy, propoxy, i-propoxy, fluorine, chlorine or cyano.

In an exceptionally preferred group of compounds of the formula (I), R is methyl or ethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G is hydrogen or ethoxycarbonyl, and preferably k is equal to 0 or 1. If k is equal to 1, then R' is selected from methyl, ethyl, n-propyl, iso-propyl, n-butyl, sec-butyl, iso-butyl, tert-butyl, fluoromethyl, difluoromethyl, difluorochloromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, methoxy, ethoxy, propoxy, i-propoxy, n-butoxy, isobutoxy, sec-butoxy and tert-butoxy, fluorine, chlorine, bromine, iodine or cyano. More preferably, R' is selected from methyl, ethyl, n-propyl, iso-propyl, fluoromethyl, difluoromethyl, difluorochloromethyl, trifluoromethyl, chloromethyl, dichloromethyl, trichloromethyl, methoxy, ethoxy, propoxy, i-propoxy, fluorine, chlorine or cyano. Even more preferably, R' is selected from methyl, trifluoromethyl, methoxy, ethoxy, fluorine, chlorine or cyano.

The invention covers also salts of the compounds of the formula I with amines, alkali metal and alkaline earth metal bases or quaternary ammonium bases.

Among the alkali metal and alkaline earth metal hydroxides as salt formers, special mention should be made of the hydroxides of lithium, sodium, potassium, magnesium and calcium, but especially the hydroxides of sodium and potassium. The compounds of formula I according to the invention also include hydrates which may be formed during the salt formation.

Examples of amines suitable for ammonium salt formation include ammonia as well as primary, secondary and tertiary C₁-C₁₈alkylamines, C₁-C₄hydroxyalkylamines and C₂-C₄alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, di-n-butylamine, di-n-amylamine, diisoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-but-2-enylamine, n-pent-2-enylamine, 2,3-dimethylbut-2-enylamine, dibut-2-enylamine, n-hex-2-enylamine, propylenediamine, trimethylamine, triethylamine, tri-n-propylamine, triisopropylamine, tri-n-butylamine, triisobutylamine, tri-sec-butylamine, tri-n-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, o-, m- and p-toluidines, phenylenediamines, benzidines, naphthylamines and o-, m- and p-chloroanilines; but especially triethylamine, isopropylamine and diisopropylamine.

Preferred quaternary ammonium bases suitable for salt formation correspond, for example, to the formula [N(Rₐ₁ R_{b1} R_{c1} R_{d1})]OH wherein Rₐ₁, R_{b1}, R_{c1} and R_{d1} are each independently of the others C₁-C₄alkyl. Further suitable tetraalkylammonium bases with other anions can be obtained, for example, by anion exchange reactions.

The compounds of the invention may be made by a variety of methods. All of the methods discussed below are applicable to any of the compounds according to formula I mentioned above wherein W can be any one of

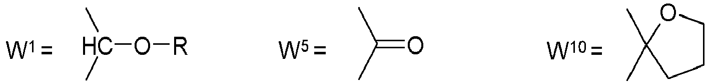

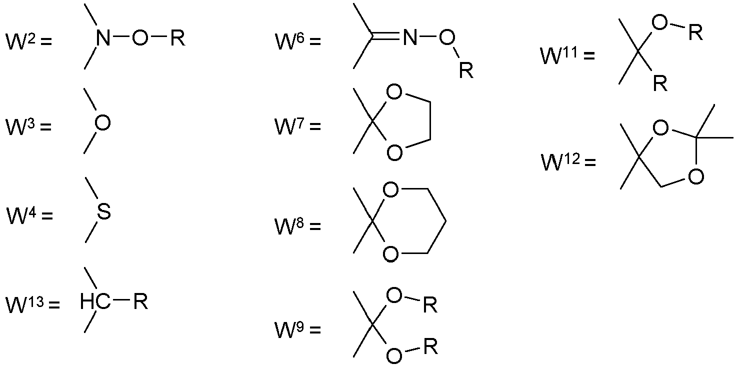

For example, the compounds of formula I, wherein the substituents have the meanings assigned to them above, can be prepared by means of processes known *per se,* e.g. by treating compounds of formula II, or salts thereof, with an alkylating, acylating, phosphorylating or sulfonylating agent G-Q in the presence of at least one equivalent of a base, where G is the alkyl, acyl, phosphoryl or sulfonyl group to be incorporated and Q is a nucleofuge: 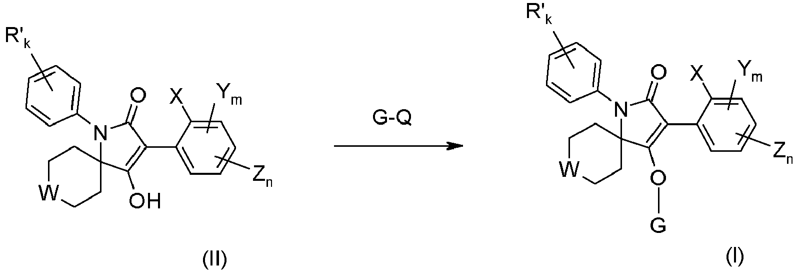 Compounds of formula I, wherein G is a latentiating group of the formula -C(X^{a})-R^{a}, -C(X^{b})-X^{c}-R^{b} or -C(X^{d})-NR^{c}R^{d} may be prepared by procedures known in the art, described for example in WO 09/049851. Typically, compounds of formula II are treated with an acylating agent such as an acid halide (especially acid chloride), acid anhydride, haloformate (especially chloroformate), halothioformate (especially chlorothioformate), isocyanate, isothiocycanate, carbamoyl halide (especially carbamoyl chloride) or thiocarbamoyl halide (especially thiocarbamoyl chloride) in the presence of at least one equivalent of a suitable base, optionally in the presence of a suitable solvent. The base may be inorganic such as an alkali metal carbonate or hydroxide or a metal hydride, or an organic base such as a tertiary amine or metal alkoxide. Examples of suitable inorganic bases include sodium carbonate, sodium or potassium hydroxide, sodium hydride, and suitable organic bases include trialkylamines such as trimethylamine and triethylamine, pyridines or other amine bases such as 1,4-diazobicyclo[2.2.2]octane and 1,8-diazabicyclo[5.4.0]undec-7-ene. Preferred bases include triethylamine and pyridine. Suitable solvents for this reaction are selected to be compatible with the reagents and include ethers such as tetrahydrofuran and 1,2-dimethoxyethane and halogenated solvents such as dichloromethane and chloroform. Certain bases, such as pyridine and triethylamine, may be employed successfully as both base and solvent. For cases, where the acylating agent is a carboxylic acid, acylation is preferably effected in the presence of a coupling agent such as 2-chloro-1-methylpyridinium iodide, N,N'-dicyclohexycarbodiimide, 1-(3-dimethylaminopropyl)-3-ethylcarbodiimide and *N,N'*-carbodiimidazole, and a base such as triethylamine or pyridine in a suitable solvent such as tetrahydrofuran, dichloromethane and acetonitrile.

Compounds of formula I, wherein G is a latentiating group of the formula C(X^{b})-X^{c}-R^{b} or -C(X^{d})-NR^{c}R^{d}, may be also be prepared by treating compounds of formula II with phosgene or a phosgene equivalent, optionally in the presence of a solvent such as toluene or ethyl acetate, and a base and reacting the resultant chloroformate, or equivalent, with an alcohol, thiol or amine under known conditions, as described, for example, in US 6774133, US 6555567 and US 6479489.

Compounds of formula I, wherein G is a latentiating group of the formula -P(X^{e})R^{f}R^{g}, may be prepared from compounds of formula II using procedures described, for example, in US 6774133, US 6555567 and US 6479489.

Compounds of formula I, wherein G is a latentiating group of the formula -SO₂R^{e}, may be prepared by reaction of compounds of formula II with an alkyl or aryl sulfonyl halide, preferably in the presence of at least one equivalent of base.

Compounds of formula I, wherein G is C₁-C₆alkyl, C₂-C₆alkenyl, C₃-C₆alkynyl or a latentiating group of the formula CH₂-X^{f}-R^{h}, may be prepared by treatment of a compound of formula II with a compound of formula G-Y" wherein Y" is a halogen (especially bromine or iodine), sulfonate (especially mesylate or tosylate) or a sulfate preferably in the presence of a base, under known conditions.

Compounds of formula III, or salts thereof, 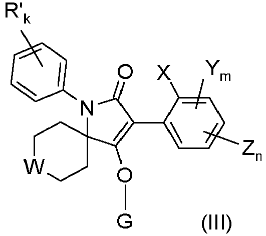 wherein W is selected from one of : 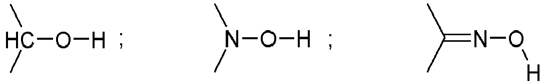 can be obtained by catalytic hydrogenation of compounds of formula I, wherein W is respectively one of: 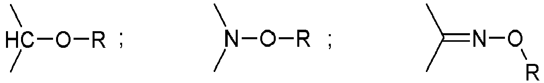 wherein R is represented by a benzyl group.

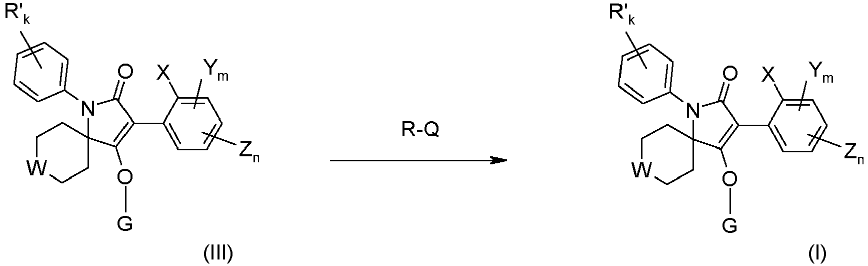

Compounds of formula I can be obtained by treating compounds of formula III, wherein W is selected from one of : 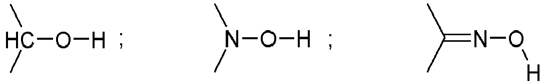 with an alkylating agent R-Q, wherein R represents the alkyl group to be incorporated and Q represents a nucleofuge, in the presence of at least one equivalent of a base, and optionally in the presence of a suitable solvent.

Compounds of formula II may be prepared via the cyclisation of compounds of formula IV, 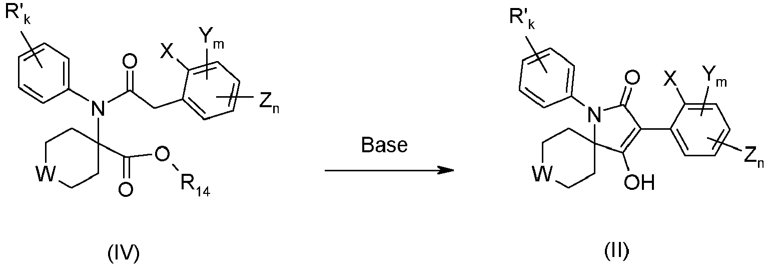 or salts thereof, wherein R₁₄ is C₁-₆alkyl, preferably in the presence of base, and optionally in the presence of a suitable solvent, in analogy to known methods described, for example, in WO 09/049851. Examples of suitable bases include metal hydrides or metal alkoxides, such as sodium hydride, potassium hydride, sodium methoxide, sodium ethoxide, sodium or potassium tert-butoxide. Examples of suitable solvents include for example toluene, xylene, ethers such as tetrahydrofuran or dioxane, or *N,N*-dimethylformamide, or *N*,*N*-dimethylacetamide.

Compounds of formula IV, wherein R₁₄ are as defined above, may be prepared by treatment of a compound of formula XVI, in which W, X, Y, Z, m, n, R and R₁₄ are as defined above, with an appropriate phenylating agent of the formula A-Y^{III}, A-B(OH)₂, BiA₃, A-Y^{IV}, or A-I⁺-A X'⁻, under amide N-arylation reaction conditions (methods 1-5, which are described below), 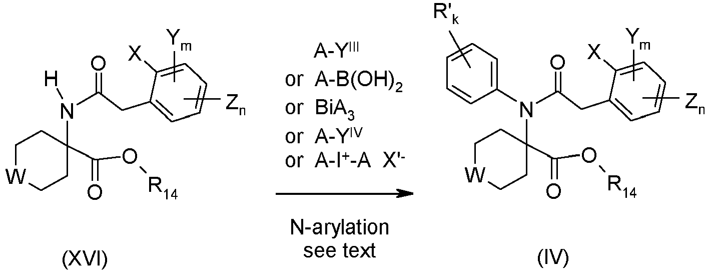 wherein A in the phenylating agent is the fragment: 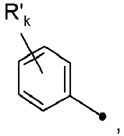 and in which R' and k are as defined above. Compounds of formula XVI are known and have been described, for example, in WO 09/049851.
N-arylation method 1: Copper-mediated Goldberg-type (Goldberg modified Ullmann condensation) reaction conditions carried out with an aryl halide substrate. Hence, compounds of formula IV may be prepared by treatment of a compound of formula XVI with an optionally substituted aryl halide of the formula A-Y^{III}, in which A is as defined above and wherein Y^{III} is a halogen (preferably iodo, bromo or chloro) group, mediated by metalic copper or copper salts, preferably copper(I) chloride, copper(I) bromide, copper(I) iodide, copper(I) oxide or copper(II) acetate, in the presence of a base such as potassium carbonate, potassium phosphate, triethylamine or pyridine, with optional additives such as diamine ligands (preferably 1,10-phenanthroline, 1,2-ethanediamine, *N,N*'-dimethyl-1,2-ethanediamine, *N,N*-dimethyl-1,2-ethanediamine, 1,2-cyclohexanediamine or *N,N*'-dimethyl-1,2-cyclohexane-diamine) or 8-quinolinol, glycine, dimethylglycine or proline, with optional use of microwave heating, in solvents such as nitrobenzene, dioxane, acetonitrile, toluene, dimethylsulfoxide, N-methylpyrrolidone, *N,N*-dimethylformamide or *N,N*-dimethylacetamide. A summary on the scope of this N-arylation method (including details, further variations and further literature about the reaction conditions) may be found, for example, in L. Kürti, B. Czakó, 'Strategic Applications of Named Reactions in Organic Synthesis', Elsevier Academic Press, 2005, pp. 464-465 and 697-698.
N-arylation method 2: Another copper-mediated or -catalyzed N-arylation method involves aryl boronic acids, according to the Chan-Lam protocol (D.M.T. Chan et al., Tetrahedron Letters (1998), 39, 2933-2936; P.Y.S. Lam et al., Tetrahedron Letters (1998), 39, 2941-2944). Accordingly, compounds of formula IV may be prepared by treatment of a compound of formula XVI with an optionally substituted arylboronic acid A-B(OH)₂, in which A is as defined above, mediated by copper salts, preferably copper(II) acetate, in the presence of a tertiary amine promoter such as triethylamine or pyridine (in some cases, mixed-base systems; see, for example, W.W.K.R. Mederski, Tetrahedron (1999), 55, 12757-12770) and with optional use of molecular sieves in solvents such as dichloromethane, 1,2-dichloroethane, chloroform, tetrahydrofuran, dioxane, acetonitrile, *N,N*-dimethylformamide or *N,N*-dimethylacetamide. Catalytic versions in copper of this C-N cross-coupling reaction can be utilized, with molecular oxygen as the oxidant, or alternatively with pyridine N-oxide, TEMPO (2,2,6,6-tetramethyl-1-piperidinyloxy), or triox (trimethylamine-N-oxide), as described, for example, by P.Y.S. Lam et al., Tetrahedron Letters (2001), 42, 3415-3418. Alternative boron-based aryl donors may include triarylboroxines, arylboronic acid esters derived from 2,2'-dimethyl-1,3-propanediol, and 1,3-propanediol and potassium aryltrifluoroborate salts. A summary on the scope of this N-arylation method with boron-based reagents (including details, further variations and further literature about the reaction conditions) may be found, for example, in A.W. Thomas, S.V. Ley, in Modern Arylation Methods, L. Ackermann Ed., Wiley-VCH, Weinheim, 2009, pp.121-154.
N-arylation method 3: A copper-mediated arylation of the amide N-H with a triarylbismuth reagent in the presence of an amine promoter constitutes a further option for this transformation. Compounds of formula IV may be prepared by treatment of a compound of formula XVI with a triarylbismuthane of the formula BiA₃, in which A is as defined above, mediated by copper salts (both the anhydrous and hydrate form), preferably copper(II) salts such as copper(II) acetate, copper(II) bromide, copper(II) chloride or copper(II) nitrate, in the presence of a tertiary amine promoter such as triethylamine or pyridine in solvents such as dichloromethane, 1,2-dichloroethane, chloroform, tetrahydrofuran, dioxane, acetonitrile, *N,N*-dimethylformamide or *N,N*-dimethylacetamide, as described, for example, by D.M.T. Chan, Tetrahedron Letters (1996), 37, 9013-9016.
N-arylation method 4: The Buchwald-Hartwig amination describing the C-N bond formation between aryl halides or aryl trifluoromethanesulfonates and amines in the presence of stoichiometric amount of base also includes the specific N-arylation of amides using palladium-mediated protocols. Accordingly, compounds of formula IV may be prepared by treatment of a compound of formula XVI with an optionally substituted aryl halide or aryl trifluoromethanesulfonate of the formula A-Y^{IV}, in which A is as defined above and wherein Y^{IV} is a halogen (preferably iodo, bromo or chloro) or a triflate group, under palladium catalysis, utilizing palladium(0) precursors such as Pd₂(dba)₃, Pd(dba)₂ (dba=dibenzyliden-acetone) or Pd(PPh₃)₄, or palladium(II) precursors such as typically palladium(II) acetate, with chelating phosphine type ligands such as BINAP (1,1'-[1,1'-binaphthalene]-2,2'-diylbis[1,1-diphenyl- phosphine]), XantPhos (1,1'-(9,9-dimethyl-9H-xanthene-4,5-diyl)bis[1,1-diphenyl- phosphine]), S-Phos (dicyclohexyl(2',6'-dimethoxy[1,1'-biphenyl]-2-yl)- phosphine), X-Phos (dicyclohexyl[2',4',6'-tris(1-methylethyl)[1,1'-biphenyl]-2-yl]-phosphine) or P(o-Tol)₃ (tris(ortho-tolyl)phosphine), in the presence of a base such as cesium carbonate, potassium carbonate, sodium tert-butoxide or lithium hexamethyldisilazane, with optional use of microwave heating, in solvents such as tetrahydrofuran, dioxane, toluene, *N,N-*dimethylformamide or *N,N*-dimethylacetamide. A summary on the scope of this N-arylation method (including details, further variations and further literature about the reaction conditions) may be found, for example, in B. Schlummer, U. Scholz, in Modern Arylation Methods, L. Ackermann Ed., Wiley-VCH, Weinheim, 2009, pp.69-120; or in L. Kürti, B. Czakó, 'Strategic Applications of Named Reactions in Organic Synthesis', Elsevier Academic Press, 2005, pp. 70-71 and 556.
N-arylation method 5: yet another method suitable for arylating amides makes use of copper and hypervalent diaryliodonium salts. Hence, compounds of formula IV may be prepared by treatment of a compound of formula XVI with an optionally substituted diaryliodonium salt of the formula A-I⁺-A X'⁻ (diphenyliodonium tetrafluoroborate is a typical example), in which A is as defined above and wherein the couteranion X'⁻ is, for example, chloride (Cl⁻), tetrafluoroborate (BF₄⁻), p-toluenesulfonate (CH₃C₆H₄SO₂O⁻) or triflate (trifluoromethanesulfonate, CF₃SO₂O⁻), mediated or catalyzed by copper salts, preferably copper(I) iodide, copper(II) acetate or copper(II) acetylacetonate (Cu[acac]₂), in the presence of a base such as potassium or sodium carbonate, or potassium or sodium acetate, in solvents such as dichloromethane, 1,2-dichloroethane, chloroform, toluene, *N,N-*dimethylformamide or *N,N*-dimethylacetamide, as described, for example, by S.K. Kang et al., Synlett (2000), 1022-1024. A summary on the synthesis and applications of these diaryl-λ³-iodanes (diaryliodonium salts) may be found, for example, in E.A. Merritt, B. Olofsson, Angew. Chem. Int. Ed. (2009), 48, 9052-9070.

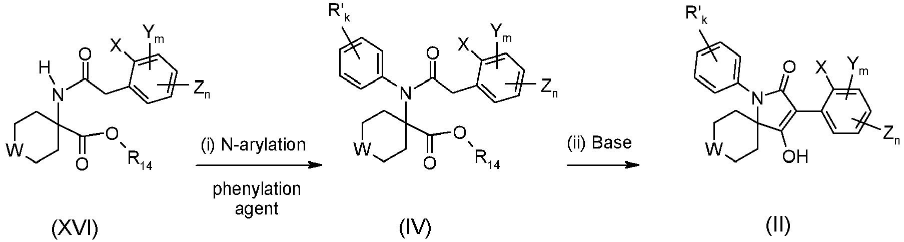

Compounds of formula II may also be prepared in a two-step one-pot process involving:
(i) amide N-phenylation of compounds of formula XVI with an appropriate phenylating agent using either one of the five N-arylation methods described above, in the presence of at least one equivalent of a base, and
(ii) cyclisation of the intermediate compounds of formula IV, preferably in the presence of additional base, at least one more equivalent, and optionally in the presence of a suitable solvent, by methods described above.

R₁₄ is preferably C₁₋₆alkyl. The base for steps (i) and (ii) may be the same or different.

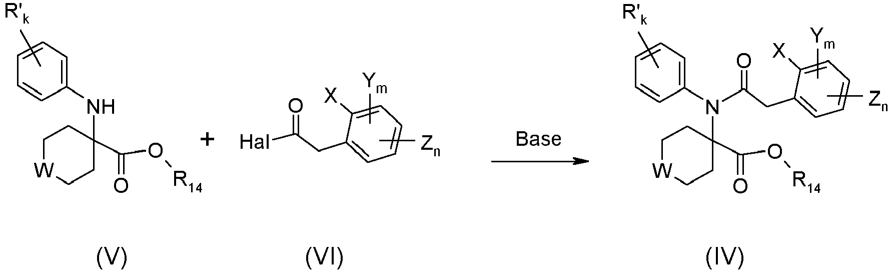

Compounds of formula IV may also be prepared by reacting N-substituted amino acid derivatives of formula V with phenylacetyl halides of formula VI, wherein X, Y, Z, m and n are as defined above and wherein Hal is a halogen (preferably chloro, fluoro or bromo; even more preferably chloro), preferably in the presence of a base in a suitable solvent, by known methods in analogy to those described, for example, in WO 09/049851. The base may be inorganic such as an alkali metal carbonate or hydroxide or a metal hydride, or an organic base such as a tertiary amine or metal alkoxide. Examples of suitable inorganic bases include sodium carbonate, sodium or potassium hydroxide or sodium hydride, and suitable organic bases include trialkylamines such as trimethylamine and triethylamine, pyridines or other amine bases such as 1,4-diazobicyclo[2.2.2]octane and 1,8-diazabicyclo[5.4.0]undec-7-ene. Preferred bases include triethylamine and pyridine. Suitable solvents for this reaction are selected to be compatible with the reagents and include ethers such as tetrahydrofuran and 1,2-dimethoxyethane and halogenated solvents such as dichloromethane and chloroform. Certain bases, such as pyridine and triethylamine, may be employed successfully as both base and solvent.

Phenylacetyl halides of formula VI, wherein Hal is chloro, fluoro or bromo, preferably chloro, are known compounds or can be prepared by known methods, described for example in WO 09/049851.

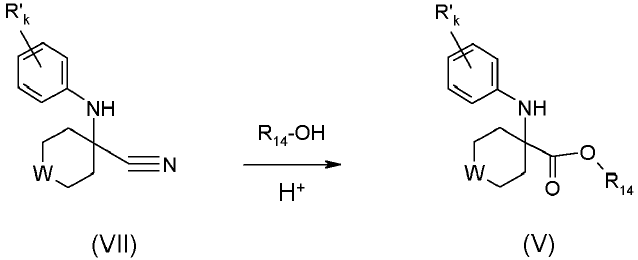

N-arylated amino acid esters of the formula V, wherein R₁₄ is C₁₋₆alkyl, may be prepared by reacting N-arylated amino nitriles of the formula VII with an alcohol of the formula R₁₄OH, preferably in the presence of a strong acid (especially sulfuric acid or hydrochloric acid), under known conditions, described for example in WO 09/049851.

N-substituted amino acid esters of the formula V, wherein R₁₄ is C₁₋₆alkyl, can also be prepared by known methods from N-substituted amino acids of formula VIII. Esterification of VIII with an alcohol of the formula R₁₄OH under thionyl chloride activation is a typical example for the preparation of esters V, as described for example in WO09/049851, but other known esterification methods may also be applied, like for example treatment of a compound of the formula VIII with an alcohol of the formula R₁₄OH under acidic conditions (typically H₂SO₄, HCl or p-toluenesulfonic acid) or under azeotrope water removal conditions (refluxing of VIII with R₁₄OH in toluene while collecting the azeotrope via a Dean-Stark trap: see, for example, J.A. Colapret et al., J. Med. Chem. (1989), 32, 968-974). For the particular situation where R₁₄ is methyl, a compound of the formula VIII may also be treated with diazomethane or trimethylsilyldiazomethane, or with acetyl chloride in methanol. The compounds VIII, VII and V can be reacted and/or isolated as free amines or amine salts (eg. a hydrohalide salt, more specifically a hydrochloride or hydrobromide salt, or any other equivalent salt).

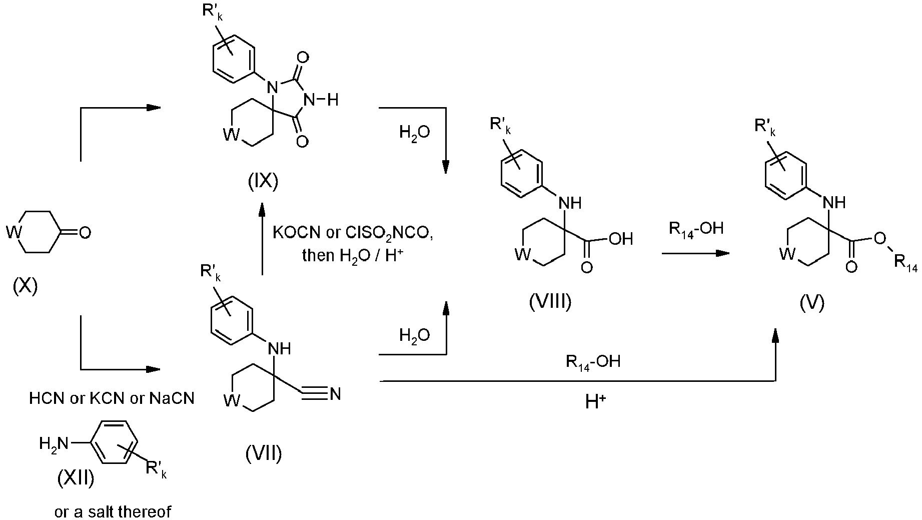

N-substituted amino acids of formula VIII can themselves be prepared by known methods, typically under hydrolysis conditions usually either acidic or basic, from N-substituted amino nitriles of the formula VII. A representative example for the nitrile hydrolysis into its corresponding carboxylic acid functionality under, optionally aqueous, H₂SO₄ and/or HCl conditions may be found, for example, in P.G.H. Van Daele et al., Arzneimittel-Forschung (1976), 26, 1521-1531. Another nitrile VII to carboxylic acid VIII hydrolysis example, with isolation (after treatment with concentrated H₂SO₄) and further hydrolysis (eg. KOH in 1,2-propylene glycol) of the corresponding intermediate carboxamide, may be found, for example, in V.D. Kiricojevic et al., Journal of the Serbian Chemical Society (2002), 67, 793-802.

Ultimately, N-arylated amino acids of formula VIII can also be prepared from ketones of formula X, wherein W and R are as defined above, by means of a Strecker-type synthesis via N-arylated amino nitriles of formula VII. The transformation ketone X to N-arylated amino nitriles VII (Strecker reaction) is a well described one-pot three components coupling involving, besides ketones X, hydrogen cyanide HCN or various alkali cyanides (eg. KCN, NaCN, etc.) in buffered aqueous media or trimethylsilyl cyanide TMS-CN, optionally in presence of a catalytic amount of a Lewis acid, for example Znl₂, and an N-arylated amine (that is an aniline) of the formula XII, 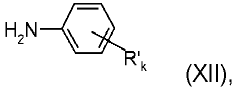 wherein R' and k are as defined above, either as free amine or amine salt (eg. a hydrohalide salt, more specifically a hydrochloride or hydrobromide salt, or any other equivalent salt of the aniline). An appropriate source of cyanide (eg. HCN, KCN or NaCN) may also be added to a preformed ketimine (or iminium salt) between ketone X and the aniline XII, or a salt thereof (see, for example, Synthesis (1992), (7), 664-666). A summary on the scope of the Strecker reaction may be found, for example, in L. Kürti, B. Czakó, 'Strategic Applications of Named Reactions in Organic Synthesis', Elsevier Academic Press, 2005, pp. 446-447 and 690-691. The transformation ketone X to the N-arylated amino nitriles VII may also typically involve an aniline of the formula XII (either as free amine or amine salt), wherein R' and k are as defined above, and an alkali cyanide (such as KCN or NaCN), or trimethylsilyl cyanide TMS-CN, in acetic acid, optionally in the presence of a co-solvent such as methanol, ethanol, isopropanol, water, dichloromethane or acetonitrile, in analogy to, for example, P.G.H. Van Daele et al., Arzneimittel-Forschung (1976), 26, 1521-1531, or P.L. Feldman et al., J. Org. Chem. (1990), 55, 4207-4209, or V.D. Kiricojevic et al., Journal of the Serbian Chemical Society (2002), 67, 793-802, or J.L. Marco et al., Tetrahedron (1999), 55, 7625-7644.

Alternatively, amino acids of formula VIII can also be prepared from ketones of formula X by means of a Bucherer Bergs reaction, described for example in Th. Wieland et al., Methoden Org. Chem. (Houben-Weyl) (1959), Bd. XI/2, 305-306, via N1-substituted hydantoins of formula IX. The transformation ketone X into hydantoin IX can be achieved, for example, in analogy to L. Tang et al., Heterocycles (2007), 74, 999-1008. N-1 arylated hydantoins IX may also be conveniently prepared from N-arylated amino nitriles VII by treatment with an alkali cyanate (eg. potassium cyanate KOCN), followed by acidic aqueous hydrolysis in analogy to, for example, G.M. Carrera et al., J. Heterocyclic Chem. (1992), 29, 847-850 or I.M. Bell et al., Bioorg. Med. Chem. Lett. (2006), 16, 6165-6169. Yet another option for the cyclisation of N-arylated amino nitriles VII into spirohydantoins IX is the reaction with chlorosulfonyl isocyanate ClSO₂NCO in, for example, dichloromethane, followed by acidic aqueous hydrolysis in analogy to, for example, P.L. Feldman et al., J. Org. Chem. (1990), 55, 4207-4209 or M.W. Rowbottom, Bioorg. Med. Chem. Lett. (2007), 17, 2171-2178. N-arylated amino acids of formula VIII can be prepared by known methods, typically under thermal hydrolysis conditions usually either acidic or basic, from N1-arylated hydantoins IX. A representative example for the hydantoin hydrolysis into the corresponding amino acid functionality under aqueous NaOH conditions may be found, for example, in P.L. Feldman et al., J. Org. Chem. (1990), 55, 4207-4209.

The compounds VIII, VII and V can be reacted and/or isolated as free amines or amine salts (eg a hydrohalide salt, more specifically a hydrochloride or hydrobromide salt, or any other equivalent salt).

Compounds of formula X, wherein W is >NOR (W=W²) and R is hydrogen, C₁₋₆alkyl, C₁₋₆haloalkyl, C₁₋₆cyanoalkyl, C₂₋₆alkenyl, C₂₋₆haloalkenyl, C₃₋₆alkynyl, benzyl, C₁₋₄alkoxy(C₁₋₄)alkyl or C₁₋₄alkoxy(C₁₋₄)alkoxy(C₁₋₄)alkyl, are known or can be obtained, for example, according to Major and Dursch, Journal of Organic Chemistry (1961), 26, 1867-74.

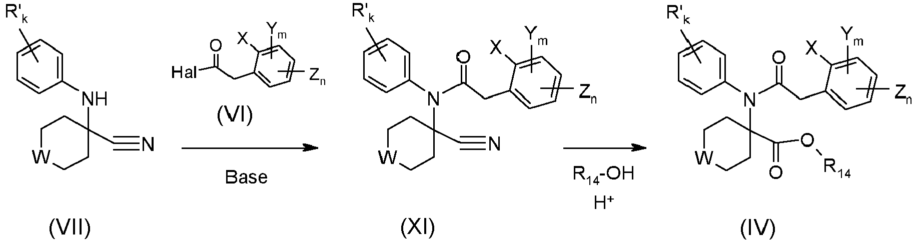

Alternatively, compounds of formula IV may be prepared by subjecting derivatives of formula XI to alcoholysis with R₁₄OH, preferably in strong acidic media (especially sulfuric acid or hydrochloric acid) by known methods in analogy to those described, for example, in WO 09/049851.

Compounds of formula XI may be themselves prepared by reacting N-arylated amino nitriles of formula VII with phenylacetyl halides of formula VI, wherein X, Y, Z, m and n are as defined above and wherein Hal is a halogen (preferably chloro, fluoro or bromo; even more preferably chloro), preferably in the presence of base in a suitable solvent, by known methods in analogy to those described, for example, in WO 09/049851. The base may be inorganic such as an alkali metal carbonate or hydroxide or a metal hydride, or an organic base such as a tertiary amine or metal alkoxide. Examples of suitable inorganic bases include sodium carbonate, sodium or potassium hydroxide, sodium hydride, and suitable organic bases include trialkylamines such as trimethylamine and triethylamine, pyridines or other amine bases such as 1,4-diazobicyclo[2.2.2]octane and 1,8-diazabicyclo[5.4.0]undec-7-ene. Preferred bases include triethylamine and pyridine. Suitable solvents for this reaction are selected to be compatible with the reagents and include ethers such as tetrahydrofuran and 1,2-dimethoxyethane and halogenated solvents such as dichloromethane and chloroform. Certain bases, such as pyridine and triethylamine, may be employed successfully as both base and solvent.

Compounds of formula XI may also be prepared by treatment of a compound of formula XVII with an appropriate N-phenylating agent of the formula A-Y^{III}, A-B(OH)₂, BiA₃, A-Y^{IV}, or A-I⁺-A X'⁻, wherein A is the aryl group to be incorporated and is the fragment of the formula 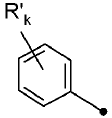 and in which R' and k are as defined above, according to either one of the five N-arylation methods described above.

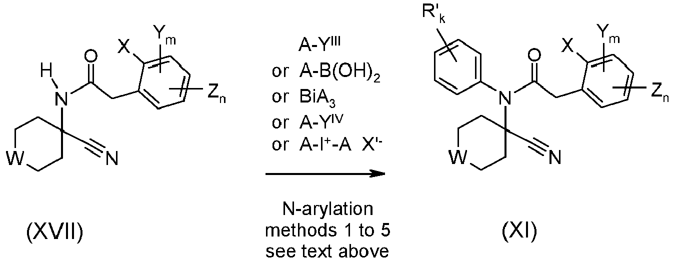

Suitable conditions for this step are the same as described above for the conversion of compounds of formula XVI to compounds of formula IV. Compounds of formula XVII are known and have been described, for example, in WO 09/049851.

Compounds of formula I may also be prepared by treatment of a compound of formula XV, wherein W, X, Y, Z, m, n and G are as defined above, with an appropriate N-phenylating agent of the formula A-Y^{III}, A-B(OH)₂, BiA₃, A-Y^{IV}, or A-I⁺-A X'⁻, wherein A is the aryl group to be incorporated and is the fragment of the formula 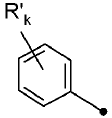 and in which R' and k are as defined above, using either one of the five N-arylation methods described above.

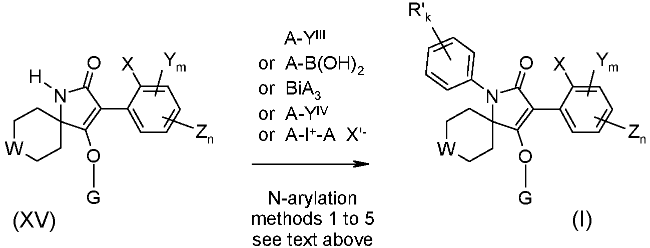

Suitable conditions for this step are the same as described above for the conversion of compounds of formula XVI to compounds of formula IV. Compounds of formula XV are known and have been described, for example, in WO 09/049851.

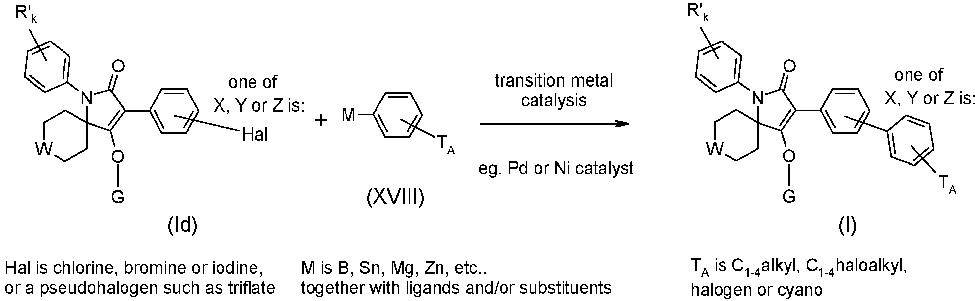

Compounds of the formula I, wherein X, Y or Z is phenyl or phenyl substituted by C₁₋₄alkyl, C₁₋₄haloalkyl, halogen or cyano, may be prepared by reacting a corresponding halogen precursor of the formula Id, wherein Hal is chlorine, bromine, iodine or a pseudohalogen such as C₁₋₄haloalkylsulfonate, especially triflate, with an appropriate organometallic phenyl species of the formula XVIII, wherein T_{A} is C₁₋₄alkyl, C₁₋₄haloalkyl, halogen or cyano and M is for example B, Sn, Si, Mg or Zn holding further ligands and/or substituents, by means of a transition metal-catalyzed reaction. The organometallic species of the formula XVIII is for example an aryl boronic acid T_{A}-Phenyl-B(OH)₂, or a suitable salt or ester thereof, which will react with a compound of the formula Id under palladium- or nickel-catalyzed conditions, such as for example the Suzuki-Miyaura conditions. A variety of metals, catalysts and ligands may be used in this reaction type. Reaction conditions and catalytic systems for such a transformation have been described, for example, in WO08/071405.

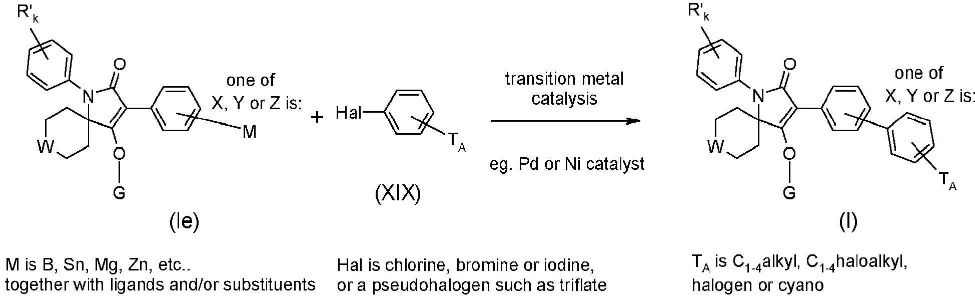

One person skilled in the art will recognize that the polarity at the two reacting centers in this cross-coupling process may be reversed. Compounds of the formula I, wherein X, Y or Z is phenyl or phenyl substituted by C₁₋₄alkyl, C₁₋₄haloalkyl, halogen or cyano, may be also prepared by reacting a corresponding organometallic species of the formula Ie, wherein M is for example B, Sn, Si, Mg or Zn holding further ligands and/or substituents, with an aryl halide of the formula XIX, wherein Hal is chlorine, bromine, iodine or a pseudohalogen such as C₁₋₄haloalkylsulfonate, especially triflate, by means of a transition metal-catalyzed reaction and under similar conditions as described above.

The compounds shown below of the formula IV*, V*, IV, V, VII, VIII and XI, and salts thereof, are novel, have been specifically designed for the synthesis of the compounds of the formula I and as such form a further aspect of the invention:

The compounds of the formula IV* 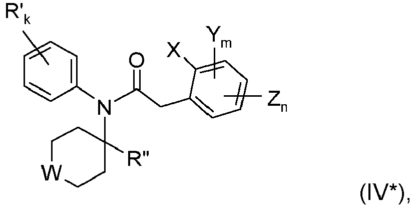 or salts thereof, wherein W, X, Y, Z, k, m, n, and R' have the meanings assigned to them above, and R" is either-C≡N or -C(O)OR₁₄, wherein R₁₄ is C₁₋₆alkyl.
• In one embodiment of the formula IV*, R" is -C(O)OR₁₄, wherein R₁₄ is C₁₋₆alkyl, according to the compounds of the formula IV 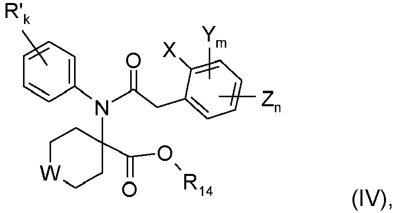 and salts thereof, wherein W, X, Y, Z, k, m, n, and R' have the meanings assigned to them above;
• In an alternative embodiment of the formula IV*, R" is -C≡N, according to the compounds of the formula XI 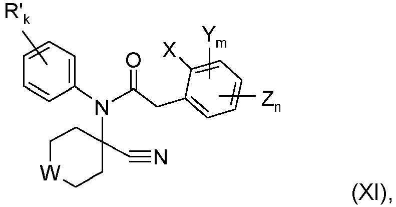 and salts thereof, wherein W, X, Y, Z, k, m, n, and R' have the meanings assigned to them above;
   the compounds of the formula V* 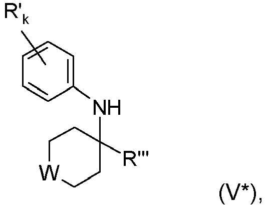 or salts thereof, wherein W, R' and k have the meanings assigned to them above, and R"' is either-C≡N, -C(O)OH or -C(O)OR₁₄, wherein R₁₄ is C₁₋₆alkyl.
• Thus in one embodiment of the formula V*, R"' is -C(O)OR₁₄, wherein R₁₄ is C₁₋₆alkyl, according to the compounds of the formula V 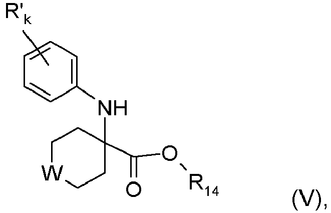 and salts thereof, wherein W, R' and k have the meanings assigned to them above;
• In an alternative embodiment of the formula V*, R'" is -C(O)OH, according to the compounds of the formula VIII 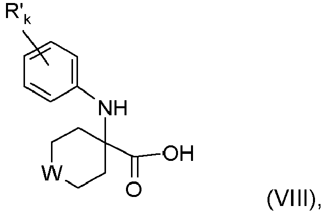 and salts thereof, wherein W, R' and k have the meanings assigned to them above;
• In another alternative embodiment of the formula V*, R"' is -C≡N, according to the compounds of the formula VII 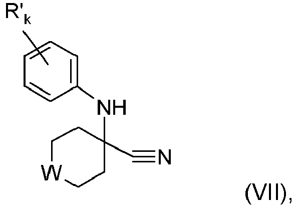 and salts thereof, wherein W, R' and k have the meanings assigned to them above;

The remaining starting compounds and intermediates of the reaction schemes are known or can be prepared according to methods known to a person skilled in the art.

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reaction is advantageously carried out in a temperature range from approximately - 80°C to approximately +140°C, preferably from approximately -30°C to approximately +100°C, in many cases in the range between ambient temperature and approximately +80°C.

A compound I can be converted in a manner known per se into another compound I by replacing one or more substituents of the starting compound I in the customary manner by (an)other substituent(s) according to the invention.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds I can be prepared in a manner known per se. Thus, for example, acid addition salts of compounds I are obtained by treatment with a suitable acid or a suitable ion exchanger reagent and salts with bases are obtained by treatment with a suitable base or with a suitable ion exchanger reagent.

Salts of compounds I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds I can be converted in a manner known per se into other salts of compounds I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds I, which have salt-forming properties can be obtained in free form or in the form of salts.

The compounds I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds according to the following Tables 1 to 17 below can be prepared according to the methods described above.

The examples which follow are intended to illustrate the invention and show preferred compounds of formula I.

### Table 1: This table discloses the 146 compounds T1.001 to T1.146 of the formula la:

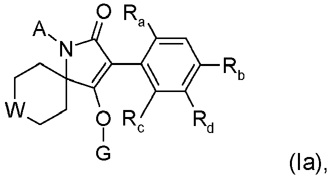

wherein W is W¹, R is CH₃, A is phenyl, G is hydrogen and Rₐ, R_{b}, R_{c} and R_{d} are as defined below:

| No. | Rₐ | R_{b} | R_{c} | R_{d} |
|---|---|---|---|---|
| T1.001 | Br | H | H | H |
| T1.002 | Cl | H | H | H |
| T1.003 | CH₃ | H | H | H |
| T1.004 | CH₂CH₃ | H | H | H |
| T1.005 | OCH₃ | H | H | H |
| T1.006 | Br | Cl | H | H |
| T1.007 | Cl | Br | H | H |
| T1.008 | Cl | Cl | H | H |
| T1.009 | Cl | CH₃ | H | H |
| T1.010 | CH₃ | Cl | H | H |
| T1.011 | CH₃ | CH₃ | H | H |
| T1.012 | Cl | H | Cl | H |
| T1.013 | Cl | H | CH₃ | H |
| T1.014 | Cl | H | CH₂CH₃ | H |
| T1.015 | Cl | H | OCH₃ | H |
| T1.016 | CH₃ | H | CH₃ | H |
| T1.017 | CH₃ | H | CH₂CH₃ | H |
| T1.018 | CH₃ | H | OCH₃ | H |
| T1.019 | CH₂CH₃ | H | CH₂CH₃ | H |
| T1.020 | CH₂CH₃ | H | OCH₃ | H |
| T1.021 | OCH₃ | H | OCH₃ | H |
| T1.022 | Br | H | H | Cl |
| T1.023 | Br | H | H | CH₃ |
| T1.024 | Br | H | H | 4-Cl-C₆H₄ |
| T1.025 | Cl | H | H | Cl |
| T1.026 | Cl | H | H | CH₃ |
| T1.027 | Cl | H | H | 4-Cl-C₆H₄ |
| T1.028 | CH₃ | H | H | Br |
| T1.029 | CH₃ | H | H | Cl |
| T1.030 | CH₃ | H | H | CH₃ |
| T1.031 | CH₃ | H | H | C₆H₅ |
| T1.032 | CH₃ | H | H | 4-Cl-C₆H₄ |
| T1.033 | CH₂CH₃ | H | H | CH₃ |
| T1.034 | CH₂CH₃ | H | H | 4-Cl-C₆H₄ |
| T1.035 | OCH₃ | H | H | CH₃ |
| T1.036 | OCH₃ | H | H | 4-Cl-C₆H₄ |
| T1.037 | Cl | H | Cl | Br |
| T1.038 | CH₃ | H | CH₃ | Br |
| T1.039 | CH₃ | H | CH₃ | Cl |
| T1.040 | CH₃ | H | CH₃ | 4-Cl-C₆H₄ |
| T1.041 | Br | Cl | H | CH₃ |
| T1.042 | Br | CH₃ | H | CH₃ |
| T1.043 | Cl | Cl | H | Cl |
| T1.044 | Cl | Br | H | CH₃ |
| T1.045 | Cl | Cl | H | CH₃ |
| T1.046 | Cl | CH₃ | H | Cl |
| T1.047 | Cl | CH₃ | H | CH₃ |
| T1.048 | CH₃ | Br | H | CH₃ |
| T1.049 | CH₃ | Cl | H | CH₃ |
| T1.050 | CH₃ | CH₃ | H | CH₃ |
| T1.051 | CH₃ | CH₃ | H | 4-Cl-C₆H₄ |
| T1.052 | Br | Br | CH₃ | H |
| T1.053 | Br | Cl | CH₃ | H |
| T1.054 | Br | CH₃ | Br | H |
| T1.055 | Br | CH₃ | Cl | H |
| T1.056 | Cl | Br | CH₃ | H |
| T1.057 | Cl | Cl | Cl | H |
| T1.058 | Cl | Cl | CH₃ | H |
| T1.059 | Cl | CH₃ | Cl | H |
| T1.060 | Cl | CH₃ | CH₂CH₃ | H |
| T1.061 | Cl | CH₃ | OCH₃ | H |
| T1.062 | Cl | 4-Cl-C₆H₄ | Cl | H |
| T1.063 | Cl | 4-Cl-C₆H₄ | CH₃ | H |
| T1.064 | Cl | 4-Cl-C₆H₄ | CH₂CH₃ | H |
| T1.065 | Cl | 4-Cl-C₆H₄ | OCH₃ | H |
| T1.066 | CH₃ | Br | CH₃ | H |
| T1.067 | CH₃ | Cl | CH₃ | H |
| T1.068 | CH₃ | CH₃ | Br | H |
| T1.069 | CH₃ | CH₃ | Cl | H |
| T1.070 | CH₃ | CH₃ | CH₃ | H |
| T1.071 | CH₃ | CH₃ | CH₂CH₃ | H |
| T1.072 | CH₃ | CH₃ | OCH₃ | H |
| T1.073 | CH₃ | 4-Cl-C₆H₄ | CH₃ | H |
| T1.074 | CH₃ | 4-Cl-C₆H₄ | CH₂CH₃ | H |
| T1.075 | CH₃ | 4-Cl-C₆H₄ | OCH₃ | H |
| T1.076 | CH₂CH₃ | Br | Br | H |
| T1.077 | CH₂CH₃ | Br | Cl | H |
| T1.078 | CH₂CH₃ | Br | CH₃ | H |
| T1.079 | CH₂CH₃ | Br | CH₂CH₃ | H |
| T1.080 | CH₂CH₃ | Br | OCH₃ | H |
| T1.081 | CH₂CH₃ | Cl | Br | H |
| T1.082 | CH₂CH₃ | Cl | Cl | H |
| T1.083 | CH₂CH₃ | Cl | CH₃ | H |
| T1.084 | CH₂CH₃ | Cl | CH₂CH₃ | H |
| T1.085 | CH₂CH₃ | Cl | OCH₃ | H |
| T1.086 | CH₂CH₃ | CH₃ | Br | H |
| T1.087 | CH₂CH₃ | CH₃ | Cl | H |
| T1.088 | CH₂CH₃ | CH₃ | CH₂CH₃ | H |
| T1.089 | CH₂CH₃ | CH₃ | OCH₃ | H |
| T1.090 | CH₂CH₃ | CH₂CH₃ | CH₃ | H |
| T1.091 | CH₂CH₃ | CH₂CH₃ | CH₂CH₃ | H |
| T1.092 | CH₂CH₃ | 4-Cl-C₆H₄ | Br | H |
| T1.093 | CH₂CH₃ | 4-Cl-C₆H₄ | CH₂CH₃ | H |
| T1.094 | CH₂CH₃ | 4-Cl-C₆H₄ | OCH₃ | H |
| T1.095 | OCH₃ | Br | CH₃ | H |
| T1.096 | OCH₃ | Cl | CH₃ | H |
| T1.097 | OCH₃ | CH₃ | Br | H |
| T1.098 | OCH₃ | CH₃ | Cl | H |
| T1.099 | OCH₃ | CH₃ | OCH₃ | H |
| T1.100 | OCH₃ | 4-Cl-C₆H₄ | OCH₃ | H |
| T1.101 | CH₃ | CH₃ | CH₃ | F |
| T1.102 | CH₃ | CH₃ | CH₃ | Cl |
| T1.103 | CH₃ | CH₃ | CH₃ | Br |
| T1.104 | CH₃ | CH₃ | CH₃ | CH₃ |
| T1.105 | CH₃ | CH₃ | CH₃ | 4-Cl-C₆H₄ |
| T1.106 | Cl | CH₃ | CH₃ | CH₃ |
| T1.107 | CH₃ | Cl | CH₃ | CH₃ |
| T1.108 | CH₃ | CH₃ | Cl | CH₃ |
| T1.109 | CH₂CH₃ | CH₃ | CH₃ | CH₃ |
| T1.110 | OCH₃ | CH₃ | CH₃ | CH₃ |
| T1.111 | Cyclo-C3 | CH₃ | CH₃ | CH₃ |
| T1.112 | CH₃ | CH₃ | Cyclo-C3 | H |
| T1.113 | CH₃ | F | H | Br |
| T1.114 | CH₃ | CH₃ | H | Br |
| T1.115 | CH₂CH₃ | CH₃ | H | CH₃ |
| T1.116 | OCH₃ | CH₃ | H | CH₃ |
| T1.117 | Cyclo-C3 | CH₃ | H | CH₃ |
| T1.118 | CH₂CH₃ | Cl | H | CH₃ |
| T1.119 | OCH₃ | Cl | H | CH₃ |
| T1.120 | Cyclo-C3 | Cl | H | CH₃ |
| T1.121 | Cl | H | CH₃ | CH₃ |
| T1.122 | CH₃ | H | CH₃ | CH₃ |
| T1.123 | CH₂CH₃ | H | CH₃ | CH₃ |
| T1.124 | OCH₃ | H | CH₃ | CH₃ |
| T1.125 | Cyclo-C3 | H | CH₃ | CH₃ |
| T1.126 | F | H | Cl | CH₃ |
| T1.127 | Cl | H | F | CH₃ |
| T1.128 | H | CH₃ | CH₃ | CH₃ |
| T1.129 | Br | CH₃ | CH₃ | CH₃ |
| T1.130 | CH₃ | H | Cl | CH₃ |
| T1.131 | CH₃ | H | Br | CH₃ |
| T1.132 | Br | H | CH₃ | CH₃ |
| T1.133 | CH₃ | CH=CH₂ | CH₃ | H |
| T1.134 | CH₃ | CH₃ | CH=CH₂ | H |
| T1.135 | CH₃ | C≡CH | CH₃ | H |
| T1.136 | CH₃ | CH₃ | C≡CH | H |
| T1.137 | CH₃ | I | CH₃ | H |
| T1.138 | CH₃ | CH₃ | I | H |
| T1.139 | CH₃ | CH₃ | H | I |
| T1.140 | CH₃ | CF₃ | CH₃ | H |
| T1.141 | CH₃ | CH₃ | CF₃ | H |
| T1.142 | CH₃ | CHF₂ | CH₃ | H |
| T1.143 | CH₃ | CH₃ | CHF₂ | H |
| T1.144 | CH₃ | Cyclo-C3 | CH₃ | H |
| T1.145 | CH=CH₂ | CH₃ | CH=CH₂ | H |
| T1.146 | C≡CH | CH₃ | C≡CH | H |

| | | | | |
|---|---|---|---|---|
| Cyclo-C3 means cyclopropyl. | | | | |

Table 2: This table discloses the 146 compounds T2.001 to T2.146 of the formula la, wherein W is W¹, R is CH₂CH₃, A is phenyl, G is hydrogen and Rₐ, R_{b}, R_{c} and R_{d} are as defined in Table 1.
Table 3: This table discloses the 146 compounds T3.001 to T3.146 of the formula la, wherein W is W¹, R is CH₃, A is 4-Cl-phenyl, G is hydrogen and Rₐ, R_{b}, R_{c} and R_{d} are as defined in Table 1.
Table 4: This table discloses the 146 compounds T4.001 to T4.146 of the formula la, wherein W is W¹, R is CH₃, A is 4-F-phenyl, G is hydrogen and Rₐ, R_{b}, R_{c} and R_{d} are as defined in Table 1.
Table 5: This table discloses the 146 compounds T5.001 to T5.146 of the formula la, wherein W is W¹, R is CH₃, A is 4-CH₃-phenyl, G is hydrogen and Rₐ, R_{b}, R_{c} and R_{d} are as defined in Table 1.
Table 6: This table discloses the 146 compounds T6.001 to T6.146 of the formula la, wherein W is W¹, R is CH₃, A is 4-CH₃O-phenyl, G is hydrogen and Rₐ, R_{b}, R_{c} and R_{d} are as defined in Table 1.
Table 7: This table discloses the 146 compounds T7.001 to T7.146 of the formula la, wherein W is W¹, R is CH₃, A is 3,5-dichloro-phenyl, G is hydrogen and Rₐ, R_{b}, R_{c} and R_{d} are as defined in Table 1.
Table 8: This table discloses the 146 compounds T8.001 to T8.146 of the formula la, wherein W is W², R is hydrogen, A is phenyl, G is hydrogen and Rₐ, R_{b}, R_{c} and R_{d} are as defined in Table 1.
Table 9: This table discloses the 146 compounds T9.001 to T9.146 of the formula la, wherein W is W², R is CH₃, A is phenyl, G is hydrogen and Rₐ, R_{b}, R_{c} and R_{d} are as defined in Table 1.
Table 10: This table discloses the 146 compounds T10.001 to T10.146 of the formula la, wherein W is W², R is CH₂CH₃, A is phenyl, G is hydrogen and Rₐ, R_{b}, R_{c} and R_{d} are as defined in Table 1.
Table 11: This table discloses the 146 compounds T11.001 to T11.146 of the formula la, wherein W is W², R is CH₂CF₃, A is phenyl, G is hydrogen and Rₐ, R_{b}, R_{c} and R_{d} are as defined in Table 1.
Table 12: This table discloses the 146 compounds T12.001 to T12.146 of the formula la, wherein W is W², R is CH₃, A is 4-Cl-phenyl, G is hydrogen and Rₐ, R_{b}, R_{c} and R_{d} are as defined in Table 1.
Table 13: This table discloses the 146 compounds T13.001 to T13.146 of the formula la, wherein W is W², R is CH₃, A is 4-F-phenyl, G is hydrogen and Rₐ, R_{b}, R_{c} and R_{d} are as defined in Table 1.
Table 14: This table discloses the 146 compounds T14.001 to T14.146 of the formula la, wherein W is W², R is CH₃, A is 4-CH₃-phenyl, G is hydrogen and Rₐ, R_{b}, R_{c} and R_{d} are as defined in Table 1.
Table 15: This table discloses the 146 compounds T15.001 to T15.146 of the formula la, wherein W is W², R is CH₃, A is 4-CH₃O-phenyl, G is hydrogen and Rₐ, R_{b}, R_{c} and R_{d} are as defined in Table 1.
Table 16: This table discloses the 146 compounds T16.001 to T16.146 of the formula la, wherein W is W², R is CH₃, A is 4-cyano-phenyl, G is hydrogen and Rₐ, R_{b}, R_{c} and R_{d} are as defined in Table 1.
Table 17: This table discloses the 146 compounds T17.001 to T17.146 of the formula la, wherein W is W², R is CH₃, A is 3,5-dichloro-phenyl, G is hydrogen and Rₐ, R_{b}, R_{c} and R_{d} are as defined in Table 1.

In one aspect of the invention, the compounds according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

The compounds of formula I can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fibre products), horticulture and animal husbandry, companion animals, forestry and the storage of products of vegetable origin (such as fruit, grain and timber); those pests associated with the damage of man-made structures and the transmission of diseases of man and animals; and also nuisance pests (such as flies). The compounds of formula I of the invention are particularly effective against insects, acarines and/or nematodes. This list does not represent any limitation.

Examples of pest species which may be controlled by the compounds of formula I include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobacco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite), *Polyphagotarsonemus latus* (broad mite), *Brevipalpus* spp. (flat mites), *Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), *Culex* spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus, Reticulitermes flavipes, R. speratu, R. virginicus, R. hesperus,* and *R*. *santonensis*) and the Termitidae (for example *Globitermes sulphureus*), *Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp. (citrus nematodes), *Haemonchus contortus* (barber pole worm), *Caenorhabditis elegans* (vinegar eelworm), *Trichostrongylus* spp. (gastro intestinal nematodes) and *Deroceras reticulatum* (slug).

In another embodiment, the compounds according to the present invention are also particularly effective against the following pests:
from the order *Acarina,* for example,
   Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
   Haematopinus spp., Linognathus spp., Pediculus spp., Pemphigus spp. and Phylloxera spp.;
from the order *Coleoptera,* for example,
   Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.;
from the order *Diptera,* for example,
   Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
   Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euchistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
from the order *Homoptera,* for example,
   Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris;
from the order *Hymenoptera,* for example,
   Acromyrmex, Arge spp, Atta spp., Cephus spp., Diprion spp., Diprionidae, Gilpinia polytoma, Hoplocampa spp., Lasius spp., Monomorium pharaonis, Neodiprion spp., Pogonomyrmex spp, Slenopsis invicta, Solenopsis spp. and Vespa spp.;
from the order *Isoptera,* for example,
   Coptotermes spp, Corniternes cumulans, Incisitermes spp, Macrotermes spp, Mastotermes spp, Microtermes spp, Reticulitermes spp.; Solenopsis geminate;
from the order *Lepidoptera,* for example,
   Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
   Damalinea spp. and Trichodectes spp.;
from the order *Orthoptera,* for example,
   Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
   Liposcelis spp.;
from the order *Siphonaptera,* for example,
   Ceratophyllus spp., Ctenocephalides spp. and Xenopsylla cheopis;
from the order *Thysanoptera,* for example,
   Calliothrips phaseoli, Frankliniella spp., Heliothrips spp, Hercinothrips spp., Parthenothrips spp, Scirtothrips aurantii, Sericothrips variabilis, Taeniothrips spp., Thrips spp; and
from the order *Thysanura,* for example,
   Lepisma saccharina.

This list does not represent any limitation.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

The combinations of the present invention are of particular interest for controlling pests in various useful plants or their seeds, including in lawns, fruit, vegetables and ornamentals in horticulture and viticulture.

Suitable target crops are, in particular, soybean, alfalfa, brassicas, or oil crops, such as oilseed rape, mustard, canola, poppies, olives, sunflowers, coconut, castor, cocoa or ground nuts, or potatoes (including sweet potatoes), almonds, fruiting vegetables (e.g. tomatoes, pepper, chili,eggplant, cucumber, squash etc.), bulb vegetables (e.g. onion, leek etc.), grapes, fruit, for instance pomaceous fruit, stone fruit or soft fruit (e.g. apples, pears, plums, peaches, almonds, cherries etc.) or berries, for example strawberries, raspberries or blackberries.

Other suitable target crops are, in particular, cereals, such as wheat, barley, rye, oats, rice, maize or sorghum; beet, such as sugar or fodder beet; leguminous crops, such as beans, lentils, peas or soya; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, or bell peppers; Lauraceae, such as avocado, Cinnamonium or camphor; and also tobacco, nuts, coffee, eggplants, sugarcane, tea, pepper, grapevines, hops, the plantain family, latex plants and ornamentals.

The compounds of formula I of the invention may be used on soybean to control, for example, Elasmopalpus lignosellus, Diloboderus abderus, Diabrotica speciosa, Sternechus subsignatus, Formicidae, Agrotis ypsilon, Julus sspp., Anticarsia gemmatalis, Megascelis ssp., Procornitermes ssp., Gryllotalpidae, Nezara viridula, Piezodorus spp., Acrosternum spp., Neomegalotomus spp., Cerotoma trifurcata, Popillia japonica, Edessa spp., Liogenys fuscus, Euchistus heros, stalk borer, Scaptocoris castanea, phyllophaga spp., Pseudoplusia includens, Spodoptera spp., Bemisia tabaci, Agriotes spp. The compounds of formula I of the invention are preferably used on soybean to control Diloboderus abderus, Diabrotica speciosa, Nezara viridula, Piezodorus spp., Acrosternum spp., Cerotoma trifurcata, Popillia japonica, Euchistus heros, phyllophaga spp., Agriotes sp

The compounds of formula I of the invention may be used on corn to control, for example, Euchistus heros, Dichelops furcatus, Diloboderus abderus, Elasmopalpus lignosellus, Spodoptera frugiperda, Nezara viridula, Cerotoma trifurcata, Popillia japonica, Agrotis ypsilon, Diabrotica speciosa, Heteroptera, Procornitermes ssp., Scaptocoris castanea, Formicidae, Julus ssp., Dalbulus maidis, Diabrotica virgifera, Mocis latipes, Bemisia tabaci, heliothis spp., Tetranychus spp., thrips spp., phyllophaga spp., scaptocoris spp., Liogenys fuscus, Spodoptera spp., Ostrinia spp., Sesamia spp.,. Agriotes spp. The compounds of formula I of the invention are preferably used on corn to control Euchistus heros, Dichelops furcatus, Diloboderus abderus, Nezara viridula, Cerotoma trifurcata, Popillia japonica, Diabrotica speciosa, Diabrotica virgifera, Tetranychus spp., thrips spp., phyllophaga spp., scaptocoris spp., Agriotes spp.

The compounds of formula I of the invention may be used on sugar cane to control, for example, Sphenophorus spp., termites, Mahanarva spp.. The compounds of formula I of the invention are preferably used on sugar cane to control termites, Mahanarva spp.

The compounds of formula I of the invention may be used on alfalfa to control, for example, Hypera brunneipennis, Hypera postica, Colias eurytheme, Collops spp., Empoasca solana, Epitrix, Geocoris spp., Lygus hesperus, Lygus lineolaris, Spissistilus spp., Spodoptera spp., Trichoplusia ni. The compounds of formula I of the invention are preferably used on alfalfa to control Hypera brunneipennis, Hypera postica, Empoasca solana, Epitrix, Lygus hesperus, Lygus lineolaris, Trichoplusia ni.

The compounds of formula I of the invention may be used on brassicas to control, for example, Plutella xylostella, Pieris spp., Mamestra spp., Plusia spp., Trichoplusia ni, Phyllotreta spp., Spodoptera spp., Empoasca solana, thrips spp., Spodoptera spp., Delia spp. The compounds of formula I of the invention are preferably used on brassicas to control Plutella xylostella Pieris spp., Plusia spp., Trichoplusia ni, Phyllotreta spp., thrips sp The compounds of formula I of the invention may be used on oil seed rape, e.g. canola, to control, for example, Meligethes spp., Ceutorhynchus napi, Psylloides spp.

The compounds of formula I of the invention may be used on potatoes, including sweet potatoes, to control, for example, Empoasca spp., Leptinotarsa spp., Diabrotica speciosa, Phthorimaea spp., Paratrioza spp., Maladera matrida, Agriotes spp. The compounds of formula I of the invention are preferably used on potatoes, including sweet potatoes, to control Empoasca spp., Leptinotarsa spp., Diabrotica speciosa, Phthorimaea spp., Paratrioza spp., Agriotes spp.

The compounds of formula I of the invention may be used on cotton to control, for example, Anthonomus grandis, Pectinophora spp., heliothis spp., Spodoptera spp., Tetranychus spp., Empoasca spp., thrips spp., Bemisia tabaci, Lygus spp., phyllophaga spp., Scaptocoris spp. The compounds of formula I of the invention are preferably used on cotton to control Anthonomus grandis, Tetranychus spp., Empoasca spp., thrips spp., Lygus spp., phyllophaga spp., Scaptocoris spp.

The compounds of formula I of the invention may be used on rice to control, for example, Leptocorisa spp., Cnaphalocrosis spp., Chilo spp., Scirpophaga spp., Lissorhoptrus spp., Oebalus pugnax. The compounds of formula I of the invention are preferably used on rice to control Leptocorisa spp., Lissorhoptrus spp., Oebalus pugnax.

The compounds of formula I of the invention may be used on coffee to control, for example, Hypothenemus Hampei, Perileucoptera Coffeella, Tetranychus spp. The compounds of formula I of the invention are preferably used on coffee to control Hypothenemus Hampei, Perileucoptera Coffeella.

The compounds of formula I of the invention may be used on citrus to control, for example, Panonychus citri, Phyllocoptruta oleivora, Brevipalpus spp., Diaphorina citri, Scirtothrips spp., thrips spp., Unaspis spp., Ceratitis capitata, Phyllocnistis spp. The compounds of formula I of the invention are preferably used on citrus to control Panonychus citri, Phyllocoptruta oleivora, Brevipalpus spp., Diaphorina citri, Scirtothrips spp., thrips spp., Phyllocnistis spp.

The compounds of formula I of the invention may be used on almonds to control, for example, Amyelois transitella, Tetranychus spp.

The compounds of formula I of the invention may be used on fruiting vegetable, including tomatoes, pepper, chili, eggplant, cucumber, squash etc, to control thrips spp., Tetranychus spp., Polyphagotarsonemus spp., Aculops spp., Empoasca spp., Spodoptera spp., heliothis spp., Tuta absoluta, Liriomyza spp., Bemisia tabaci, Trialeurodes spp., Paratrioza spp., Frankliniella occidentalis, Frankliniella spp., Anthonomus spp., Phyllotreta spp., Amrasca spp., Epilachna spp., Halyomorpha spp., Scirtothrips spp., Leucinodes spp., Neoleucinodes spp.. The compounds of formula I of the invention are preferably used on fruiting vegetable, including tomatoes, pepper, chili, eggplant, cucumber, squash etc, to control, for example, thrips spp., Tetranychus spp., Polyphagotarsonemus spp., Aculops spp., Empoasca spp., Spodoptera spp., heliothis spp., Tuta absoluta, Liriomyza spp., Paratrioza spp., Frankliniella occidentalis, Frankliniella spp., Amrasca spp., Scirtothrips spp., Leucinodes spp., Neoleucinodes spp.

The compounds of formula I of the invention may be used on tea to control, for example, Pseudaulacaspis spp., Empoasca spp., Scirtothrips spp., Caloptilia theivora. The compounds of formula I of the invention are prefrerably used on tea to control Empoasca spp., Scirtothrips spp.

The compounds of formula I of the invention may be used on bulb vegetables, including onion, leek etc to control, for example, thrips spp., Spodoptera spp., heliothis spp. The compounds of formula I of the invention are preferably used on bulb vegetables, including onion, leek etc to control thrips spp.

The compounds of formula I of the invention may be used on grapes to control, for example, Empoasca spp., Lobesia spp., Frankliniella spp., thrips spp., Tetranychus spp., Rhipiphorothrips Cruentatus, Eotetranychus Willamettei, Erythroneura Elegantula, Scaphoides spp.. The compounds of formula I of the invention are preferably used on grapes to control Frankliniella spp., thrips spp., Tetranychus spp., Rhipiphorothrips Cruentatus, Scaphoides spp.

The compounds of formula I of the invention may be used on pome fruit, including apples, pairs etc, to control, for example, Cacopsylla spp., Psylla spp., Panonychus ulmi, Cydia pomonella. The compounds of formula I of the invention are preferably used on pome fruit, including apples, pairs etc, to control Cacopsylla spp., Psylla spp., Panonychus ulmi.

The compounds of formula I of the invention may be used on stone fruit to control, for example, Grapholita molesta, Scirtothrips spp., thrips spp., Frankliniella spp., Tetranychus spp. The compounds of formula I of the invention are preferably used on stone fruit to control Scirtothrips spp., thrips spp., Frankliniella spp., Tetranychus spp.

This list does not represent any limitation.

The term "crops" or "useful plants" is to be understood as including also crops that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors) as a result of conventional methods of breeding or genetic engineering. An example of a crop that has been rendered tolerant to imidazolinones, e.g. imazamox, by conventional methods of breeding (mutagenesis) is Clearfield® summer rape (Canola). Examples of crops that have been rendered tolerant to herbicides or classes of herbicides by genetic engineering methods include glyphosate- and glufosinate-resistant maize varieties commercially available under the trade names RoundupReady® and LibertyLink®.

The term "crops" or "useful plants" is also to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1A(b), Cry1A(c), Cry1 F, Cry1 F(a2), Cry2A(b), Cry3A, Cry3B(b1) or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1A(b), Cry1A(c), Cry1F, Cry1F(a2), Cry2A(b), Cry3A, Cry3B(b1) or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). An example for a truncated toxin is a truncated Cry1A(b), which is expressed in the Bt11 maize from Syngenta Seed SAS, as described below. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard® (maize variety that expresses a Cry1A(b) toxin); YieldGard Rootworm® (maize variety that expresses a Cry3B(b1) toxin); YieldGard Plus® (maize variety that expresses a Cry1A(b) and a Cry3B(b1) toxin); Starlink® (maize variety that expresses a Cry9(c) toxin); Herculex I® (maize variety that expresses a Cry1 F(a2) toxin and the enzyme phosphinothricine N-acetyltransferase (PAT) to achieve tolerance to the herbicide glufosinate ammonium); NuCOTN 33B® (cotton variety that expresses a Cry1A(c) toxin); Bollgard I® (cotton variety that expresses a Cry1A(c) toxin); Bollgard II® (cotton variety that expresses a Cry1A(c) and a Cry2A(b) toxin); VipCOT® (cotton variety that expresses a Vip3A and a Cry1Ab toxin); NewLeaf® (potato variety that expresses a Cry3A toxin); NatureGard® and Protecta®.

Further examples of such transgenic crops are:
1. Bt11 Maize from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1A(b) toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified Zea *mays* which has been rendered resistant to attack by the European corn borer (*Ostrinia nubilalis* and *Sesamia nonagrioides*) by transgenic expression of a Cry1A(b) toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3B(b1) toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1 F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 x MON 810 Maize transgenically expresses the protein CP4 EPSPS, obtained from *Agrobacterium sp.* strain CP4, which imparts tolerance to the herbicide Roundup® (contains glyphosate), and also a Cry1A(b) toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003.

The term "crops" or "useful plants" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Crops may also be modified for enhanced resistance to fungal (for example *Fusarium, Anthracnose*, or *Phytophthora*), bacterial (for example *Pseudomonas*) or viral (for example potato leafroll virus, tomato spotted wilt virus, cucumber mosaic virus) pathogens.

Crops also include those that have enhanced resistance to nematodes, such as the soybean cyst nematode.

Crops that are tolerant to abiotic stress include those that have enhanced tolerance to drought, high salt, high temperature, chill, frost, or light radiation, for example through expression of NF-YB or other proteins known in the art.

Crops that exhibit enhanced yield or quality include those with improved flowering or fruit ripening properties (such as delayed ripening); modified oil, starch, amino acid, fatty acid, vitamin, phenolic or other content (such as Vistive™ soybean variety); enhanced nutrient utilisation (such as improved nitrogen assimilation); and enhanced quality plant product (such as higher quality cotton fibre).

Useful plants of elevated interest in connection with present invention are cereals; soybean; rice; oil seed rape; pome fruits; stone fruits; peanuts; coffee; tea; strawberries; turf; vines and vegetables, such as tomatoes, potatoes, cucurbits and lettuce.

The term "locus" of a useful plant as used herein is intended to embrace the place on which the useful plants are growing, where the plant propagation materials of the useful plants are sown or where the plant propagation materials of the useful plants will be placed into the soil. An example for such a locus is a field, on which crop plants are growing.

The term "plant propagation material" is understood to denote generative parts of a plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

A further aspect of the instant invention is a method of protecting natural substances of plant and/or animal origin, which have been taken from the natural life cycle, and/or their processed forms against attack of pests, which comprises applying to said natural substances of plant and/or animal origin or their processed forms, a compound of the formula I.

According to the instant invention, the term "natural substances of plant origin, which have been taken from the natural life cycle" denotes plants or parts thereof which have been harvested from the natural life cycle and which are in the freshly harvested form. Examples of such natural substances of plant origin are stalks, leafs, tubers, seeds, fruits or grains. According to the instant invention, the term "processed form of a natural substance of plant origin" is understood to denote a form of a natural substance of plant origin that is the result of a modification process. Such modification processes can be used to transform the natural substance of plant origin in a more storable form of such a substance (a storage good). Examples of such modification processes are pre-drying, moistening, crushing, comminuting, grounding, compressing or roasting. Also falling under the definition of a processed form of a natural substance of plant origin is timber, whether in the form of crude timber, such as construction timber, electricity pylons and barriers, or in the form of finished articles, such as furniture or objects made from wood.

According to the instant invention, the term "natural substances of animal origin, which have been taken from the natural life cycle and/or their processed forms" is understood to denote material of animal origin such as skin, hides, leather, furs, hairs and the like.

A preferred embodiment is a method of protecting natural substances of plant origin, which have been taken from the natural life cycle, and/or their processed forms against attack of pests, which comprises applying to said natural substances of plant and/or animal origin or their processed forms, a compound of the formula I.

A further preferred embodiment is a method of protecting fruits, preferably pomes, stone fruits, soft fruits and citrus fruits, which have been taken from the natural life cycle, and/or their processed forms, which comprises applying to said fruits and/or their processed forms, a compound of the formula I.

Further areas of use of the compounds and compositions according to the invention are the protection of stored goods and storerooms and the protection of raw materials, such as wood, textiles, floor coverings or buildings, and also in the hygiene sector, especially the protection of humans, domestic animals and productive livestock against pests of the mentioned type.

In the hygiene sector, the compounds and compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..
Of the order Mallophagida: Trimenopon spp., Menopon spp., Trinoton spp., Bovicola spp., Werneckiella spp., Lepikentron spp., Damalina spp., Trichodectes spp. and Felicola spp..
Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..
Of the order Siphonapterida, for example Pulex spp., Ctenocephalides spp., Xenopsylla spp., Ceratophyllus spp..
Of the order Heteropterida, for example Cimex spp., Triatoma spp., Rhodnius spp., Panstrongylus spp..
Of the order Blattarida, for example Blatta orientalis, Periplaneta americana, Blattelagermanica and Supella spp..
Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..
Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compounds and compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The invention therefore provides a method of combating and controlling insects, acarines, nematodes or molluscs which comprises applying an insecticidally, acaricidally, nematicidally or molluscicidally effective amount of a compound of formula I, or a composition containing a compound of formula I, to a pest, a locus of pest, or to a plant susceptible to attack by a pest. The compounds of formula I are preferably used against insects or acarines or nematodes.

The term "plant" as used herein includes seedlings, bushes and trees.

The invention also relates to pesticidal compositions such as emulsifiable concentrates, suspension concentrates, directly sprayable or dilutable solutions, spreadable pastes, dilute emulsions, soluble powders, dispersible powders, wettable powders, dusts, granules or encapsulations in polymeric substances, which comprise - at least - one of the active ingredients according to the invention and which are to be selected to suit the intended aims and the prevailing circumstances.

In these compositions, the active ingredient is employed in pure form, a solid active ingredient for example in a specific particle size, or, preferably, together with - at least - one of the auxiliaries conventionally used in the art of formulation, such as extenders, for example solvents or solid carriers, or such as surface-active compounds (surfactants) or penetration enhancers, formulation adjuvants.

Preferably, for pesticidal and in particular for insecticidal uses, examples of suitable solvents are: unhydrogenated or partially hydrogenated aromatic hydrocarbons, preferably the fractions C8 to C12 of alkylbenzenes, such as xylene mixtures, alkylated naphthalenes or tetrahydronaphthalene, aliphatic or cycloaliphatic hydrocarbons, such as paraffins or cyclohexane, alcohols such as ethanol, propanol or butanol, glycols and their ethers and esters such as propylene glycol, dipropylene glycol ether, ethylene glycol or ethylene glycol monomethyl ether or ethylene glycol monoethyl ether, ketones, such as cyclohexanone, isophorone or diacetone alcohol, strongly polar solvents, such as N-methylpyrrolid-2-one, dimethyl sulfoxide or N,N-dimethylformamide, water, unepoxidized or epoxidized vegetable oils, such as unexpodized or epoxidized rapeseed, castor, coconut or soya oil, and silicone oils.

Solid carriers which are used for example for dusts and dispersible powders are, as a rule, ground natural minerals such as calcite, talc, kaolin, montmorillonite or attapulgite. To improve the physical properties, it is also possible to add highly disperse silicas or highly disperse absorbtive polymers. Suitable particulate adsorptive carriers for granules are porous types, such as pumice, brick grit, sepiolite or bentonite, and suitable non-sorptive carrier materials are calcite or sand. In addition, a large number of granulated materials of inorganic or organic nature can be used, in particular dolomite or comminuted plant residues.

Suitable surface-active compounds are, depending on the type of the active ingredient to be formulated, non-ionic, cationic and/or anionic surfactants or surfactant mixtures which have good emulsifying, dispersing and wetting properties. The surfactants mentioned below are only to be considered as examples; a large number of further surfactants which are conventionally used in the art of formulation and suitable according to the invention are described in the relevant literature.

Suitable non-ionic surfactants are, especially, polyglycol ether derivatives of aliphatic or cycloaliphatic alcohols, of saturated or unsaturated fatty acids or of alkyl phenols which may contain approximately 3 to approximately 30 glycol ether groups and approximately 8 to approximately 20 carbon atoms in the (cyclo)aliphatic hydrocarbon radical or approximately 6 to approximately 18 carbon atoms in the alkyl moiety of the alkyl phenols. Also suitable are water-soluble polyethylene oxide adducts with polypropylene glycol, ethylenediaminopolypropylene glycol or alkyl polypropylene glycol having 1 to approximately 10 carbon atoms in the alkyl chain and approximately 20 to approximately 250 ethylene glycol ether groups and approximately 10 to approximately 100 propylene glycol ether groups. Normally, the abovementioned compounds contain 1 to approximately 5 ethylene glycol units per propylene glycol unit. Examples which may be mentioned are nonylphenoxypolyethoxyethanol, castor oil polyglycol ether, polypropylene glycol/polyethylene oxide adducts, tributylphenoxypolyethoxyethanol, polyethylene glycol or octylphenoxypolyethoxyethanol. Also suitable are fatty acid esters of polyoxyethylene sorbitan, such as polyoxyethylene sorbitan trioleate.

The cationic surfactants are, especially, quarternary ammonium salts which generally have at least one alkyl radical of approximately 8 to approximately 22 C atoms as substituents and as further substituents (unhalogenated or halogenated) lower alkyl or hydroxyalkyl or benzyl radicals. The salts are preferably in the form of halides, methylsulfates or ethylsulfates. Examples are stearyltrimethylammonium chloride and benzylbis(2-chloroethyl)ethylammonium bromide.

Examples of suitable anionic surfactants are water-soluble soaps or water-soluble synthetic surface-active compounds. Examples of suitable soaps are the alkali, alkaline earth or (unsubstituted or substituted) ammonium salts of fatty acids having approximately 10 to approximately 22 C atoms, such as the sodium or potassium salts of oleic or stearic acid, or of natural fatty acid mixtures which are obtainable for example from coconut or tall oil; mention must also be made of the fatty acid methyl taurates. However, synthetic surfactants are used more frequently, in particular fatty sulfonates, fatty sulfates, sulfonated benzimidazole derivatives or alkylaryl sulfonates. As a rule, the fatty sulfonates and fatty sulfates are present as alkali, alkaline earth or (substituted or unsubstituted) ammonium salts and they generally have an alkyl radical of approximately 8 to approximately 22 C atoms, alkyl also to be understood as including the alkyl moiety of acyl radicals; examples which may be mentioned are the sodium or calcium salts of lignosulfonic acid, of the dodecylsulfuric ester or of a fatty alcohol sulfate mixture prepared from natural fatty acids. This group also includes the salts of the sulfuric esters and sulfonic acids of fatty alcohol/ethylene oxide adducts. The sulfonated benzimidazole derivatives preferably contain 2 sulfonyl groups and a fatty acid radical of approximately 8 to approximately 22 C atoms. Examples of alkylarylsulfonates are the sodium, calcium or triethanolammonium salts of decylbenzenesulfonic acid, of dibutyl-naphthalenesulfonic acid or of a naphthalenesulfonic acid/formaldehyde condensate. Also possible are, furthermore, suitable phosphates, such as salts of the phosphoric ester of a p-nonylphenol/(4-14)ethylene oxide adduct, or phospholipids. Further suitable phosphates are tris-esters of phosphoric acid with aliphatic or aromatic alcohols and/or bis-esters of alkyl phosphonic acids with aliphatic or aromatic alcohols, which are a high performance oil-type adjuvant. These tris-esters have been described, for example, in WO0147356, WO0056146, EP-A-0579052 or EP-A-1018299 or are commercially available under their chemical name. Preferred tris-esters of phosphoric acid for use in the new compositions are tris-(2-ethylhexyl) phosphate, tris-n-octyl phosphate and tris-butoxyethyl phosphate, where tris-(2-ethylhexyl) phosphate is most preferred. Suitable bis-ester of alkyl phosphonic acids are bis-(2-ethylhexyl)-(2-ethylhexyl)-phosphonate, bis-(2-ethylhexyl)-(n-octyl)-phosphonate, dibutyl-butyl phosphonate and bis(2-ethylhexyl)-tripropylene-phosphonate, where bis-(2-ethylhexyl)-(n-octyl)-phosphonate is particularly preferred.

The compositions according to the invention can preferably additionally include an additive comprising an oil of vegetable or animal origin, a mineral oil, alkyl esters of such oils or mixtures of such oils and oil derivatives. The amount of oil additive used in the composition according to the invention is generally from 0.01 to 10 %, based on the spray mixture. For example, the oil additive can be added to the spray tank in the desired concentration after the spray mixture has been prepared. Preferred oil additives comprise mineral oils or an oil of vegetable origin, for example rapeseed oil such as ADIGOR® and MERO®, olive oil or sunflower oil, emulsified vegetable oil, such as AMIGO® (Rhône-Poulenc Canada Inc.), alkyl esters of oils of vegetable origin, for example the methyl derivatives, or an oil of animal origin, such as fish oil or beef tallow. A preferred additive contains, for example, as active components essentially 80 % by weight alkyl esters of fish oils and 15 % by weight methylated rapeseed oil, and also 5 % by weight of customary emulsifiers and pH modifiers. Especially preferred oil additives comprise alkyl esters of C₈-C₂₂ fatty acids, especially the methyl derivatives of C₁₂-C₁₈ fatty acids, for example the methyl esters of lauric acid, palmitic acid and oleic acid, being important. Those esters are known as methyl laurate (CAS-111-82-0), methyl palmitate (CAS-112-39-0) and methyl oleate (CAS-112-62-9). A preferred fatty acid methyl ester derivative is Emery® 2230 and 2231 (Cognis GmbH). Those and other oil derivatives are also known from the Compendium of Herbicide Adjuvants, 5th Edition, Southern Illinois University, 2000. Also, alkoxylated fatty acids can be used as additives in the inventive compositions as well as polymethylsiloxane based additives, which have been described in WO08/037373.

The application and action of the oil additives can be further improved by combining them with surface-active substances, such as non-ionic, anionic or cationic surfactants. Examples of suitable anionic, non-ionic and cationic surfactants are listed on pages 7 and 8 of WO 97/34485. Preferred surface-active substances are anionic surfactants of the dodecyl-benzylsulfonate type, especially the calcium salts thereof, and also non-ionic surfactants of the fatty alcohol ethoxylate type. Special preference is given to ethoxylated C₁₂-C₂₂ fatty alcohols having a degree of ethoxylation of from 5 to 40. Examples of commercially available surfactants are the Genapol types (Clariant AG). Also preferred are silicone surfactants, especially polyalkyl-oxide-modified heptamethyltrisiloxanes, which are commercially available e.g. as Silwet L-77®, and also perfluorinated surfactants. The concentration of surface-active substances in relation to the total additive is generally from 1 to 30 % by weight. Examples of oil additives that consist of mixtures of oils or mineral oils or derivatives thereof with surfactants are Edenor ME SU®, Turbocharge® (Syngenta AG, CH) and Actipron® (BP Oil UK Limited, GB).

The said surface-active substances may also be used in the formulations alone, that is to say without oil additives.

Furthermore, the addition of an organic solvent to the oil additive/surfactant mixture can contribute to a further enhancement of action. Suitable solvents are, for example, Solvesso® (ESSO) and Aromatic Solvent® (Exxon Corporation).The concentration of such solvents can be from 10 to 80 % by weight of the total weight. Such oil additives, which may be in admixture with solvents, are described, for example, in US-A-4 834 908. A commercially available oil additive disclosed therein is known by the name MERGE® (BASF Corporation). A further oil additive that is preferred according to the invention is SCORE® (Syngenta Crop Protection Canada.)

In addition to the oil additives listed above, in order to enhance the activity of the compositions according to the invention it is also possible for formulations of alkylpyrrolidones, (e.g. Agrimax®) to be added to the spray mixture. Formulations of synthetic latices, such as, for example, polyacrylamide, polyvinyl compounds or poly-1-p-menthene (e.g. Bond®, Courier® or Emerald®) can also be used. Solutions that contain propionic acid, for example Eurogkem Pen-e-trate®, can also be mixed into the spray mixture as activity-enhancing agents.

As a rule, the compositions comprise 0.1 to 99%, especially 0.1 to 95%, of active ingredient of thre formula land 1 to 99.9%, especially 5 to 99.9%, of at least one solid or liquid adjuvant, it being possible as a rule for 0 to 25%, especially 0.1 to 20%, of the composition to be surfactants(% in each case meaning percent by weight). Whereas concentrated compositions tend to be preferred for commercial goods, the end consumer as a rule uses dilute compositions which have substantially lower concentrations of active ingredient.

Preferred compositions are composed as follows (% = percent by weight):

| Emulsifiable concentrates: | | | |
|---|---|---|---|
| active ingredient: | 1 to 95%, preferably 5 to 50%, more preferably 5 to 20% | | |
| surfactant: | 1 to 30%, preferably 10 to 20 % | | |
| solvent: | 5 to 98%, preferably 70 to 85% | | |

| Dusts: | | | |
|---|---|---|---|
| active ingredient: | 0.1 to 10%, preferably 2 to 5%, | | |
| solid carrier: | 99.9 to 90%, preferably 99.9 to 99% | | |

| Suspension concentrates: | | | |
|---|---|---|---|
| active ingredient: | 5 to 75%, preferably 10 to 50%, more preferably 10 to 40% | | |
| water: | 94 to 24%, preferably 88 to 30% | | |
| surfactant: | 1 to 40%, preferably 2 to 30% | | |

| Oil-based suspension concentrates: | | | |
|---|---|---|---|
| active ingredient: | 2 to 75%, preferably 5 to 50%, more preferably 10 to 25% | | |
| oil: | 94 to 24%, preferably 88 to 30% | | |
| surfactant: | 1 to 40%, preferably 2 to 30% | | |

| Wettable powders: | | | |
|---|---|---|---|
| active ingredient: | 0.5 to 90%, preferably 1 to 80%, more preferably 25 75% | to | |
| surfactant: | 0.5 to 20%, preferably 1 to 15% | | |
| solid carrier: | 5 to 99%, preferably 15 to 98% | | |

| Granulates: | | | |
|---|---|---|---|
| active ingredient: | 0.5 to 30%, preferably 3 to 25%, more preferably 3 to 15% | | |
| solid carrier: | 99.5 to 70%, preferably 97 to 85% | | |

| Formulation Examples (% = percent by weight) in particular for pesticidal uses | | | |
|---|---|---|---|
| Example F1: Emulsion concentrates | a) | b) | c) |
| Active ingredient | 25 % | 40 % | 50 % |
| Calcium dodecylbenzenesulfonate | 5 % | 8 % | 6 % |
| Castor oil polyethylene glycol ether (36 mol of EO) | 5 % | - | - |
| Tributylphenoxypolyethylene glycol ether (30 mol of EO) | - | 12 % | 4 % |
| Cyclohexanone | - | 15 % | 20 % |
| Xylene mixture | 65 % | 25 % | 20 % |

Emulsions of any desired concentration can be prepared from such concentrates by dilution with water.

| Example F2: Solutions | a) | b) | c) | d) |
|---|---|---|---|---|
| Active ingredient | 80 % | 10 % | 5 % | 95 % |
| Ethylene glycol monomethyl ether | 20 % | - | - | - |
| Polyethylene glycol MW 400 | - | 70 % | - | - |
| N-Methylpyrrolid-2-one | - | 20 % | - | - |
| Epoxidized coconut oil | - | - | 1 % | 5 % |
| Petroleum ether (boiling range: 160-190°) | - | - | 94 % | - |
| The solutions are suitable for use in the form of microdrops. | | | | |

| Example F3: Granules | a) | b) | c) | d) |
|---|---|---|---|---|
| Active ingredient | 5 % | 10 % | 8 % | 21 % |
| Kaolin | 94 % | - | 79 % | 54 % |
| Highly disperse silica | 1 % | - | 13 % | 7 % |
| Attapulgite | - | 90 % | - | 18 % |

The active ingredient is dissolved in dichloromethane, the solution is sprayed onto the carrier(s), and the solvent is subsequently evaporated in vacuo.

| Example F4: Dusts | a) | b) |
|---|---|---|
| Active ingredient | 2 % | 5 % |
| Highly disperse silica | 1 % | 5 % |
| Talc | 97 % | - |
| Kaolin | - | 90 % |

Ready-to-use dusts are obtained by intimately mixing the carriers and the active ingredient.

| Example F5: Wettable powders | a) | b) | c) |
|---|---|---|---|
| Active ingredient | 25 % | 50 % | 75 % |
| Sodium lignosulfonate | 5 % | 5 % | - |
| Sodium lauryl sulfate | 3 % | - | 5 % |
| Sodium diisobutylnaphthalenesulfonate | - | 6 % | 10 % |
| Octylphenoxypolyethylene glycol ether (7-8 mol of EO) | - | 2 % | - |
| Highly disperse silica | 5 % | 10 % | 10 % |
| Kaolin | 62 % | 27 % | - |

The active ingredient is mixed with the additives and the mixture is ground thoroughly in a suitable mill. This gives wettable powders, which can be diluted with water to give suspensions of any desired concentration.

| Example F6: Extruder granules | |
|---|---|
| Active ingredient | 10 % |
| Sodium lignosulfonate | 2 % |
| Carboxymethylcellulose | 1 % |
| Kaolin | 87 % |

The active ingredient is mixed with the additives, and the mixture is ground, moistened with water, extruded, granulated and dried in a stream of air.

| Example F7: Coated granules | |
|---|---|
| Active ingredient | 3 % |
| Polyethylene glycol (MW 200) | 3 % |
| Kaolin | 94 % |

In a mixer, the finely ground active ingredient is applied uniformly to the kaolin, which has been moistened with the polyethylene glycol. This gives dust-free coated granules.

| Example F8a: Suspension concentrate | |
|---|---|
| Active ingredient | 40 % |
| Ethylene glycol | 10 % |
| Nonylphenoxypolyethylene glycol ether (15 mol of EO) | 6 % |
| Sodium lignosulfonate | 10 % |
| Carboxymethylcellulose | 1 % |
| 37 % aqueous formaldehyde solution | 0.2 % |
| Silicone oil (75 % aqueous emulsion) | 0.8 % |
| Water | 32 % |

| Example F8b: Suspension concentrate | |
|---|---|
| Active ingredient | 10% |
| Naphthalenesulfonic acid, sodium salt condensed with formaldehyde | 2% |
| Solution of an acrylic graft copolymer in water and propyleneglycole | 8% |
| Silicone antifoam emulsion | 0.5% |
| DL-propanediol-(1,2) | 3% |
| Heteropolysaccharide | 0.5% |
| 1,2-Benzisothiazol-3-one | 0.2% |
| Water | 75.8% |

The finely ground active ingredient is mixed intimately with the additives. Suspensions of any desired concentration can be prepared from the thus resulting suspension concentrate by dilution with water.

| Example F9: Powders for dry seed treatment | a) | b) | c) |
|---|---|---|---|
| active ingredient | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 % |

The active ingredient is thoroughly mixed with the adjuvants and the mixture is thoroughly ground in a suitable mill, affording powders that can be used directly for seed treatment.

| Example F10: Flowable concentrate for seed treatment | |
|---|---|
| active ingredient | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

| Example F11a: Oil-based suspension concentrate (based on a vegetable oil) | |
|---|---|
| Active ingredient | 10% |
| Tristyrylphenole with 16 moles EO | 10% |
| Block copolymer of polyhydroxystearic acid and polyalkylene glycols | 2% |
| AEROSIL 200 | 1% |
| Rape seed oil methyl ester | 12% |
| Oleic acid | 65% |

| Example F11b: Oil-based suspension concentrate (based on a mineral oil) | |
|---|---|
| Active ingredient | 10% |
| Ethoxylated alcohols, C16-18 and C18-unsatd | 5% |
| Dodecyl-benzene sulfonic acid Ca-salt linear | 2.5% |
| 2-Pyrrolidinone, 1-ethenylhexadecyl-, homopolymer | 1% |
| Organophilic clay | 1% |
| Mixture of petroleum | 80.5% |

The finely ground active ingredient is mixed intimately with the additives. Suspensions of any desired concentration can be prepared from the thus resulting suspension concentrate by dilution with water.

Preferably, the term "active ingredient" used above refers to one of the compounds selected from Tables 1 to 17 shown above. It also refers to mixtures of the compound of formula I, in particular a compound selected from said Tables 1 to 17, with other insecticides, fungicides, herbicides, safeners, adjuvants and the like, which mixtures are specifically disclosed below.

The compositions can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers; fertilizers, in particular nitrogen containing fertilizers such as ammonium nitrates and urea as described in WO08/017388, which can enhance the efficacy of the inventive compounds; or other active ingredients for achieving specific effects, for example ammonium or phosphonium salts, in particular halides, (hydrogen)sulphates, nitrates, (hydrogen)carbonates, citrates, tartrates, formiates and acetates, as described in WO07/068427 and WO07/068428, which also can enhance the efficacy of the inventive compounds and which can be used in combination with penetration enhancers such as alkoxalated fatty acids; bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest or weed in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compositions according to the invention are also suitable for the protection of plant propagation material, for example seeds, such as fruit, tubers or kernels, or nursery plants, against pests of the abovementioned type. The propagation material can be treated with the compositions prior to planting, for example seed can be treated prior to sowing. Alternatively, the compositions can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention.

Further methods of application of the compositions according to the invention comprise drip application onto the soil, dipping of parts of plants such as roots bulbs or tubers, drenching the soil, as well as soil injection. These methods are known in the art.

In order to apply a compound of formula I as an insecticide, acaricide, nematicide or molluscicide to a pest, a locus of pest, or to a plant susceptible to attack by a pest, a compound of formula I is usually formulated into a composition which includes, in addition to the compound of formula I, a suitable inert diluent or carrier and, optionally, a formulation adjuvant in form of a surface active agent (SFA) as described herein or, for example, in EP-B-1062217. SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula I. The composition is generally used for the control of pests such that a compound of formula I is applied at a rate of from 0.1 g to 10kg per hectare, preferably from 1 g to 6kg per hectare, more preferably from 1 g to 1 kg per hectare.

When used in a seed dressing, a compound of formula I is used at a rate of 0.0001 g to 10g (for example 0.001g or 0.05g), preferably 0.005g to 10g, more preferably 0.005g to 4g, per kilogram of seed.

In another aspect the present invention provides an insecticidal, acaricidal, nematicidal or molluscicidal composition comprising an insecticidally, acaricidally, nematicidally or molluscicidally effective amount of a compound of formula I and a suitable carrier or diluent therefor.

In a still further aspect the invention provides a method of combating and controlling pests at a locus which comprises treating the pests or the locus of the pests with an insecticidally, acaricidally, nematicidally or molluscicidally effective amount of a composition comprising a compound of formula I.

The compositions of the invention can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), oil-based suspension concentrate (OD), aerosols, fogging/smoke formulations, capsule suspensions (CS) and seed treatment formulations. The formulation type chosen in any instance will depend upon the particular purpose en-visaged and the physical, chemical and biological properties of the compound of formula I.

Dustable powders (DP) may be prepared by mixing a compound of formula I with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula I with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula I with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula I and one or more powdered solid diluents or carriers, or from pre-formed blank granules by absorbing a compound of formula I (or a solution thereof, in a suitable agent) in a porous granular material (such as pumice, attapulgite clays, fuller's earth, kieselguhr, diatomaceous earths or ground corn cobs) or by adsorbing a compound of formula I (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula I in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula I in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula I either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula I is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula I. SCs may be prepared by ball or bead milling the solid compound of formula I in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula I may be dry milled and added to water, containing agents hereinbefore described, to produce the desired end product.

Oil-based suspension concentrate (OD) may be prepared similarly by suspending finely divided insoluble solid particles of a compound of formula I in an organic fluid (for example at least one mineral oil or vegetable oil). ODs may further comprise at least one penetration promoter (for example an alcohol ethoxylate or a related compound), at least one non-ionic surfactants and/or at least one anionic surfactant, and optionally at least one additive from the group of emulsifiers, foam-inhibiting agents, preservatives, anti-oxidants, dyestuffs, and/or inert filler materials. An OD is intended and suitable for dilution with water before use to produce a spray solution with sufficient stability to allow spray application through appropriate equipment.

Aerosol formulations comprise a compound of formula I and a suitable propellant (for example n-butane). A compound of formula I may also be dissolved or dispersed in a suitable medium (for example water or a water miscible liquid, such as *n*-propanol) to provide compositions for use in non-pressurised, hand-actuated spray pumps.

A compound of formula I may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula I and, optionally, a carrier or diluent therefor. The polymeric shell may be produced by either an interfacial polycondensation reaction or by a coacervation procedure. The compositions may provide for controlled release of the compound of formula I and they may be used for seed treatment. A compound of formula I may also be formulated in a biodegradable polymeric matrix to provide a slow, controlled release of the compound.

A compound of formula I may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC, OD and DC compositions described above. Compositions for treating seed may include an agent for assisting the adhesion of the composition to the seed (for example a mineral oil or a film-forming barrier).

A composition of the present invention may include one or more additives to improve the biological performance of the composition (for example by improving wetting, retention or distribution on surfaces; resistance to rain on treated surfaces; or uptake or mobility of a compound of formula I). Such additives include surface active agents (SFAs), spray additives based on oils, for example certain mineral oils, vegetable oils or natural plant oils (such as soy bean and rape seed oil), and blends of these with other bio-enhancing adjuvants (ingredients which may aid or modify the action of a compound of formula I). Increasing the effect of a compound of formula I may for example be achieved by adding ammonium and/or phosphonium salts, and/or optionally at least one penetration promotor such as fatty alcohol alkoxylates (for example rape oil methyl ester) or vegetable oil esters.

Wetting agents, dispersing agents and emulsifying agents may be surface active agents (SFAs) of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula I may be applied by any of the known means of applying pesticidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula I may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ODs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula I (for example 0.0001 to 10%, by weight) depending upon the purpose for which they are to be used.

A compound of formula I may be used in mixtures with fertilisers (for example nitrogen-, potassium- or phosphorus-containing fertilisers, and more particularly ammonium nitrate and/or urea fertilizers). Suitable formulation types include granules of fertiliser. The mixtures suitably contain up to 25% by weight of the compound of formula I.

The invention therefore also provides a fertiliser composition comprising a fertiliser and a compound of formula I.

The compositions of this invention may contain other compounds having biological activity, for example micronutrients or compounds having fungicidal activity or which possess plant growth regulating, herbicidal, safening, insecticidal, nematicidal or acaricidal activity.

The compound of formula I may be the sole active ingredient of the composition or it may be admixed with one or more additional active ingredients such as a pesticide (insect, acarine, mollusc and nematode pesticide), fungicide, synergist, herbicide, safener or plant growth regulator where appropriate. The activity of the compositions according to the invention may thereby be broadened considerably and may have surprising advantages which can also be described, in a wider sense, as synergistic activity. An additional active ingredient may: provide a composition having a broader spectrum of activity or increased persistence at a locus; provide a composition demonstrating better plant/crop tolerance by reducing phytotoxicity; provide a composition controlling insects in their different development stages; synergise the activity or complement the activity (for example by increasing the speed of effect or overcoming repellency) of the compound of formula I; or help to overcome or prevent the development of resistance to individual components. The particular additional active ingredient will depend upon the intended utility of the composition.

Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1 R,3S)-2,2-dimethyl-3-(2-oxothiolan-3-ylidenemethyl)cyclopropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e) Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, or spinosad, spinetoram or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Neonicotinoid compounds such as imidacloprid, thiacloprid, acetamiprid, clothianidin, nitenpyram, dinotefuran or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine or pyrifluquinazon;
r) Spirotetramat, spirodiclofen or spiromesifen;
s) Flubendiamide, chloranthraliniprole, or cyanthraniliprole;
t) Cyenopyrafen or cyflumetofen; or
u) Sulfoxaflor.

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

The following mixtures of the compounds of formula I with active ingredients are preferred, wherein, preferably, the term "COMPOUND OF FORMULA I" refers to a compound selected from the Tables 1 to 17:
an adjuvant selected from the group of substances consisting of an oil of vegetable or animal origin, a mineral oil, alkyl esters of such oils or mixtures of such oils, and petroleum oils (alternative name) (628) + COMPOUND OF FORMULA I,
an acaricide selected from the group of substances consisting of 1,1-bis(4-chlorophenyl)-2-ethoxyethanol (IUPAC name) (910) + COMPOUND OF FORMULA I, 2,4-dichlorophenyl benzenesulfonate (IUPAC/Chemical Abstracts name) (1059) + COMPOUND OF FORMULA I, 2-fluoro-*N*-methyl-*N*-1-naphthylacetamide (IUPAC name) (1295) + COMPOUND OF FORMULA I, 4-chlorophenyl phenyl sulfone (IUPAC name) (981) + COMPOUND OF FORMULA I, abamectin (1) + COMPOUND OF FORMULA I, acequinocyl (3) + COMPOUND OF FORMULA I, acetoprole [CCN] + COMPOUND OF FORMULA I, acrinathrin (9) + COMPOUND OF FORMULA I, aldicarb (16) + COMPOUND OF FORMULA I, aldoxycarb (863) + COMPOUND OF FORMULA I, alpha-cypermethrin (202) + COMPOUND OF FORMULA I, amidithion (870) + COMPOUND OF FORMULA I, amidoflumet [CCN] + COMPOUND OF FORMULA I, amidothioate (872) + COMPOUND OF FORMULA I, amiton (875) + COMPOUND OF FORMULA I, amiton hydrogen oxalate (875) + COMPOUND OF FORMULA I, amitraz (24) + COMPOUND OF FORMULA I, aramite (881) + COMPOUND OF FORMULA I, arsenous oxide (882) + COMPOUND OF FORMULA I, AVI 382 (compound code) + COMPOUND OF FORMULA I, AZ 60541 (compound code) + COMPOUND OF FORMULA I, azinphos-ethyl (44) + COMPOUND OF FORMULA I, azinphos-methyl (45) + COMPOUND OF FORMULA I, azobenzene (IUPAC name) (888) + COMPOUND OF FORMULA I, azocyclotin (46) + COMPOUND OF FORMULA I, azothoate (889) + COMPOUND OF FORMULA I, benomyl (62) + COMPOUND OF FORMULA I, benoxafos (alternative name) [CCN] + COMPOUND OF FORMULA I, benzoximate (71) + COMPOUND OF FORMULA I, benzyl benzoate (IUPAC name) [CCN] + COMPOUND OF FORMULA I, bifenazate (74) + COMPOUND OF FORMULA I, bifenthrin (76) + COMPOUND OF FORMULA I, binapacryl (907) + COMPOUND OF FORMULA I, brofenvalerate (alternative name) + COMPOUND OF FORMULA I, bromocyclen (918) + COMPOUND OF FORMULA I, bromophos (920) + COMPOUND OF FORMULA I, bromophos-ethyl (921) + COMPOUND OF FORMULA I, bromopropylate (94) + COMPOUND OF FORMULA I, buprofezin (99) + COMPOUND OF FORMULA I, butocarboxim (103) + COMPOUND OF FORMULA I, butoxycarboxim (104) + COMPOUND OF FORMULA I, butylpyridaben (alternative name) + COMPOUND OF FORMULA I, calcium polysulfide (IUPAC name) (111) + COMPOUND OF FORMULA I, camphechlor (941) + COMPOUND OF FORMULA I, carbanolate (943) + COMPOUND OF FORMULA I, carbaryl (115) + COMPOUND OF FORMULA I, carbofuran (118) + COMPOUND OF FORMULA I, carbophenothion (947) + COMPOUND OF FORMULA I, CGA 50'439 (development code) (125) + COMPOUND OF FORMULA I, chinomethionat (126) + COMPOUND OF FORMULA I, chlorbenside (959) + COMPOUND OF FORMULA I, chlordimeform (964) + COMPOUND OF FORMULA I, chlordimeform hydrochloride (964) + COMPOUND OF FORMULA I, chlorfenapyr (130) + COMPOUND OF FORMULA I, chlorfenethol (968) + COMPOUND OF FORMULA I, chlorfenson (970) + COMPOUND OF FORMULA I, chlorfensulphide (971) + COMPOUND OF FORMULA I, chlorfenvinphos (131) + COMPOUND OF FORMULA I, chlorobenzilate (975) + COMPOUND OF FORMULA I, chloromebuform (977) + COMPOUND OF FORMULA I, chloromethiuron (978) + COMPOUND OF FORMULA I, chloropropylate (983) + COMPOUND OF FORMULA I, chlorpyrifos (145) + COMPOUND OF FORMULA I, chlorpyrifos-methyl (146) + COMPOUND OF FORMULA I, chlorthiophos (994) + COMPOUND OF FORMULA I, cinerin I (696) + COMPOUND OF FORMULA I, cinerin II (696) + COMPOUND OF FORMULA I, cinerins (696) + COMPOUND OF FORMULA I, clofentezine (158) + COMPOUND OF FORMULA I, closantel (alternative name) [CCN] + COMPOUND OF FORMULA I, coumaphos (174) + COMPOUND OF FORMULA I, crotamiton (alternative name) [CCN] + COMPOUND OF FORMULA I, crotoxyphos (1010) + COMPOUND OF FORMULA I, cufraneb (1013) + COMPOUND OF FORMULA I, cyanthoate (1020) + COMPOUND OF FORMULA I, cyenopyrafen [CCN] + COMPOUND OF FORMULA I, cyflumetofen (CAS Reg. No.: 400882-07-7) + COMPOUND OF FORMULA I, cyhalothrin (196) + COMPOUND OF FORMULA I, cyhexatin (199) + COMPOUND OF FORMULA I, cypermethrin (201) + COMPOUND OF FORMULA I, DCPM (1032) + COMPOUND OF FORMULA I, DDT (219) + COMPOUND OF FORMULA I, demephion (1037) + COMPOUND OF FORMULA I, demephion-O (1037) + COMPOUND OF FORMULA I, demephion-S (1037) + COMPOUND OF FORMULA I, demeton (1038) + COMPOUND OF FORMULA I, demeton-methyl (224) + COMPOUND OF FORMULA I, demeton-O (1038) + COMPOUND OF FORMULA I, demeton-O-methyl (224) + COMPOUND OF FORMULA I, demeton-S (1038) + COMPOUND OF FORMULA I, demeton-S-methyl (224) + COMPOUND OF FORMULA I, demeton-S-methylsulphon (1039) + COMPOUND OF FORMULA I, diafenthiuron (226) + COMPOUND OF FORMULA I, dialifos (1042) + COMPOUND OF FORMULA I, diazinon (227) + COMPOUND OF FORMULA I, dichlofluanid (230) + COMPOUND OF FORMULA I, dichlorvos (236) + COMPOUND OF FORMULA I, dicliphos (alternative name) + COMPOUND OF FORMULA I, dicofol (242) + COMPOUND OF FORMULA I, dicrotophos (243) + COMPOUND OF FORMULA I, dienochlor (1071) + COMPOUND OF FORMULA I, diflovidazin [CCN] + COMPOUND OF FORMULA I, dimefox (1081) + COMPOUND OF FORMULA I, dimethoate (262) + COMPOUND OF FORMULA I, dinactin (alternative name) (653) + COMPOUND OF FORMULA I, dinex (1089) + COMPOUND OF FORMULA I, dinex-diclexine (1089) + COMPOUND OF FORMULA I, dinobuton (269) + COMPOUND OF FORMULA I, dinocap (270) + COMPOUND OF FORMULA I, dinocap-4 [CCN] + COMPOUND OF FORMULA I, dinocap-6 [CCN] + COMPOUND OF FORMULA I, dinocton (1090) + COMPOUND OF FORMULA I, dinopenton (1092) + COMPOUND OF FORMULA I, dinosulfon (1097) + COMPOUND OF FORMULA I, dinoterbon (1098) + COMPOUND OF FORMULA I, dioxathion (1102) + COMPOUND OF FORMULA I, diphenyl sulfone (IUPAC name) (1103) + COMPOUND OF FORMULA I, disulfiram (alternative name) [CCN] + COMPOUND OF FORMULA I, disulfoton (278) + COMPOUND OF FORMULA I, DNOC (282) + COMPOUND OF FORMULA I, dofenapyn (1113) + COMPOUND OF FORMULA I, doramectin (alternative name) [CCN] + COMPOUND OF FORMULA I, endosulfan (294) + COMPOUND OF FORMULA I, endothion (1121) + COMPOUND OF FORMULA I, EPN (297) + COMPOUND OF FORMULA I, eprinomectin (alternative name) [CCN] + COMPOUND OF FORMULA I, ethion (309) + COMPOUND OF FORMULA I, ethoate-methyl (1134) + COMPOUND OF FORMULA I, etoxazole (320) + COMPOUND OF FORMULA I, etrimfos (1142) + COMPOUND OF FORMULA I, fenazaflor (1147) + COMPOUND OF FORMULA I, fenazaquin (328) + COMPOUND OF FORMULA I, fenbutatin oxide (330) + COMPOUND OF FORMULA I, fenothiocarb (337) + COMPOUND OF FORMULA I, fenpropathrin (342) + COMPOUND OF FORMULA I, fenpyrad (alternative name) + COMPOUND OF FORMULA I, fenpyroximate (345) + COMPOUND OF FORMULA I, fenson (1157) + COMPOUND OF FORMULA I, fentrifanil (1161) + COMPOUND OF FORMULA I, fenvalerate (349) + COMPOUND OF FORMULA I, fipronil (354) + COMPOUND OF FORMULA I, fluacrypyrim (360) + COMPOUND OF FORMULA I, fluazuron (1166) + COMPOUND OF FORMULA I, flubenzimine (1167) + COMPOUND OF FORMULA I, flucycloxuron (366) + COMPOUND OF FORMULA I, flucythrinate (367) + COMPOUND OF FORMULA I, fluenetil (1169) + COMPOUND OF FORMULA I, flufenoxuron (370) + COMPOUND OF FORMULA I, flumethrin (372) + COMPOUND OF FORMULA I, fluorbenside (1174) + COMPOUND OF FORMULA I, fluvalinate (1184) + COMPOUND OF FORMULA I, FMC 1137 (development code) (1185) + COMPOUND OF FORMULA I, formetanate (405) + COMPOUND OF FORMULA I, formetanate hydrochloride (405) + COMPOUND OF FORMULA I, formothion (1192) + COMPOUND OF FORMULA I, formparanate (1193) + COMPOUND OF FORMULA I, gamma-HCH (430) + COMPOUND OF FORMULA I, glyodin (1205) + COMPOUND OF FORMULA I, halfenprox (424) + COMPOUND OF FORMULA I, heptenophos (432) + COMPOUND OF FORMULA I, hexadecyl cyclopropanecarboxylate (IUPAC/Chemical Abstracts name) (1216) + COMPOUND OF FORMULA I, hexythiazox (441) + COMPOUND OF FORMULA I, IKA 2002 (CAS Reg. No.: 211923-74-9) + COMPOUND OF FORMULA I, iodomethane (IUPAC name) (542) + COMPOUND OF FORMULA I, isocarbophos (alternative name) (473) + COMPOUND OF FORMULA I, isopropyl O-(methoxyaminothiophosphoryl)salicylate (IUPAC name) (473) + COMPOUND OF FORMULA I, ivermectin (alternative name) [CCN] + COMPOUND OF FORMULA I, jasmolin I (696) + COMPOUND OF FORMULA I, jasmolin II (696) + COMPOUND OF FORMULA I, jodfenphos (1248) + COMPOUND OF FORMULA I, lindane (430) + COMPOUND OF FORMULA I, lufenuron (490) + COMPOUND OF FORMULA I, malathion (492) + COMPOUND OF FORMULA I, malonoben (1254) + COMPOUND OF FORMULA I, mecarbam (502) + COMPOUND OF FORMULA I, mephosfolan (1261) + COMPOUND OF FORMULA I, mesulfen (alternative name) [CCN] + COMPOUND OF FORMULA I, methacrifos (1266) + COMPOUND OF FORMULA I, methamidophos (527) + COMPOUND OF FORMULA I, methidathion (529) + COMPOUND OF FORMULA I, methiocarb (530) + COMPOUND OF FORMULA I, methomyl (531) + COMPOUND OF FORMULA I, methyl bromide (537) + COMPOUND OF FORMULA I, metolcarb (550) + COMPOUND OF FORMULA I, mevinphos (556) + COMPOUND OF FORMULA I, mexacarbate (1290) + COMPOUND OF FORMULA I, milbemectin (557) + COMPOUND OF FORMULA I, milbemycin oxime (alternative name) [CCN] + COMPOUND OF FORMULA I, mipafox (1293) + COMPOUND OF FORMULA I, monocrotophos (561) + COMPOUND OF FORMULA I, morphothion (1300) + COMPOUND OF FORMULA I, moxidectin (alternative name) [CCN] + COMPOUND OF FORMULA I, naled (567) + COMPOUND OF FORMULA I, NC-184 (compound code) + COMPOUND OF FORMULA I, NC-512 (compound code) + COMPOUND OF FORMULA I, nifluridide (1309) + COMPOUND OF FORMULA I, nikkomycins (alternative name) [CCN] + COMPOUND OF FORMULA I, nitrilacarb (1313) + COMPOUND OF FORMULA I, nitrilacarb 1:1 zinc chloride complex (1313) + COMPOUND OF FORMULA I, NNI-0101 (compound code) + COMPOUND OF FORMULA I, NNI-0250 (compound code) + COMPOUND OF FORMULA I, omethoate (594) + COMPOUND OF FORMULA I, oxamyl (602) + COMPOUND OF FORMULA I, oxydeprofos (1324) + COMPOUND OF FORMULA I, oxydisulfoton (1325) + COMPOUND OF FORMULA I, pp'-DDT (219) + COMPOUND OF FORMULA I, parathion (615) + COMPOUND OF FORMULA I, permethrin (626) + COMPOUND OF FORMULA I, petroleum oils (alternative name) (628) + COMPOUND OF FORMULA I, phenkapton (1330) + COMPOUND OF FORMULA I, phenthoate (631) + COMPOUND OF FORMULA I, phorate (636) + COMPOUND OF FORMULA I, phosalone (637) + COMPOUND OF FORMULA I, phosfolan (1338) + COMPOUND OF FORMULA I, phosmet (638) + COMPOUND OF FORMULA I, phosphamidon (639) + COMPOUND OF FORMULA I, phoxim (642) + COMPOUND OF FORMULA I, pirimiphos-methyl (652) + COMPOUND OF FORMULA I, polychloroterpenes (traditional name) (1347) + COMPOUND OF FORMULA I, polynactins (alternative name) (653) + COMPOUND OF FORMULA I, proclonol (1350) + COMPOUND OF FORMULA I, profenofos (662) + COMPOUND OF FORMULA I, promacyl (1354) + COMPOUND OF FORMULA I, propargite (671) + COMPOUND OF FORMULA I, propetamphos (673) + COMPOUND OF FORMULA I, propoxur (678) + COMPOUND OF FORMULA I, prothidathion (1360) + COMPOUND OF FORMULA I, prothoate (1362) + COMPOUND OF FORMULA I, pyflubumide + COMPOUND OF FORMULA I, pyrethrin I (696) + COMPOUND OF FORMULA I, pyrethrin II (696) + COMPOUND OF FORMULA I, pyrethrins (696) + COMPOUND OF FORMULA I, pyridaben (699) + COMPOUND OF FORMULA I, pyridaphenthion (701) + COMPOUND OF FORMULA I, pyrimidifen (706) + COMPOUND OF FORMULA I, pyrimitate (1370) + COMPOUND OF FORMULA I, quinalphos (711) + COMPOUND OF FORMULA I, quintiofos (1381) + COMPOUND OF FORMULA I, R-1492 (development code) (1382) + COMPOUND OF FORMULA I, RA-17 (development code) (1383) + COMPOUND OF FORMULA I, rotenone (722) + COMPOUND OF FORMULA I, schradan (1389) + COMPOUND OF FORMULA I, sebufos (alternative name) + COMPOUND OF FORMULA I, selamectin (alternative name) [CCN] + COMPOUND OF FORMULA I, SI-0009 (compound code) + COMPOUND OF FORMULA I, sophamide (1402) + COMPOUND OF FORMULA I, spirodiclofen (738) + COMPOUND OF FORMULA I, spiromesifen (739) + COMPOUND OF FORMULA I, SSI-121 (development code) (1404) + COMPOUND OF FORMULA I, sulfiram (alternative name) [CCN] + COMPOUND OF FORMULA I, sulfluramid (750) + COMPOUND OF FORMULA I, sulfotep (753) + COMPOUND OF FORMULA I, sulfur (754) + COMPOUND OF FORMULA I, SZI-121 (development code) (757) + COMPOUND OF FORMULA I, tau-fluvalinate (398) + COMPOUND OF FORMULA I, tebufenpyrad (763) + COMPOUND OF FORMULA I, TEPP (1417) + COMPOUND OF FORMULA I, terbam (alternative name) + COMPOUND OF FORMULA I, tetrachlorvinphos (777) + COMPOUND OF FORMULA I, tetradifon (786) + COMPOUND OF FORMULA I, tetranactin (alternative name) (653) + COMPOUND OF FORMULA I, tetrasul (1425) + COMPOUND OF FORMULA I, thiafenox (alternative name) + COMPOUND OF FORMULA I, thiocarboxime (1431) + COMPOUND OF FORMULA I, thiofanox (800) + COMPOUND OF FORMULA I, thiometon (801) + COMPOUND OF FORMULA I, thioquinox (1436) + COMPOUND OF FORMULA I, thuringiensin (alternative name) [CCN] + COMPOUND OF FORMULA I, triamiphos (1441) + COMPOUND OF FORMULA I, triarathene (1443) + COMPOUND OF FORMULA I, triazophos (820) + COMPOUND OF FORMULA I, triazuron (alternative name) + COMPOUND OF FORMULA I, trichlorfon (824) + COMPOUND OF FORMULA I, trifenofos (1455) + COMPOUND OF FORMULA I, trinactin (alternative name) (653) + COMPOUND OF FORMULA I, vamidothion (847) + COMPOUND OF FORMULA I, vaniliprole [CCN] and YI-5302 (compound code) + COMPOUND OF FORMULA I,
   an algicide selected from the group of substances consisting of bethoxazin [CCN] + COMPOUND OF FORMULA I, copper dioctanoate (IUPAC name) (170) + COMPOUND OF FORMULA I, I, copper sulfate (172) + COMPOUND OF FORMULA I, cybutryne [CCN] + COMPOUND OF FORMULA I, dichlone (1052) + COMPOUND OF FORMULA I, dichlorophen (232) + COMPOUND OF FORMULA I, endothal (295) + COMPOUND OF FORMULA I, fentin (347) + COMPOUND OF FORMULA I, hydrated lime [CCN] + COMPOUND OF FORMULA I, nabam (566) + COMPOUND OF FORMULA I, quinoclamine (714) + COMPOUND OF FORMULA I, quinonamid (1379) + COMPOUND OF FORMULA I, simazine (730) + COMPOUND OF FORMULA I, triphenyltin acetate (IUPAC name) (347) and triphenyltin hydroxide (IUPAC name) (347) + COMPOUND OF FORMULA I,
an anthelmintic selected from the group of substances consisting of abamectin (1) + COMPOUND OF FORMULA I, crufomate (1011) + COMPOUND OF FORMULA I, doramectin (alternative name) [CCN] + COMPOUND OF FORMULA I, emamectin (291) + COMPOUND OF FORMULA I, emamectin benzoate (291) + COMPOUND OF FORMULA I, eprinomectin (alternative name) [CCN] + COMPOUND OF FORMULA I, ivermectin (alternative name) [CCN] + COMPOUND OF FORMULA I, milbemycin oxime (alternative name) [CCN] + COMPOUND OF FORMULA I, moxidectin (alternative name) [CCN] + COMPOUND OF FORMULA I, piperazine [CCN] + COMPOUND OF FORMULA I, selamectin (alternative name) [CCN] + COMPOUND OF FORMULA I, spinosad (737) and thiophanate (1435) + COMPOUND OF FORMULA I,
an avicide selected from the group of substances consisting of chloralose (127) + COMPOUND OF FORMULA I, endrin (1122) + COMPOUND OF FORMULA I, fenthion (346) + COMPOUND OF FORMULA I, pyridin-4-amine (IUPAC name) (23) and strychnine (745) + COMPOUND OF FORMULA I,
a bactericide selected from the group of substances consisting of 1-hydroxy-1H-pyridine-2-thione (IUPAC name) (1222) + COMPOUND OF FORMULA I, 4-(quinoxalin-2-ylamino)benzenesulfonamide (IUPAC name) (748) + COMPOUND OF FORMULA I, 8-hydroxyquinoline sulfate (446) + COMPOUND OF FORMULA I, bronopol (97) + COMPOUND OF FORMULA I, copper dioctanoate (IUPAC name) (170) + COMPOUND OF FORMULA I, copper hydroxide (IUPAC name) (169) + COMPOUND OF FORMULA I, cresol [CCN] + COMPOUND OF FORMULA I, dichlorophen (232) + COMPOUND OF FORMULA I, dipyrithione (1105) + COMPOUND OF FORMULA I, dodicin (1112) + COMPOUND OF FORMULA I, fenaminosulf (1144) + COMPOUND OF FORMULA I, formaldehyde (404) + COMPOUND OF FORMULA I, hydrargaphen (alternative name) [CCN] + COMPOUND OF FORMULA I, kasugamycin (483) + COMPOUND OF FORMULA I, kasugamycin hydrochloride hydrate (483) + COMPOUND OF FORMULA I, nickel bis(dimethyldithiocarbamate) (IUPAC name) (1308) + COMPOUND OF FORMULA I, nitrapyrin (580) + COMPOUND OF FORMULA I, octhilinone (590) + COMPOUND OF FORMULA I, oxolinic acid (606) + COMPOUND OF FORMULA I, oxytetracycline (611) + COMPOUND OF FORMULA I, potassium hydroxyquinoline sulfate (446) + COMPOUND OF FORMULA I, probenazole (658) + COMPOUND OF FORMULA I, streptomycin (744) + COMPOUND OF FORMULA I, streptomycin sesquisulfate (744) + COMPOUND OF FORMULA I, tecloftalam (766) + COMPOUND OF FORMULA I, and thiomersal (alternative name) [CCN] + COMPOUND OF FORMULA I,
a biological agent selected from the group of substances consisting of *Adoxophyes orana* GV (alternative name) (12) + COMPOUND OF FORMULA I, *Agrobacterium radiobacter* (alternative name) (13) + COMPOUND OF FORMULA I, *Amblyseius* spp.
(alternative name) (19) + COMPOUND OF FORMULA I, *Anagrapha falcifera* NPV (alternative name) (28) + COMPOUND OF FORMULA I, *Anagrus atomus* (alternative name) (29) + COMPOUND OF FORMULA I, *Aphelinus abdominalis* (alternative name) (33) + COMPOUND OF FORMULA I, *Aphidius colemani* (alternative name) (34) + COMPOUND OF FORMULA I, *Aphidoletes aphidimyza* (alternative name) (35) + COMPOUND OF FORMULA I, *Autographa californica* NPV (alternative name) (38) + COMPOUND OF FORMULA I, *Bacillus firmus* (alternative name) (48) + COMPOUND OF FORMULA I, *Bacillus sphaericus* Neide (scientific name) (49) + COMPOUND OF FORMULA I, *Bacillus thuringiensis* Berliner (scientific name) (51) + COMPOUND OF FORMULA I, *Bacillus thuringiensis* subsp. *aizawai* (scientific name) (51) + COMPOUND OF FORMULA I, *Bacillus thuringiensis subsp. israelensis* (scientific name) (51) + COMPOUND OF FORMULA I, *Bacillus thuringiensis subsp. japonensis* (scientific name) (51) + COMPOUND OF FORMULA I, *Bacillus thuringiensis* subsp. *kurstaki* (scientific name) (51) + COMPOUND OF FORMULA I, *Bacillus thuringiensis* subsp. *tenebrionis* (scientific name) (51) + COMPOUND OF FORMULA I, *Beauveria bassiana* (alternative name) (53) + COMPOUND OF FORMULA I, *Beauveria brongniartii* (alternative name) (54) + COMPOUND OF FORMULA I, *Chrysoperla carnea* (alternative name) (151) + COMPOUND OF FORMULA I, *Cryptolaemus montrouzieri* (alternative name) (178) + COMPOUND OF FORMULA I, *Cydia pomonella* GV (alternative name) (191) + COMPOUND OF FORMULA I, *Dacnusa sibirica* (alternative name) (212) + COMPOUND OF FORMULA I, *Diglyphus isaea* (alternative name) (254) + COMPOUND OF FORMULA *I, Encarsia formosa* (scientific name) (293) + COMPOUND OF FORMULA I, *Eretmocerus eremicus* (alternative name) (300) + COMPOUND OF FORMULA I, *Helicoverpa zea NPV* (alternative name) (431) + COMPOUND OF FORMULA I, *Heterorhabditis bacteriophora* and *H. megidis* (alternative name) (433) + COMPOUND OF FORMULA I, *Hippodamia convergens* (alternative name) (442) + COMPOUND OF FORMULA I, *Leptomastix dactylopii* (alternative name) (488) + COMPOUND OF FORMULA I, *Macrolophus caliginosus* (alternative name) (491) + COMPOUND OF FORMULA I, *Mamestra brassicae* NPV (alternative name) (494) + COMPOUND OF FORMULA I, *Metaphycus helvolus* (alternative name) (522) + COMPOUND OF FORMULA I, *Metarhizium anisopliae* var. *acridum* (scientific name) (523) + COMPOUND OF FORMULA I, *Metarhizium anisopliae* var. *anisopliae* (scientific name) (523) + COMPOUND OF FORMULA I, *Neodiprion sertifer* NPV and *N. lecontei* NPV (alternative name) (575) + COMPOUND OF FORMULA I, *Orius* spp. (alternative name) (596) + COMPOUND OF FORMULA I, *Pasteuria usgae* (alternative name) + COMPOUND OF FORMULA I, *Paecilomyces fumosoroseus* (alternative name) (613) + COMPOUND OF FORMULA I, *Phytoseiulus persimilis* (alternative name) (644) + COMPOUND OF FORMULA I, *Spodoptera exigua* multicapsid nuclear polyhedrosis virus (scientific name) (741) + COMPOUND OF FORMULA I, *Steinernema bibionis* (alternative name) (742) + COMPOUND OF FORMULA I, *Steinernema carpocapsae* (alternative name) (742) + COMPOUND OF FORMULA I, *Steinernema feltiae* (alternative name) (742) + COMPOUND OF FORMULA I, *Steinernema glaseri* (alternative name) (742) + COMPOUND OF FORMULA I, *Steinernema riobrave* (alternative name) (742) + COMPOUND OF FORMULA I, *Steinernema riobravis* (alternative name) (742) + COMPOUND OF FORMULA I, *Steinernema scapterisci* (alternative name) (742) + COMPOUND OF FORMULA I, *Steinernema* spp. (alternative name) (742) + COMPOUND OF FORMULA I, *Trichoderma* spp. (alternative name) + COMPOUND OF FORMULA I, *Trichogramma* spp. (alternative name) (826) + COMPOUND OF FORMULA I, *Typhlodromus occidentalis* (alternative name) (844) and *Verticillium lecanii* (alternative name) (848) + COMPOUND OF FORMULA I,
a soil sterilant selected from the group of substances consisting of dimethyl disulfide (IUPAC name) + COMPOUND OF FORMULA I, iodomethane (IUPAC name) (542) and methyl bromide (537) + COMPOUND OF FORMULA I,
a chemosterilant selected from the group of substances consisting of apholate [CCN] + COMPOUND OF FORMULA I, bisazir (alternative name) [CCN] + COMPOUND OF FORMULA I, busulfan (alternative name) [CCN] + COMPOUND OF FORMULA I, diflubenzuron (250) + COMPOUND OF FORMULA I, dimatif (alternative name) [CCN] + COMPOUND OF FORMULA I, hemel [CCN] + COMPOUND OF FORMULA I, hempa [CCN] + COMPOUND OF FORMULA I, metepa [CCN] + COMPOUND OF FORMULA I, methiotepa [CCN] + COMPOUND OF FORMULA I, methyl apholate [CCN] + COMPOUND OF FORMULA I, morzid [CCN] + COMPOUND OF FORMULA I, penfluron (alternative name) [CCN] + COMPOUND OF FORMULA I, tepa [CCN] + COMPOUND OF FORMULA I, thiohempa (alternative name) [CCN] + COMPOUND OF FORMULA I, thiotepa (alternative name) [CCN] + COMPOUND OF FORMULA I, tretamine (alternative name) [CCN] and uredepa (alternative name) [CCN] + COMPOUND OF FORMULA I,
an insect pheromone selected from the group of substances consisting of (*E*)-dec-5-en-1-yl acetate with (*E*)-dec-5-en-1-ol (IUPAC name) (222) + COMPOUND OF FORMULA I, (*E*)-tridec-4-en-1-yl acetate (IUPAC name) (829) + COMPOUND OF FORMULA I, (*E*)-6-methylhept-2-en-4-ol (IUPAC name) (541) + COMPOUND OF FORMULA I, (*E*,*Z*)-tetradeca-4,10-dien-1-yl acetate (IUPAC name) (779) + COMPOUND OF FORMULA I, (*Z*)-dodec-7-en-1-yl acetate (IUPAC name) (285) + COMPOUND OF FORMULA I, (*Z*)-hexadec-11-enal (IUPAC name) (436) + COMPOUND OF FORMULA I, (Z)-hexadec-11-en-1-yl acetate (IUPAC name) (437) + COMPOUND OF FORMULA I, (Z)-hexadec-13-en-11-yn-1-yl acetate (IUPAC name) (438) + COMPOUND OF FORMULA I, (Z)-icos-13-en-10-one (IUPAC name) (448) + COMPOUND OF FORMULA I, (Z)-tetradec-7-en-1-al (IUPAC name) (782) + COMPOUND OF FORMULA I, (Z)-tetradec-9-en-1-ol (IUPAC name) (783) + COMPOUND OF FORMULA I, (Z)-tetradec-9-en-1-yl acetate (IUPAC name) (784) + COMPOUND OF FORMULA I, (7E,9Z)-dodeca-7,9-dien-1-yl acetate (IUPAC name) (283) + COMPOUND OF FORMULA I, (9Z,11E)-tetradeca-9,11-dien-1-yl acetate (IUPAC name) (780) + COMPOUND OF FORMULA I, (9Z,12E)-tetradeca-9,12-dien-1-yl acetate (IUPAC name) (781) + COMPOUND OF FORMULA I, 14-methyloctadec-1-ene (IUPAC name) (545) + COMPOUND OF FORMULA I, 4-methylnonan-5-ol with 4-methylnonan-5-one (IUPAC name) (544) + COMPOUND OF FORMULA I, alpha-multistriatin (alternative name) [CCN] + COMPOUND OF FORMULA I, brevicomin (alternative name) [CCN] + COMPOUND OF FORMULA I, codlelure (alternative name) [CCN] + COMPOUND OF FORMULA I, codlemone (alternative name) (167) + COMPOUND OF FORMULA I, cuelure (alternative name) (179) + COMPOUND OF FORMULA I, disparlure (277) + COMPOUND OF FORMULA I, (E,Z)-7,9-dodecadien-1-yl acetate (IUPAC name) + COMPOUND OF FORMULA I, dodec-8-en-1-yl acetate (IUPAC name) (286) + COMPOUND OF FORMULA I, dodec-9-en-1-yl acetate (IUPAC name) (287) + COMPOUND OF FORMULA I, dodeca-8 + COMPOUND OF FORMULA I, 10-dien-1-yl acetate (IUPAC name) (284) + COMPOUND OF FORMULA I, dominicalure (alternative name) [CCN] + COMPOUND OF FORMULA I, ethyl 4-methyloctanoate (IUPAC name) (317) + COMPOUND OF FORMULA I, eugenol (alternative name) [CCN] + COMPOUND OF FORMULA I, exosex SPTab (alternative name) + COMPOUND OF FORMULA I, frontalin (alternative name) [CCN] + COMPOUND OF FORMULA I, gossyplure (alternative name) (420) + COMPOUND OF FORMULA I, grandlure (421) + COMPOUND OF FORMULA I, grandlure I (alternative name) (421) + COMPOUND OF FORMULA I, grandlure II (alternative name) (421) + COMPOUND OF FORMULA I, grandlure III (alternative name) (421) + COMPOUND OF FORMULA I, grandlure IV (alternative name) (421) + COMPOUND OF FORMULA I, hexalure [CCN] + COMPOUND OF FORMULA I, imicyafos (alternative name) [CCN] + COMPOUND OF FORMULA I, ipsdienol (alternative name) [CCN] + COMPOUND OF FORMULA I, ipsenol (alternative name) [CCN] + COMPOUND OF FORMULA I, japonilure (alternative name) (481) + COMPOUND OF FORMULA I, lineatin (alternative name) [CCN] + COMPOUND OF FORMULA I, litlure (alternative name) [CCN] + COMPOUND OF FORMULA I, looplure (alternative name) [CCN] + COMPOUND OF FORMULA I, medlure [CCN] + COMPOUND OF FORMULA I, megatomoic acid (alternative name) [CCN] + COMPOUND OF FORMULA I, methyl eugenol (alternative name) (540) + COMPOUND OF FORMULA I, muscalure (563) + COMPOUND OF FORMULA I, octadeca-2,13-dien-1-yl acetate (IUPAC name) (588) + COMPOUND OF FORMULA I, octadeca-3,13-dien-1-yl acetate (IUPAC name) (589) + COMPOUND OF FORMULA I, orfralure (alternative name) [CCN] + COMPOUND OF FORMULA I, oryctalure (alternative name) (317) + COMPOUND OF FORMULA I, ostramone (alternative name) [CCN] + COMPOUND OF FORMULA I, siglure [CCN] + COMPOUND OF FORMULA I, sordidin (alternative name) (736) + COMPOUND OF FORMULA I, sulcatol (alternative name) [CCN] + COMPOUND OF FORMULA I, tetradec-11-en-1-yl acetate (IUPAC name) (785) + COMPOUND OF FORMULA I, trimedlure (839) + COMPOUND OF FORMULA I, trimedlure A (alternative name) (839) + COMPOUND OF FORMULA I, trimedlure B₁ (alternative name) (839) + COMPOUND OF FORMULA I, trimedlure B₂ (alternative name) (839) + COMPOUND OF FORMULA I, trimedlure C (alternative name) (839) and trunc-call (alternative name) [CCN] + COMPOUND OF FORMULA I,
an insect repellent selected from the group of substances consisting of 2-(octylthio)-ethanol (IUPAC name) (591) + COMPOUND OF FORMULA I, butopyronoxyl (933) + COMPOUND OF FORMULA I, butoxy(polypropylene glycol) (936) + COMPOUND OF FORMULA I, dibutyl adipate (IUPAC name) (1046) + COMPOUND OF FORMULA I, dibutyl phthalate (1047) + COMPOUND OF FORMULA I, dibutyl succinate (IUPAC name) (1048) + COMPOUND OF FORMULA I, diethyltoluamide [CCN] + COMPOUND OF FORMULA I, dimethyl carbate [CCN] + COMPOUND OF FORMULA I, dimethyl phthalate [CCN] + COMPOUND OF FORMULA I, ethyl hexanediol (1137) + COMPOUND OF FORMULA I, hexamide [CCN] + COMPOUND OF FORMULA I, methoquin-butyl (1276) + COMPOUND OF FORMULA I, methylneodecanamide [CCN] + COMPOUND OF FORMULA I, oxamate [CCN] and picaridin [CCN] + COMPOUND OF FORMULA I,
an insecticide selected from the group of substances consisting of 1-dichloro-1-nitroethane (IUPAC/Chemical Abstracts name) (1058) + COMPOUND OF FORMULA I, 1,1-dichloro-2,2-bis(4-ethylphenyl)ethane (IUPAC name) (1056), + COMPOUND OF FORMULA I, 1,2-dichloropropane (IUPAC/Chemical Abstracts name) (1062) + COMPOUND OF FORMULA I, 1,2-dichloropropane with 1,3-dichloropropene (IUPAC name) (1063) + COMPOUND OF FORMULA I, 1-bromo-2-chloroethane (IUPAC/Chemical Abstracts name) (916) + COMPOUND OF FORMULA I, 2,2,2-trichloro-1-(3,4-dichlorophenyl)ethyl acetate (IUPAC name) (1451) + COMPOUND OF FORMULA I, 2,2-dichlorovinyl 2-ethylsulfinylethyl methyl phosphate (IUPAC name) (1066) + COMPOUND OF FORMULA I, 2-(1,3-dithiolan-2-yl)phenyl dimethylcarbamate (IUPAC/ Chemical Abstracts name) (1109) + COMPOUND OF FORMULA I, 2-(2-butoxyethoxy)ethyl thiocyanate (IUPAC/Chemical Abstracts name) (935) + COMPOUND OF FORMULA I, 2-(4,5-dimethyl-1,3-dioxolan-2-yl)phenyl methylcarbamate (IUPAC/ Chemical Abstracts name) (1084) + COMPOUND OF FORMULA I, 2-(4-chloro-3,5-xylyloxy)ethanol (IUPAC name) (986) + COMPOUND OF FORMULA I, 2-chlorovinyl diethyl phosphate (IUPAC name) (984) + COMPOUND OF FORMULA I, 2-imidazolidone (IUPAC name) (1225) + COMPOUND OF FORMULA I, 2-isovalerylindan-1,3-dione (IUPAC name) (1246) + COMPOUND OF FORMULA I, 2-methyl(prop-2-ynyl)aminophenyl methylcarbamate (IUPAC name) (1284) + COMPOUND OF FORMULA I, 2-thiocyanatoethyl laurate (IUPAC name) (1433) + COMPOUND OF FORMULA I, 3-bromo-1-chloroprop-1-ene (IUPAC name) (917) + COMPOUND OF FORMULA I, 3-methyl-1-phenylpyrazol-5-yl dimethylcarbamate (IUPAC name) (1283) + COMPOUND OF FORMULA I, 4-methyl(prop-2-ynyl)amino-3,5-xylyl methylcarbamate (IUPAC name) (1285) + COMPOUND OF FORMULA I, 5,5-dimethyl-3-oxocyclohex-1-enyl dimethylcarbamate (IUPAC name) (1085) + COMPOUND OF FORMULA I, abamectin (1) + COMPOUND OF FORMULA I, acephate (2) + COMPOUND OF FORMULA I, acetamiprid (4) + COMPOUND OF FORMULA I, acethion (alternative name) [CCN] + COMPOUND OF FORMULA I, acetoprole [CCN] + COMPOUND OF FORMULA I, acrinathrin (9) + COMPOUND OF FORMULA I, acrylonitrile (IUPAC name) (861) + COMPOUND OF FORMULA I, afidopyropen or coprapidpen + compound of formula I, alanycarb (15) + COMPOUND OF FORMULA I, aldicarb (16) + COMPOUND OF FORMULA I, aldoxycarb (863) + COMPOUND OF FORMULA I, aldrin (864) + COMPOUND OF FORMULA I, allethrin (17) + COMPOUND OF FORMULA I, allosamidin (alternative name) [CCN] + COMPOUND OF FORMULA I, allyxycarb (866) + COMPOUND OF FORMULA I, alpha-cypermethrin (202) + COMPOUND OF FORMULA I, alpha-ecdysone (alternative name) [CCN] + COMPOUND OF FORMULA I, alpha-endosulfan [CCN] + COMPOUND OF FORMULA I, aluminium phosphide (640) + COMPOUND OF FORMULA I, amidithion (870) + COMPOUND OF FORMULA I, amidothioate (872) + COMPOUND OF FORMULA I, aminocarb (873) + COMPOUND OF FORMULA I, amiton (875) + COMPOUND OF FORMULA I, amiton hydrogen oxalate (875) + COMPOUND OF FORMULA I, amitraz (24) + COMPOUND OF FORMULA I, anabasine (877) + COMPOUND OF FORMULA I, athidathion (883) + COMPOUND OF FORMULA I, AVI 382 (compound code) + COMPOUND OF FORMULA I, AZ 60541 (compound code) + COMPOUND OF FORMULA I, azadirachtin (alternative name) (41) + COMPOUND OF FORMULA I, azamethiphos (42) + COMPOUND OF FORMULA I, azinphos-ethyl (44) + COMPOUND OF FORMULA I, azinphos-methyl (45) + COMPOUND OF FORMULA I, azothoate (889) + COMPOUND OF FORMULA I, *Bacillus thuringiensis* delta endotoxins (alternative name) (52) + COMPOUND OF FORMULA I, barium hexafluorosilicate (alternative name) [CCN] + COMPOUND OF FORMULA I, barium polysulfide (IUPAC/Chemical Abstracts name) (892) + COMPOUND OF FORMULA I, barthrin [CCN] + COMPOUND OF FORMULA I, Bayer 22/190 (development code) (893) + COMPOUND OF FORMULA I, Bayer 22408 (development code) (894) + COMPOUND OF FORMULA I, bendiocarb (58) + COMPOUND OF FORMULA I, benfuracarb (60) + COMPOUND OF FORMULA I, bensultap (66) + COMPOUND OF FORMULA I, beta-cyfluthrin (194) + COMPOUND OF FORMULA I, beta-cypermethrin (203) + COMPOUND OF FORMULA I, bifenthrin (76) + COMPOUND OF FORMULA I, bioallethrin (78) + COMPOUND OF FORMULA I, bioallethrin S-cyclopentenyl isomer (alternative name) (79) + COMPOUND OF FORMULA I, bioethanomethrin [CCN] + COMPOUND OF FORMULA I, biopermethrin (908) + COMPOUND OF FORMULA I, bioresmethrin (80) + COMPOUND OF FORMULA I, bis(2-chloroethyl) ether (IUPAC name) (909) + COMPOUND OF FORMULA I, bistrifluron (83) + COMPOUND OF FORMULA I, borax (86) + COMPOUND OF FORMULA I, brofenvalerate (alternative name) + COMPOUND OF FORMULA I, bromfenvinfos (914) + COMPOUND OF FORMULA I, bromocyclen (918) + COMPOUND OF FORMULA I, bromo-DDT (alternative name) [CCN] + COMPOUND OF FORMULA I, bromophos (920) + COMPOUND OF FORMULA I, bromophos-ethyl (921) + COMPOUND OF FORMULA I, bufencarb (924) + COMPOUND OF FORMULA I, buprofezin (99) + COMPOUND OF FORMULA I, butacarb (926) + COMPOUND OF FORMULA I, butathiofos (927) + COMPOUND OF FORMULA I, butocarboxim (103) + COMPOUND OF FORMULA I, butonate (932) + COMPOUND OF FORMULA I, butoxycarboxim (104) + COMPOUND OF FORMULA I, butylpyridaben (alternative name) + COMPOUND OF FORMULA I, cadusafos (109) + COMPOUND OF FORMULA I, calcium arsenate [CCN] + COMPOUND OF FORMULA I, calcium cyanide (444) + COMPOUND OF FORMULA I, calcium polysulfide (IUPAC name) (111) + COMPOUND OF FORMULA I, camphechlor (941) + COMPOUND OF FORMULA I, carbanolate (943) + COMPOUND OF FORMULA I, carbaryl (115) + COMPOUND OF FORMULA I, carbofuran (118) + COMPOUND OF FORMULA I, carbon disulfide (IUPAC/Chemical Abstracts name) (945) + COMPOUND OF FORMULA I, carbon tetrachloride (IUPAC name) (946) + COMPOUND OF FORMULA I, carbophenothion (947) + COMPOUND OF FORMULA I, carbosulfan (119) + COMPOUND OF FORMULA I, cartap (123) + COMPOUND OF FORMULA I, cartap hydrochloride (123) + COMPOUND OF FORMULA I, celangulin (alternative name) + COMPOUND OF FORMULA I, cevadine (alternative name) (725) + COMPOUND OF FORMULA I, chlorantraniliprole [CCN] + COMPOUND OF FORMULA I, chlorbicyclen (960) + COMPOUND OF FORMULA I, chlordane (128) + COMPOUND OF FORMULA I, chlordecone (963) + COMPOUND OF FORMULA I, chlordimeform (964) + COMPOUND OF FORMULA I, chlordimeform hydrochloride (964) + COMPOUND OF FORMULA I, chlorethoxyfos (129) + COMPOUND OF FORMULA I, chlorfenapyr (130) + COMPOUND OF FORMULA I, chlorfenvinphos (131) + COMPOUND OF FORMULA I, chlorfluazuron (132) + COMPOUND OF FORMULA I, chlormephos (136) + COMPOUND OF FORMULA I, chloroform [CCN] + COMPOUND OF FORMULA I, chloropicrin (141) + COMPOUND OF FORMULA I, chlorphoxim (989) + COMPOUND OF FORMULA I, chlorprazophos (990) + COMPOUND OF FORMULA I, chlorpyrifos (145) + COMPOUND OF FORMULA I, chlorpyrifos-methyl (146) + COMPOUND OF FORMULA I, chlorthiophos (994) + COMPOUND OF FORMULA I, chromafenozide (150) + COMPOUND OF FORMULA I, cinerin I (696) + COMPOUND OF FORMULA I, cinerin II (696) + COMPOUND OF FORMULA I, cinerins (696) + COMPOUND OF FORMULA I, cis-resmethrin (alternative name) + COMPOUND OF FORMULA I, cismethrin (80) + COMPOUND OF FORMULA I, clocythrin (alternative name) + COMPOUND OF FORMULA I, cloethocarb (999) + COMPOUND OF FORMULA I, closantel (alternative name) [CCN] + COMPOUND OF FORMULA I, clothianidin (165) + COMPOUND OF FORMULA I, copper acetoarsenite [CCN] + COMPOUND OF FORMULA I, copper arsenate [CCN] + COMPOUND OF FORMULA I, copper oleate [CCN] + COMPOUND OF FORMULA I, coumaphos (174) + COMPOUND OF FORMULA I, coumithoate (1006) + COMPOUND OF FORMULA I, crotamiton (alternative name) [CCN] + COMPOUND OF FORMULA I, crotoxyphos (1010) + COMPOUND OF FORMULA I, crufomate (1011) + COMPOUND OF FORMULA I, cryolite (alternative name) (177) + COMPOUND OF FORMULA I, CS 708 (development code) (1012) + COMPOUND OF FORMULA I, cyanofenphos (1019) + COMPOUND OF FORMULA I, cyanophos (184) + COMPOUND OF FORMULA I, cyanthoate (1020) + COMPOUND OF FORMULA I, cyantraniliprole [CCN] + COMPOUND OF FORMULA I, cyclethrin [CCN] + COMPOUND OF FORMULA I, cycloprothrin (188) + COMPOUND OF FORMULA I, cyfluthrin (193) + COMPOUND OF FORMULA I, cyhalothrin (196) + COMPOUND OF FORMULA I, cypermethrin (201) + COMPOUND OF FORMULA I, cyphenothrin (206) + COMPOUND OF FORMULA I, cyromazine (209) + COMPOUND OF FORMULA I, cythioate (alternative name) [CCN] + COMPOUND OF FORMULA I, d-limonene (alternative name) [CCN] + COMPOUND OF FORMULA I, d-tetramethrin (alternative name) (788) + COMPOUND OF FORMULA I, DAEP (1031) + COMPOUND OF FORMULA I, dazomet (216) + COMPOUND OF FORMULA I, DDT (219) + COMPOUND OF FORMULA I, decarbofuran (1034) + COMPOUND OF FORMULA I, deltamethrin (223) + COMPOUND OF FORMULA I, demephion (1037) + COMPOUND OF FORMULA I, demephion-O (1037) + COMPOUND OF FORMULA I, demephion-S (1037) + COMPOUND OF FORMULA I, demeton (1038) + COMPOUND OF FORMULA I, demeton-methyl (224) + COMPOUND OF FORMULA I, demeton-O (1038) + COMPOUND OF FORMULA I, demeton-O-methyl (224) + COMPOUND OF FORMULA I, demeton-S (1038) + COMPOUND OF FORMULA I, demeton-S-methyl (224) + COMPOUND OF FORMULA I, demeton-S-methylsulphon (1039) + COMPOUND OF FORMULA I, diafenthiuron (226) + COMPOUND OF FORMULA I, dialifos (1042) + COMPOUND OF FORMULA I, diamidafos (1044) + COMPOUND OF FORMULA I, diazinon (227) + COMPOUND OF FORMULA I, dicapthon (1050) + COMPOUND OF FORMULA I, dichlofenthion (1051) + COMPOUND OF FORMULA I, dichlorvos (236) + COMPOUND OF FORMULA I, dicliphos (alternative name) + COMPOUND OF FORMULA I, dicresyl (alternative name) [CCN] + COMPOUND OF FORMULA I, dicrotophos (243) + COMPOUND OF FORMULA I, dicyclanil (244) + COMPOUND OF FORMULA I, dieldrin (1070) + COMPOUND OF FORMULA I, diethyl 5-methylpyrazol-3-yl phosphate (IUPAC name) (1076) + COMPOUND OF FORMULA I, diflubenzuron (250) + COMPOUND OF FORMULA I, dilor (alternative name) [CCN] + COMPOUND OF FORMULA I, dimefluthrin [CCN] + COMPOUND OF FORMULA I, dimefox (1081) + COMPOUND OF FORMULA I, dimetan (1085) + COMPOUND OF FORMULA I, dimethoate (262) + COMPOUND OF FORMULA I, dimethrin (1083) + COMPOUND OF FORMULA I, dimethylvinphos (265) + COMPOUND OF FORMULA I, dimetilan (1086) + COMPOUND OF FORMULA I, dinex (1089) + COMPOUND OF FORMULA I, dinex-diclexine (1089) + COMPOUND OF FORMULA I, dinoprop (1093) + COMPOUND OF FORMULA I, dinosam (1094) + COMPOUND OF FORMULA I, dinoseb (1095) + COMPOUND OF FORMULA I, dinotefuran (271) + COMPOUND OF FORMULA I, diofenolan (1099) + COMPOUND OF FORMULA I, dioxabenzofos (1100) + COMPOUND OF FORMULA I, dioxacarb (1101) + COMPOUND OF FORMULA I, dioxathion (1102) + COMPOUND OF FORMULA I, disulfoton (278) + COMPOUND OF FORMULA I, dithicrofos (1108) + COMPOUND OF FORMULA I, DNOC (282) + COMPOUND OF FORMULA I, doramectin (alternative name) [CCN] + COMPOUND OF FORMULA I, DSP (1115) + COMPOUND OF FORMULA I, ecdysterone (alternative name) [CCN] + COMPOUND OF FORMULA I, EI 1642 (development code) (1118) + COMPOUND OF FORMULA I, emamectin (291) + COMPOUND OF FORMULA I, emamectin benzoate (291) + COMPOUND OF FORMULA I, EMPC (1120) + COMPOUND OF FORMULA I, empenthrin (292) + COMPOUND OF FORMULA I, endosulfan (294) + COMPOUND OF FORMULA I, endothion (1121) + COMPOUND OF FORMULA I, endrin (1122) + COMPOUND OF FORMULA I, EPBP (1123) + COMPOUND OF FORMULA I, EPN (297) + COMPOUND OF FORMULA I, epofenonane (1124) + COMPOUND OF FORMULA I, eprinomectin (alternative name) [CCN] + COMPOUND OF FORMULA I, eremophilone oil + COMPOUND OF FORMULA I, esfenvalerate (302) + COMPOUND OF FORMULA I, etaphos (alternative name) [CCN] + COMPOUND OF FORMULA I, ethiofencarb (308) + COMPOUND OF FORMULA I, ethion (309) + COMPOUND OF FORMULA I, ethiprole (310) + COMPOUND OF FORMULA I, ethoate-methyl (1134) + COMPOUND OF FORMULA I, ethoprophos (312) + COMPOUND OF FORMULA I, ethyl formate (IUPAC name) [CCN] + COMPOUND OF FORMULA I, ethyl-DDD (alternative name) (1056) + COMPOUND OF FORMULA I, ethylene dibromide (316) + COMPOUND OF FORMULA I, ethylene dichloride (chemical name) (1136) + COMPOUND OF FORMULA I, ethylene oxide [CCN] + COMPOUND OF FORMULA I, etofenprox (319) + COMPOUND OF FORMULA I, etrimfos (1142) + COMPOUND OF FORMULA I, EXD (1143) + COMPOUND OF FORMULA I, famphur (323) + COMPOUND OF FORMULA I, fenamiphos (326) + COMPOUND OF FORMULA I, fenazaflor (1147) + COMPOUND OF FORMULA I, fenchlorphos (1148) + COMPOUND OF FORMULA I, fenethacarb (1149) + COMPOUND OF FORMULA I, fenfluthrin (1150) + COMPOUND OF FORMULA I, fenitrothion (335) + COMPOUND OF FORMULA I, fenobucarb (336) + COMPOUND OF FORMULA I, fenoxacrim (1153) + COMPOUND OF FORMULA I, fenoxycarb (340) + COMPOUND OF FORMULA I, fenpirithrin (1155) + COMPOUND OF FORMULA I, fenpropathrin (342) + COMPOUND OF FORMULA I, fenpyrad (alternative name) + COMPOUND OF FORMULA I, fensulfothion (1158) + COMPOUND OF FORMULA I, fenthion (346) + COMPOUND OF FORMULA I, fenthionethyl [CCN] + COMPOUND OF FORMULA I, fenvalerate (349) + COMPOUND OF FORMULA I, fipronil (354) + COMPOUND OF FORMULA I, flometoquin [CCN] + COMPOUND OF FORMULA I, flonicamid (358) + COMPOUND OF FORMULA I, flubendiamide (CAS. Reg. No.: 272451-65-7) + COMPOUND OF FORMULA I, flucofuron (1168) + COMPOUND OF FORMULA I, flucycloxuron (366) + COMPOUND OF FORMULA I, flucythrinate (367) + COMPOUND OF FORMULA I, fluenetil (1169) + COMPOUND OF FORMULA I, fluensulfone [CCN] + COMPOUND OF FORMULA I, flufenerim [CCN] + COMPOUND OF FORMULA I, flufenoxuron (370) + COMPOUND OF FORMULA I, flufenprox (1171) + COMPOUND OF FORMULA I, flufiprole [CCN] + COMPOUND OF FORMULA I, flumethrin (372) + COMPOUND OF FORMULA I, flupyradifurone [CCN] + COMPOUND OF FORMULA I, fluvalinate (1184) + COMPOUND OF FORMULA I, FMC 1137 (development code) (1185) + COMPOUND OF FORMULA I, fonofos (1191) + COMPOUND OF FORMULA I, formetanate (405) + COMPOUND OF FORMULA I, formetanate hydrochloride (405) + COMPOUND OF FORMULA I, formothion (1192) + COMPOUND OF FORMULA I, formparanate (1193) + COMPOUND OF FORMULA I, fosmethilan (1194) + COMPOUND OF FORMULA I, fospirate (1195) + COMPOUND OF FORMULA I, fosthiazate (408) + COMPOUND OF FORMULA I, fosthietan (1196) + COMPOUND OF FORMULA I, furathiocarb (412) + COMPOUND OF FORMULA I, furethrin (1200) + COMPOUND OF FORMULA I, gamma-cyhalothrin (197) + COMPOUND OF FORMULA I, gamma-HCH (430) + COMPOUND OF FORMULA I, guazatine (422) + COMPOUND OF FORMULA I, guazatine acetates (422) + COMPOUND OF FORMULA I, GY-81 (development code) (423) + COMPOUND OF FORMULA I, halfenprox (424) + COMPOUND OF FORMULA I, halofenozide (425) + COMPOUND OF FORMULA I, HCH (430) + COMPOUND OF FORMULA I, HEOD (1070) + COMPOUND OF FORMULA I, heptachlor (1211) + COMPOUND OF FORMULA I, heptenophos (432) + COMPOUND OF FORMULA I, heterophos [CCN] + COMPOUND OF FORMULA I, hexaflumuron (439) + COMPOUND OF FORMULA I, HHDN (864) + COMPOUND OF FORMULA I, hydramethylnon (443) + COMPOUND OF FORMULA I, hydrogen cyanide (444) + COMPOUND OF FORMULA I, hydroprene (445) + COMPOUND OF FORMULA I, hyquincarb (1223) + COMPOUND OF FORMULA I, imidacloprid (458) + COMPOUND OF FORMULA I, imiprothrin (460) + COMPOUND OF FORMULA I, indoxacarb (465) + COMPOUND OF FORMULA I, iodomethane (IUPAC name) (542) + COMPOUND OF FORMULA I, IPPA-152004 (compound code) + COMPOUND OF FORMULA I, IPSP (1229) + COMPOUND OF FORMULA I, isazofos (1231) + COMPOUND OF FORMULA I, isobenzan (1232) + COMPOUND OF FORMULA I, isocarbophos (alternative name) (473) + COMPOUND OF FORMULA I, isodrin (1235) + COMPOUND OF FORMULA I, isofenphos (1236) + COMPOUND OF FORMULA I, isolane (1237) + COMPOUND OF FORMULA I, isoprocarb (472) + COMPOUND OF FORMULA I, isopropyl O-(methoxy-aminothiophosphoryl)salicylate (IUPAC name) (473) + COMPOUND OF FORMULA I, isoprothiolane (474) + COMPOUND OF FORMULA I, isothioate (1244) + COMPOUND OF FORMULA I, isoxathion (480) + COMPOUND OF FORMULA I, ivermectin (alternative name) [CCN] + COMPOUND OF FORMULA I, jasmolin I (696) + COMPOUND OF FORMULA I, jasmolin II (696) + COMPOUND OF FORMULA I, jodfenphos (1248) + COMPOUND OF FORMULA I, juvenile hormone I (alternative name) [CCN] + COMPOUND OF FORMULA I, juvenile hormone II (alternative name) [CCN] + COMPOUND OF FORMULA I, juvenile hormone III (alternative name) [CCN] + COMPOUND OF FORMULA I, kelevan (1249) + COMPOUND OF FORMULA I, kinoprene (484) + COMPOUND OF FORMULA I, lambda-cyhalothrin (198) + COMPOUND OF FORMULA I, lead arsenate [CCN] + COMPOUND OF FORMULA I, lepimectin (CCN) + COMPOUND OF FORMULA I, leptophos (1250) + COMPOUND OF FORMULA I, lindane (430) + COMPOUND OF FORMULA I, lirimfos (1251) + COMPOUND OF FORMULA I, lufenuron (490) + COMPOUND OF FORMULA I, lythidathion (1253) + COMPOUND OF FORMULA I, m-cumenyl methylcarbamate (IUPAC name) (1014) + COMPOUND OF FORMULA I, magnesium phosphide (IUPAC name) (640) + COMPOUND OF FORMULA I, malathion (492) + COMPOUND OF FORMULA I, malonoben (1254) + COMPOUND OF FORMULA I, mazidox (1255) + COMPOUND OF FORMULA I, mecarbam (502) + COMPOUND OF FORMULA I, mecarphon (1258) + COMPOUND OF FORMULA I, menazon (1260) + COMPOUND OF FORMULA I, mephosfolan (1261) + COMPOUND OF FORMULA I, mercurous chloride (513) + COMPOUND OF FORMULA I, mesulfenfos (1263) + COMPOUND OF FORMULA I, metaflumizone (CCN) + COMPOUND OF FORMULA I, metam (519) + COMPOUND OF FORMULA I, metam-potassium (alternative name) (519) + COMPOUND OF FORMULA I, metam-sodium (519) + COMPOUND OF FORMULA I, methacrifos (1266) + COMPOUND OF FORMULA I, methamidophos (527) + COMPOUND OF FORMULA I, methanesulfonyl fluoride (IUPAC/Chemical Abstracts name) (1268) + COMPOUND OF FORMULA I, methidathion (529) + COMPOUND OF FORMULA I, methiocarb (530) + COMPOUND OF FORMULA I, methocrotophos (1273) + COMPOUND OF FORMULA I, methomyl (531) + COMPOUND OF FORMULA I, methoprene (532) + COMPOUND OF FORMULA I, methoquin-butyl (1276) + COMPOUND OF FORMULA I, methothrin (alternative name) (533) + COMPOUND OF FORMULA I, methoxychlor (534) + COMPOUND OF FORMULA I, methoxyfenozide (535) + COMPOUND OF FORMULA I, methyl bromide (537) + COMPOUND OF FORMULA I, methyl isothiocyanate (543) + COMPOUND OF FORMULA I, methylchloroform (alternative name) [CCN] + COMPOUND OF FORMULA I, methylene chloride [CCN] + COMPOUND OF FORMULA I, metofluthrin [CCN] + COMPOUND OF FORMULA I, metolcarb (550) + COMPOUND OF FORMULA I, metoxadiazone (1288) + COMPOUND OF FORMULA I, mevinphos (556) + COMPOUND OF FORMULA I, mexacarbate (1290) + COMPOUND OF FORMULA I, milbemectin (557) + COMPOUND OF FORMULA I, milbemycin oxime (alternative name) [CCN] + COMPOUND OF FORMULA I, mipafox (1293) + COMPOUND OF FORMULA I, mirex (1294) + COMPOUND OF FORMULA I, monocrotophos (561) + COMPOUND OF FORMULA I, morphothion (1300) + COMPOUND OF FORMULA I, moxidectin (alternative name) [CCN] + COMPOUND OF FORMULA I, naftalofos (alternative name) [CCN] + COMPOUND OF FORMULA I, naled (567) + COMPOUND OF FORMULA I, naphthalene (IUPAC/Chemical Abstracts name) (1303) + COMPOUND OF FORMULA I, NC-170 (development code) (1306) + COMPOUND OF FORMULA I, NC-184 (compound code) + COMPOUND OF FORMULA I, nicotine (578) + COMPOUND OF FORMULA I, nicotine sulfate (578) + COMPOUND OF FORMULA I, nifluridide (1309) + COMPOUND OF FORMULA I, nitenpyram (579) + COMPOUND OF FORMULA I, nithiazine (1311) + COMPOUND OF FORMULA I, nitrilacarb (1313) + COMPOUND OF FORMULA I, nitrilacarb 1:1 zinc chloride complex (1313) + COMPOUND OF FORMULA I, NNI-0101 (compound code) + COMPOUND OF FORMULA I, NNI-0250 (compound code) + COMPOUND OF FORMULA I, nornicotine (traditional name) (1319) + COMPOUND OF FORMULA I, novaluron (585) + COMPOUND OF FORMULA I, noviflumuron (586) + COMPOUND OF FORMULA I, O-5-dichloro-4-iodophenyl O-ethyl ethylphosphonothioate (IUPAC name) (1057) + COMPOUND OF FORMULA I, O,O-diethyl O-4-methyl-2-oxo-2H-chromen-7-yl phosphorothioate (IUPAC name) (1074) + COMPOUND OF FORMULA I, O,O-diethyl O-6-methyl-2-propylpyrimidin-4-yl phosphorothioate (IUPAC name) (1075) + COMPOUND OF FORMULA I, O,O,O',O'-tetrapropyl dithiopyrophosphate (IUPAC name) (1424) + COMPOUND OF FORMULA I, oleic acid (IUPAC name) (593) + COMPOUND OF FORMULA I, omethoate (594) + COMPOUND OF FORMULA I, oxamyl (602) + COMPOUND OF FORMULA I, oxydemeton-methyl (609) + COMPOUND OF FORMULA I, oxydeprofos (1324) + COMPOUND OF FORMULA I, oxydisulfoton (1325) + COMPOUND OF FORMULA I, pp'-DDT (219) + COMPOUND OF FORMULA I, para-dichlorobenzene [CCN] + COMPOUND OF FORMULA I, parathion (615) + COMPOUND OF FORMULA I, parathion-methyl (616) + COMPOUND OF FORMULA I, penfluron (alternative name) [CCN] + COMPOUND OF FORMULA I, pentachlorophenol (623) + COMPOUND OF FORMULA I, pentachlorophenyl laurate (IUPAC name) (623) + COMPOUND OF FORMULA I, permethrin (626) + COMPOUND OF FORMULA I, petroleum oils (alternative name) (628) + COMPOUND OF FORMULA I, PH 60-38 (development code) (1328) + COMPOUND OF FORMULA I, phenkapton (1330) + COMPOUND OF FORMULA I, phenothrin (630) + COMPOUND OF FORMULA I, phenthoate (631) + COMPOUND OF FORMULA I, phorate (636) + COMPOUND OF FORMULA I, phosalone (637) + COMPOUND OF FORMULA I, phosfolan (1338) + COMPOUND OF FORMULA I, phosmet (638) + COMPOUND OF FORMULA I, phosnichlor (1339) + COMPOUND OF FORMULA I, phosphamidon (639) + COMPOUND OF FORMULA I, phosphine (IUPAC name) (640) + COMPOUND OF FORMULA I, phoxim (642) + COMPOUND OF FORMULA I, phoxim-methyl (1340) + COMPOUND OF FORMULA I, pirimetaphos (1344) + COMPOUND OF FORMULA I, pirimicarb (651) + COMPOUND OF FORMULA I, pirimiphos-ethyl (1345) + COMPOUND OF FORMULA I, pirimiphos-methyl (652) + COMPOUND OF FORMULA I, polychlorodicyclopentadiene isomers (IUPAC name) (1346) + COMPOUND OF FORMULA I, polychloroterpenes (traditional name) (1347) + COMPOUND OF FORMULA I, potassium arsenite [CCN] + COMPOUND OF FORMULA I, potassium thiocyanate [CCN] + COMPOUND OF FORMULA I, prallethrin (655) + COMPOUND OF FORMULA I, precocene I (alternative name) [CCN] + COMPOUND OF FORMULA I, precocene II (alternative name) [CCN] + COMPOUND OF FORMULA I, precocene III (alternative name) [CCN] + COMPOUND OF FORMULA I, primidophos (1349) + COMPOUND OF FORMULA I, profenofos (662) + COMPOUND OF FORMULA I, profluthrin [CCN] + COMPOUND OF FORMULA I, promacyl (1354) + COMPOUND OF FORMULA I, promecarb (1355) + COMPOUND OF FORMULA I, propaphos (1356) + COMPOUND OF FORMULA I, propetamphos (673) + COMPOUND OF FORMULA I, propoxur (678) + COMPOUND OF FORMULA I, prothidathion (1360) + COMPOUND OF FORMULA I, prothiofos (686) + COMPOUND OF FORMULA I, prothoate (1362) + COMPOUND OF FORMULA I, protrifenbute [CCN] + COMPOUND OF FORMULA I, pymetrozine (688) + COMPOUND OF FORMULA I, pyraclofos (689) + COMPOUND OF FORMULA I, pyrafluprole [CCN] + COMPOUND OF FORMULA I, pyrazophos (693) + COMPOUND OF FORMULA I, pyresmethrin (1367) + COMPOUND OF FORMULA I, pyrethrin I (696) + COMPOUND OF FORMULA I, pyrethrin II (696) + COMPOUND OF FORMULA I, pyrethrins (696) + COMPOUND OF FORMULA I, pyridaben (699) + COMPOUND OF FORMULA I, pyridalyl (700) + COMPOUND OF FORMULA I, pyridaphenthion (701) + COMPOUND OF FORMULA I, pyrifluquinazon [CCN] + COMPOUND OF FORMULA I, pyrimidifen (706) + COMPOUND OF FORMULA I, pyrimitate (1370) + COMPOUND OF FORMULA I, pyriprole [CCN] + COMPOUND OF FORMULA I, pyriproxyfen (708) + COMPOUND OF FORMULA I, quassia (alternative name) [CCN] + COMPOUND OF FORMULA I, quinalphos (711) + COMPOUND OF FORMULA I, quinalphos-methyl (1376) + COMPOUND OF FORMULA I, quinothion (1380) + COMPOUND OF FORMULA I, quintiofos (1381) + COMPOUND OF FORMULA I, R-1492 (development code) (1382) + COMPOUND OF FORMULA I, rafoxanide (alternative name) [CCN] + COMPOUND OF FORMULA I, resmethrin (719) + COMPOUND OF FORMULA I, rotenone (722) + COMPOUND OF FORMULA I, RU 15525 (development code) (723) + COMPOUND OF FORMULA I, RU 25475 (development code) (1386) + COMPOUND OF FORMULA I, ryania (alternative name) (1387) + COMPOUND OF FORMULA I, ryanodine (traditional name) (1387) + COMPOUND OF FORMULA I, sabadilla (alternative name) (725) + COMPOUND OF FORMULA I, schradan (1389) + COMPOUND OF FORMULA I, sebufos (alternative name) + COMPOUND OF FORMULA I, selamectin (alternative name) [CCN] + COMPOUND OF FORMULA I, SI-0009 (compound code) + COMPOUND OF FORMULA I, SI-0205 (compound code) + COMPOUND OF FORMULA I, SI-0404 (compound code) + COMPOUND OF FORMULA I, SI-0405 (compound code) + COMPOUND OF FORMULA I, silafluofen (728) + COMPOUND OF FORMULA I, SN 72129 (development code) (1397) + COMPOUND OF FORMULA I, sodium arsenite [CCN] + COMPOUND OF FORMULA I, sodium cyanide (444) + COMPOUND OF FORMULA I, sodium fluoride (IUPAC/Chemical Abstracts name) (1399) + COMPOUND OF FORMULA I, sodium hexafluorosilicate (1400) + COMPOUND OF FORMULA I, sodium pentachlorophenoxide (623) + COMPOUND OF FORMULA I, sodium selenate (IUPAC name) (1401) + COMPOUND OF FORMULA I, sodium thiocyanate [CCN] + COMPOUND OF FORMULA I, sophamide (1402) + COMPOUND OF FORMULA I, spinetoram [CCN] + COMPOUND OF FORMULA I, spinosad (737) + COMPOUND OF FORMULA I, spiromesifen (739) + COMPOUND OF FORMULA I, spirotetramat [CCN] + COMPOUND OF FORMULA I, sulcofuron (746) + COMPOUND OF FORMULA I, sulcofuron-sodium (746) + COMPOUND OF FORMULA I, sulfluramid (750) + COMPOUND OF FORMULA I, sulfotep (753) + COMPOUND OF FORMULA I, sulfoxaflor [CCN] + COMPOUND OF FORMULA I, sulfuryl fluoride (756) + COMPOUND OF FORMULA I, sulprofos (1408) + COMPOUND OF FORMULA I, tar oils (alternative name) (758) + COMPOUND OF FORMULA I, tau-fluvalinate (398) + COMPOUND OF FORMULA I, tazimcarb (1412) + COMPOUND OF FORMULA I, TDE (1414) + COMPOUND OF FORMULA I, tebufenozide (762) + COMPOUND OF FORMULA I, tebufenpyrad (763) + COMPOUND OF FORMULA I, tebupirimfos (764) + COMPOUND OF FORMULA I, teflubenzuron (768) + COMPOUND OF FORMULA I, tefluthrin (769) + COMPOUND OF FORMULA I, temephos (770) + COMPOUND OF FORMULA I, TEPP (1417) + COMPOUND OF FORMULA I, terallethrin (1418) + COMPOUND OF FORMULA I, terbam (alternative name) + COMPOUND OF FORMULA I, terbufos (773) + COMPOUND OF FORMULA I, tetrachloroethane [CCN] + COMPOUND OF FORMULA I, tetrachlorvinphos (777) + COMPOUND OF FORMULA I, tetramethrin (787) + COMPOUND OF FORMULA I, tetramethylfluthrin (CAS. Reg. No.: 84937-88-2) + COMPOUND OF FORMULA I, theta-cypermethrin (204) + COMPOUND OF FORMULA I, thiacloprid (791) + COMPOUND OF FORMULA I, thiafenox (alternative name) + COMPOUND OF FORMULA I, thiamethoxam (792) + COMPOUND OF FORMULA I, thicrofos (1428) + COMPOUND OF FORMULA I, thiocarboxime (1431) + COMPOUND OF FORMULA I, thiocyclam (798) + COMPOUND OF FORMULA I, thiocyclam hydrogen oxalate (798) + COMPOUND OF FORMULA I, thiodicarb (799) + COMPOUND OF FORMULA I, thiofanox (800) + COMPOUND OF FORMULA I, thiometon (801) + COMPOUND OF FORMULA I, thionazin (1434) + COMPOUND OF FORMULA I, thiosultap (803) + COMPOUND OF FORMULA I, thiosultap-sodium (803) + COMPOUND OF FORMULA I, thuringiensin (alternative name) [CCN] + COMPOUND OF FORMULA I, tolfenpyrad (809) + COMPOUND OF FORMULA I, tralomethrin (812) + COMPOUND OF FORMULA I, transfluthrin (813) + COMPOUND OF FORMULA I, transpermethrin (1440) + COMPOUND OF FORMULA I, triamiphos (1441) + COMPOUND OF FORMULA I, triazamate (818) + COMPOUND OF FORMULA I, triazophos (820) + COMPOUND OF FORMULA I, triazuron (alternative name) + COMPOUND OF FORMULA I, trichlorfon (824) + COMPOUND OF FORMULA I, trichlormetaphos-3 (alternative name) [CCN] + COMPOUND OF FORMULA I, trichloronat (1452) + COMPOUND OF FORMULA I, trifenofos (1455) + COMPOUND OF FORMULA I, triflumuron (835) + COMPOUND OF FORMULA I, trimethacarb (840) + COMPOUND OF FORMULA I, triprene (1459) + COMPOUND OF FORMULA I, vamidothion (847) + COMPOUND OF FORMULA I, vaniliprole [CCN] + COMPOUND OF FORMULA I, veratridine (alternative name) (725) + COMPOUND OF FORMULA I, veratrine (alternative name) (725) + COMPOUND OF FORMULA I, XMC (853) + COMPOUND OF FORMULA I, xylylcarb (854) + COMPOUND OF FORMULA I, YI-5302 (compound code) + COMPOUND OF FORMULA I, zeta-cypermethrin (205) + COMPOUND OF FORMULA I, zetamethrin (alternative name) + COMPOUND OF FORMULA I, zinc phosphide (640) + COMPOUND OF FORMULA I, zolaprofos (1469), ZJ0967 (development code) + COMPOUND OF FORMULA I, ZJ3757 (development code) + COMPOUND OF FORMULA I, and ZXI 8901 (development code) (858) + COMPOUND OF FORMULA I,
a molluscicide selected from the group of substances consisting of bis(tributyltin) oxide (IUPAC name) (913) + COMPOUND OF FORMULA I, bromoacetamide [CCN] + COMPOUND OF FORMULA I, calcium arsenate [CCN] + COMPOUND OF FORMULA I, cloethocarb (999) + COMPOUND OF FORMULA I, copper acetoarsenite [CCN] + COMPOUND OF FORMULA I, copper sulfate (172) + COMPOUND OF FORMULA I, fentin (347) + COMPOUND OF FORMULA I, ferric phosphate (IUPAC name) (352) + COMPOUND OF FORMULA I, metaldehyde (518) + COMPOUND OF FORMULA I, methiocarb (530) + COMPOUND OF FORMULA I, niclosamide (576) + COMPOUND OF FORMULA I, niclosamide-olamine (576) + COMPOUND OF FORMULA I, pentachlorophenol (623) + COMPOUND OF FORMULA I, sodium pentachlorophenoxide (623) + COMPOUND OF FORMULA I, tazimcarb (1412) + COMPOUND OF FORMULA I, thiodicarb (799) + COMPOUND OF FORMULA I, tralopyril [CCN] + COMPOUND OF FORMULA I, tributyltin oxide (913) + COMPOUND OF FORMULA I, trifenmorph (1454) + COMPOUND OF FORMULA I, trimethacarb (840) + COMPOUND OF FORMULA I, triphenyltin acetate (IUPAC name) (347) and triphenyltin hydroxide (IUPAC name) (347) + COMPOUND OF FORMULA I,
a nematicide selected from the group of substances consisting of AKD-3088 (compound code) + COMPOUND OF FORMULA I, 1,2-dibromo-3-chloropropane (IUPAC/Chemical Abstracts name) (1045) + COMPOUND OF FORMULA I, 1,2-dichloropropane (IUPAC/ Chemical Abstracts name) (1062) + COMPOUND OF FORMULA I, 1,2-dichloropropane with 1,3-dichloropropene (IUPAC name) (1063) + COMPOUND OF FORMULA I, 1,3-dichloropropene (233) + COMPOUND OF FORMULA I, 3,4-dichlorotetrahydrothiophene 1,1-dioxide (IUPAC/Chemical Abstracts name) (1065) + COMPOUND OF FORMULA I, 3-(4-chlorophenyl)-5-methylrhodanine (IUPAC name) (980) + COMPOUND OF FORMULA I, 5-methyl-6-thioxo-1,3,5-thiadiazinan-3-ylacetic acid (IUPAC name) (1286) + COMPOUND OF FORMULA I, 6-isopentenylaminopurine (alternative name) (210) + COMPOUND OF FORMULA I, abamectin (1) + COMPOUND OF FORMULA I, acetoprole [CCN] + COMPOUND OF FORMULA I, alanycarb (15) + COMPOUND OF FORMULA I, aldicarb (16) + COMPOUND OF FORMULA I, aldoxycarb (863) + COMPOUND OF FORMULA I, AZ 60541 (compound code) + COMPOUND OF FORMULA I, benclothiaz [CCN] + COMPOUND OF FORMULA I, benomyl (62) + COMPOUND OF FORMULA I, butylpyridaben (alternative name) + COMPOUND OF FORMULA I, cadusafos (109) + COMPOUND OF FORMULA I, carbofuran (118) + COMPOUND OF FORMULA I, carbon disulfide (945) + COMPOUND OF FORMULA I, carbosulfan (119) + COMPOUND OF FORMULA I, chloropicrin (141) + COMPOUND OF FORMULA I, chlorpyrifos (145) + COMPOUND OF FORMULA I, cloethocarb (999) + COMPOUND OF FORMULA I, cytokinins (alternative name) (210) + COMPOUND OF FORMULA I, dazomet (216) + COMPOUND OF FORMULA I, DBCP (1045) + COMPOUND OF FORMULA I, DCIP (218) + COMPOUND OF FORMULA I, diamidafos (1044) + COMPOUND OF FORMULA I, dichlofenthion (1051) + COMPOUND OF FORMULA I, dicliphos (alternative name) + COMPOUND OF FORMULA I, dimethoate (262) + COMPOUND OF FORMULA I, doramectin (alternative name) [CCN] + COMPOUND OF FORMULA I, emamectin (291) + COMPOUND OF FORMULA I, emamectin benzoate (291) + COMPOUND OF FORMULA I, eprinomectin (alternative name) [CCN] + COMPOUND OF FORMULA I, ethoprophos (312) + COMPOUND OF FORMULA I, ethylene dibromide (316) + COMPOUND OF FORMULA I, fenamiphos (326) + COMPOUND OF FORMULA I, fenpyrad (alternative name) + COMPOUND OF FORMULA I, fensulfothion (1158) + COMPOUND OF FORMULA I, fluensulfone (CAS. Reg. No.: 318290-98-1) + COMPOUND OF FORMULA I, fosthiazate (408) + COMPOUND OF FORMULA I, fosthietan (1196) + COMPOUND OF FORMULA I, furfural (alternative name) [CCN] + COMPOUND OF FORMULA I, GY-81 (development code) (423) + COMPOUND OF FORMULA I, heterophos [CCN] + COMPOUND OF FORMULA I, imicyafos [CCN] + COMPOUND OF FORMULA I, imicyafos (alternative name) [CCN] + COMPOUND OF FORMULA I, iodomethane (IUPAC name) (542) + COMPOUND OF FORMULA I, isamidofos (1230) + COMPOUND OF FORMULA I, isazofos (1231) + COMPOUND OF FORMULA I, ivermectin (alternative name) [CCN] + COMPOUND OF FORMULA I, kinetin (alternative name) (210) + COMPOUND OF FORMULA I, mecarphon (1258) + COMPOUND OF FORMULA I, metam (519) + COMPOUND OF FORMULA I, metam-potassium (alternative name) (519) + COMPOUND OF FORMULA I, metam-sodium (519) + COMPOUND OF FORMULA I, methyl bromide (537) + COMPOUND OF FORMULA I, methyl isothiocyanate (543) + COMPOUND OF FORMULA I, milbemycin oxime (alternative name) [CCN] + COMPOUND OF FORMULA I, moxidectin (alternative name) [CCN] + COMPOUND OF FORMULA I, *Myrothecium verrucaria* composition (alternative name) (565) + COMPOUND OF FORMULA I, NC-184 (compound code) + COMPOUND OF FORMULA I, oxamyl (602) + COMPOUND OF FORMULA I, phorate (636) + COMPOUND OF FORMULA I, phosphamidon (639) + COMPOUND OF FORMULA I, phosphocarb [CCN] + COMPOUND OF FORMULA I, sebufos (alternative name) + COMPOUND OF FORMULA I, selamectin (alternative name) [CCN] + COMPOUND OF FORMULA I, spinosad (737) + COMPOUND OF FORMULA I, terbam (alternative name) + COMPOUND OF FORMULA I, terbufos (773) + COMPOUND OF FORMULA I, tetrachlorothiophene (IUPAC/ Chemical Abstracts name) (1422) + COMPOUND OF FORMULA I, thiafenox (alternative name) + COMPOUND OF FORMULA I, thionazin (1434) + COMPOUND OF FORMULA I, triazophos (820) + COMPOUND OF FORMULA I, triazuron (alternative name) + COMPOUND OF FORMULA I, xylenols [CCN] + COMPOUND OF FORMULA I, YI-5302 (compound code) and zeatin (alternative name) (210) + COMPOUND OF FORMULA I,
a nitrification inhibitor selected from the group of substances consisting of potassium ethylxanthate [CCN] and nitrapyrin (580) + COMPOUND OF FORMULA I,
a plant activator selected from the group of substances consisting of acibenzolar (6) + COMPOUND OF FORMULA I, acibenzolar-S-methyl (6) + COMPOUND OF FORMULA I, probenazole (658) and *Reynoutria sachalinensis* extract (alternative name) (720) + COMPOUND OF FORMULA I,
a rodenticide selected from the group of substances consisting of 2-isovalerylindan-1,3-dione (IUPAC name) (1246) + COMPOUND OF FORMULA I, 4-(quinoxalin-2-ylamino)benzenesulfonamide (IUPAC name) (748) + COMPOUND OF FORMULA I, alpha-chlorohydrin [CCN] + COMPOUND OF FORMULA I, aluminium phosphide (640) + COMPOUND OF FORMULA I, antu (880) + COMPOUND OF FORMULA I, arsenous oxide (882) + COMPOUND OF FORMULA I, barium carbonate (891) + COMPOUND OF FORMULA I, bisthiosemi (912) + COMPOUND OF FORMULA I, brodifacoum (89) + COMPOUND OF FORMULA I, bromadiolone (91) + COMPOUND OF FORMULA I, bromethalin (92) + COMPOUND OF FORMULA I, calcium cyanide (444) + COMPOUND OF FORMULA I, chloralose (127) + COMPOUND OF FORMULA I, chlorophacinone (140) + COMPOUND OF FORMULA I, cholecalciferol (alternative name) (850) + COMPOUND OF FORMULA I, colecalciferol + COMPOUND OF FORMULA I, coumachlor (1004) + COMPOUND OF FORMULA I, coumafuryl (1005) + COMPOUND OF FORMULA I, coumatetralyl (175) + COMPOUND OF FORMULA I, crimidine (1009) + COMPOUND OF FORMULA I, difenacoum (246) + COMPOUND OF FORMULA I, difethialone (249) + COMPOUND OF FORMULA I, diphacinone (273) + COMPOUND OF FORMULA I, ergocalciferol (301) + COMPOUND OF FORMULA I, flocoumafen (357) + COMPOUND OF FORMULA I, fluoroacetamide (379) + COMPOUND OF FORMULA I, flupropadine (1183) + COMPOUND OF FORMULA I, flupropadine hydrochloride (1183) + COMPOUND OF FORMULA I, gamma-HCH (430) + COMPOUND OF FORMULA I, HCH (430) + COMPOUND OF FORMULA I, hydrogen cyanide (444) + COMPOUND OF FORMULA I, iodomethane (IUPAC name) (542) + COMPOUND OF FORMULA I, lindane (430) + COMPOUND OF FORMULA I, magnesium phosphide (IUPAC name) (640) + COMPOUND OF FORMULA I, methyl bromide (537) + COMPOUND OF FORMULA I, norbormide (1318) + COMPOUND OF FORMULA I, phosacetim (1336) + COMPOUND OF FORMULA I, phosphine (IUPAC name) (640) + COMPOUND OF FORMULA I, phosphorus [CCN] + COMPOUND OF FORMULA I, pindone (1341) + COMPOUND OF FORMULA I, potassium arsenite [CCN] + COMPOUND OF FORMULA I, pyrinuron (1371) + COMPOUND OF FORMULA I, scilliroside (1390) + COMPOUND OF FORMULA I, sodium arsenite [CCN] + COMPOUND OF FORMULA I, sodium cyanide (444) + COMPOUND OF FORMULA I, sodium fluoroacetate (735) + COMPOUND OF FORMULA I, strychnine (745) + COMPOUND OF FORMULA I, thallium sulfate [CCN] + COMPOUND OF FORMULA I, warfarin (851) and zinc phosphide (640) + COMPOUND OF FORMULA I,
a synergist selected from the group of substances consisting of 2-(2-butoxyethoxy)-ethyl piperonylate (IUPAC name) (934) + COMPOUND OF FORMULA I, 5-(1,3-benzodioxol-5-yl)-3-hexylcyclohex-2-enone (IUPAC name) (903) + COMPOUND OF FORMULA I, farnesol with nerolidol (alternative name) (324) + COMPOUND OF FORMULA I, MB-599 (development code) (498) + COMPOUND OF FORMULA I, MGK 264 (development code) (296) + COMPOUND OF FORMULA I, piperonyl butoxide (649) + COMPOUND OF FORMULA I, piprotal (1343) + COMPOUND OF FORMULA I, propyl isomer (1358) + COMPOUND OF FORMULA I, S421 (development code) (724) + COMPOUND OF FORMULA I, sesamex (1393) + COMPOUND OF FORMULA I, sesasmolin (1394) and sulfoxide (1406) + COMPOUND OF FORMULA I,
an animal repellent selected from the group of substances consisting of anthraquinone (32) + COMPOUND OF FORMULA I, chloralose (127) + COMPOUND OF FORMULA I, copper naphthenate [CCN] + COMPOUND OF FORMULA I, copper oxychloride (171) + COMPOUND OF FORMULA I, diazinon (227) + COMPOUND OF FORMULA I, dicyclopentadiene (chemical name) (1069) + COMPOUND OF FORMULA I, guazatine (422) + COMPOUND OF FORMULA I, guazatine acetates (422) + COMPOUND OF FORMULA I, methiocarb (530) + COMPOUND OF FORMULA I, pyridin-4-amine (IUPAC name) (23) + COMPOUND OF FORMULA I, thiram (804) + COMPOUND OF FORMULA I, trimethacarb (840) + COMPOUND OF FORMULA I, zinc naphthenate [CCN] and ziram (856) + COMPOUND OF FORMULA I,
a virucide selected from the group of substances consisting of imanin (alternative name) [CCN] and ribavirin (alternative name) [CCN] + COMPOUND OF FORMULA I,
a wound protectant selected from the group of substances consisting of mercuric oxide (512) + COMPOUND OF FORMULA I, octhilinone (590) and thiophanate-methyl (802) + COMPOUND OF FORMULA I,
an insecticide selected from the group consisting of the compound of the formula A-1 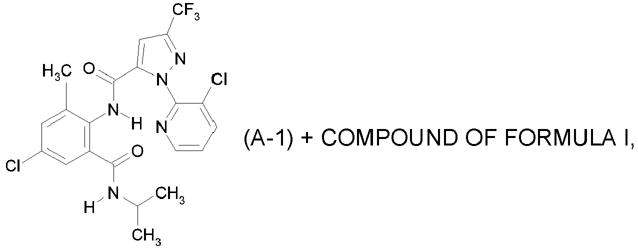 the formula A-2 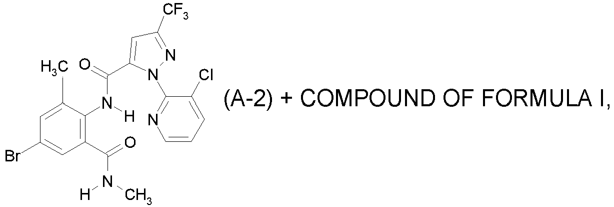 the formula A-3 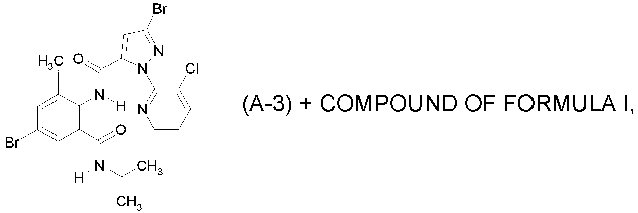 the formula A-4 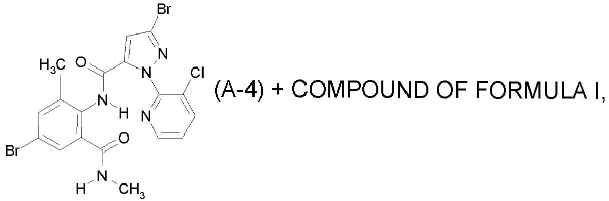 the formula A-5 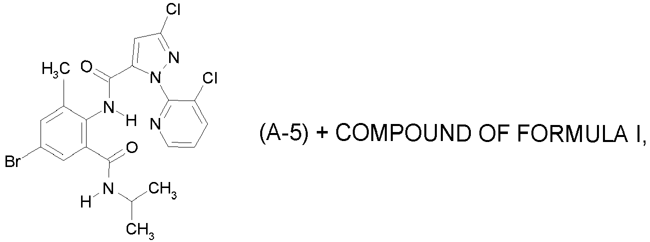 the formula A-6 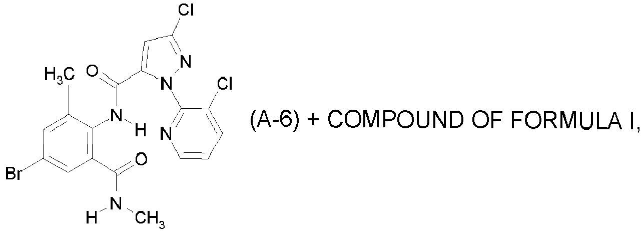 the formula A-7 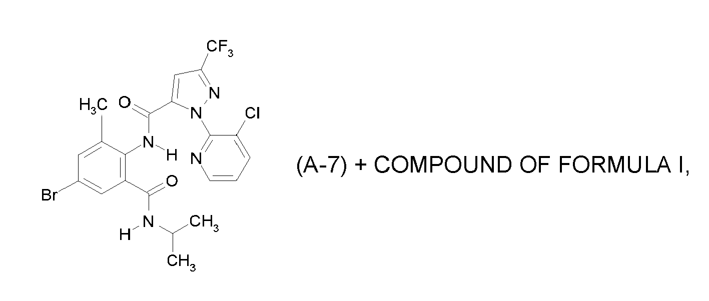 the formula A-8 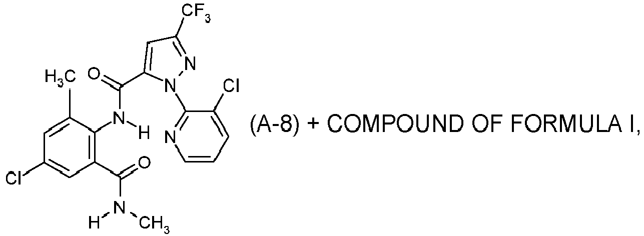 the formula A-9 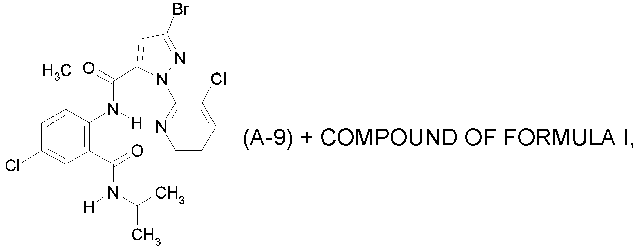 the formula A-10 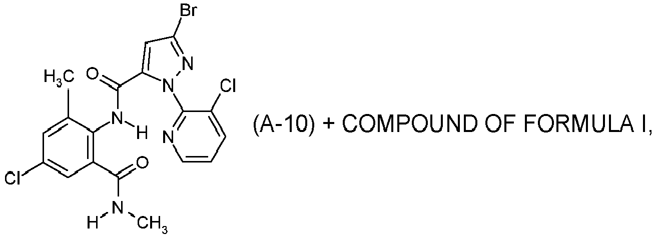 the formula A-11 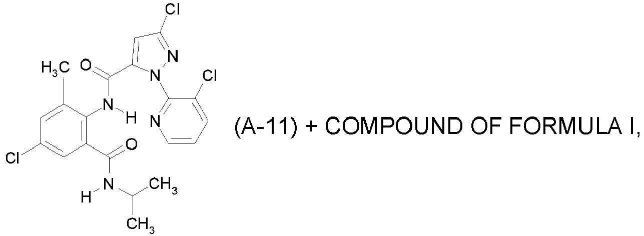 the formula A-12 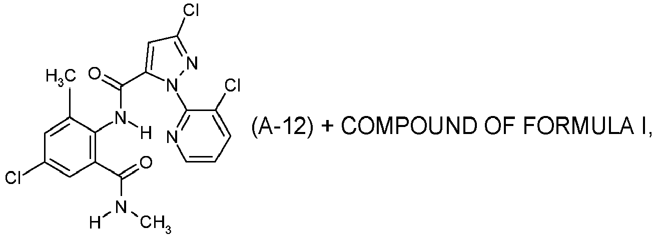 the formula A-13 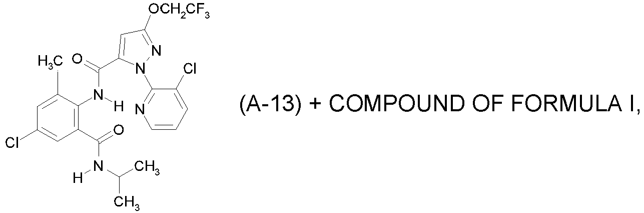 the formula A-14 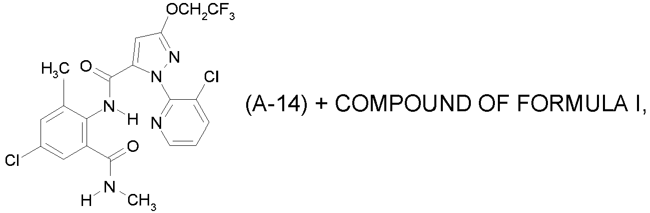 the formula A-15 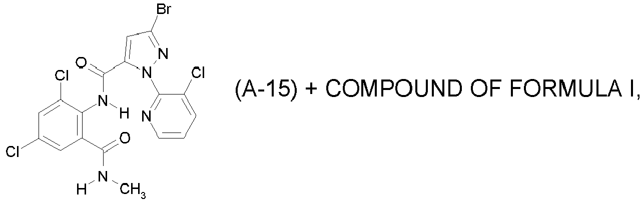 the formula A-16 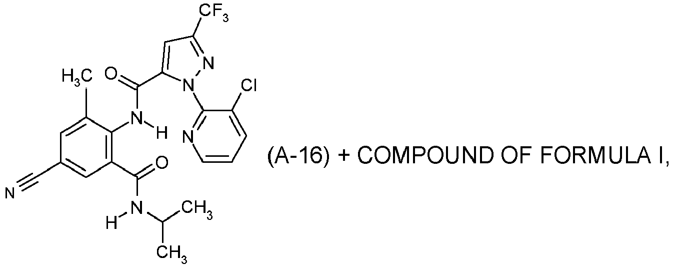 the formula A-17 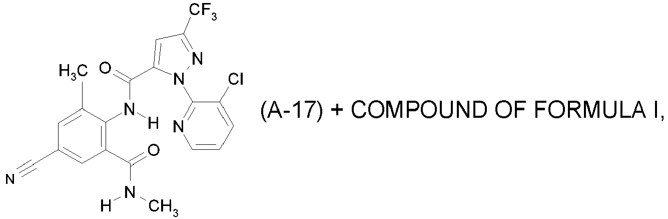 the formula A-18 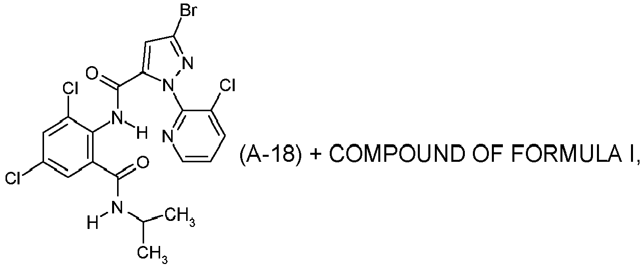 the formula A-19 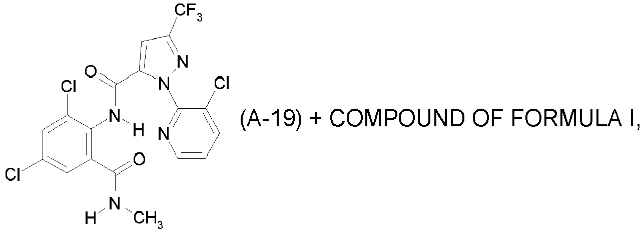 the formula A-20 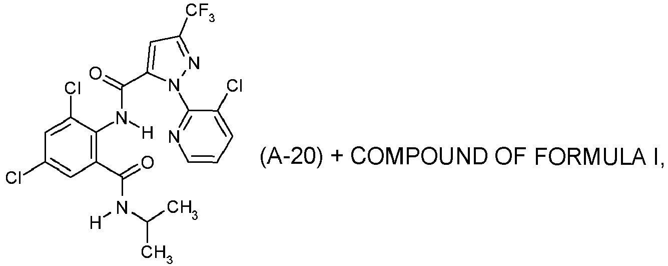 the formula A-21 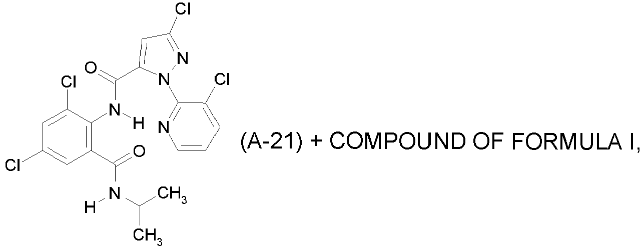 the formula A-22 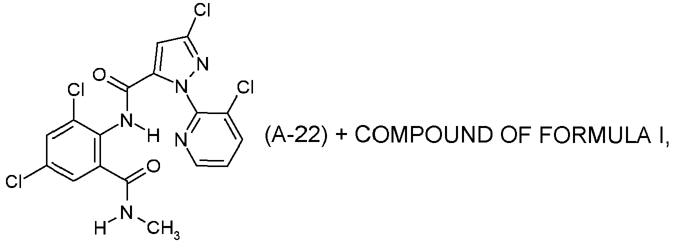 the formula A-23 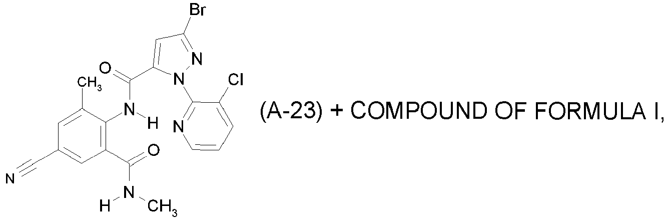 the formula A-24 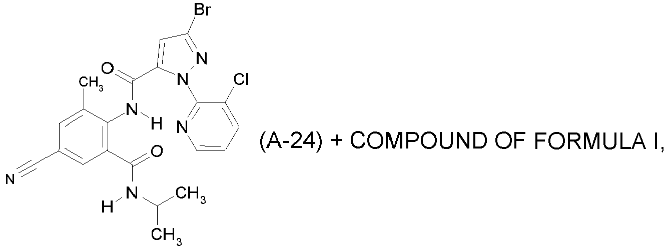 the formula A-25 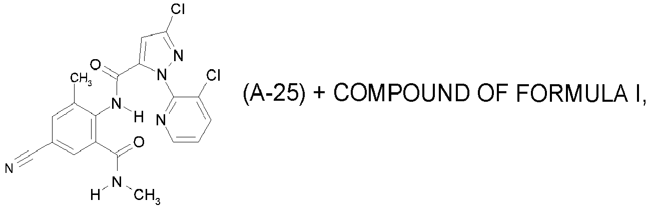 the formula A-26 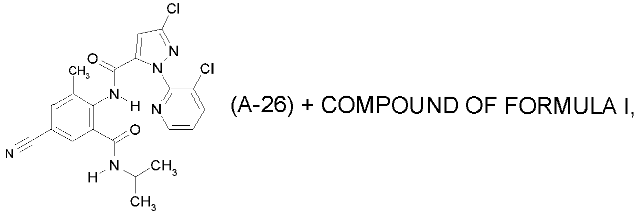 and the formula A-27 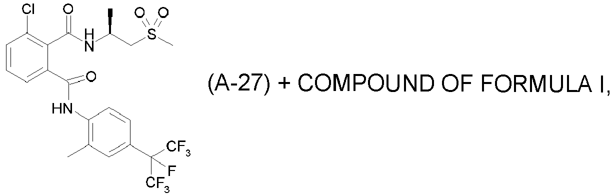 an insecticide selected from the group consisting of the compound of the formula A-28 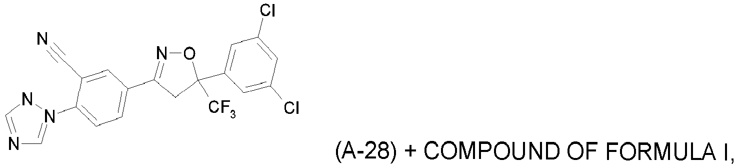 and the formula A-29 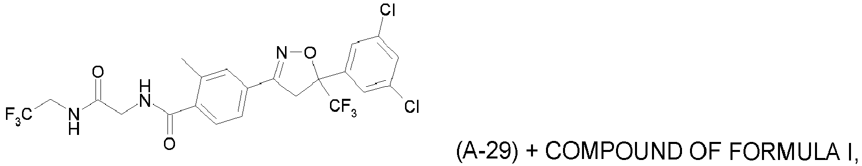 and the formula A-30 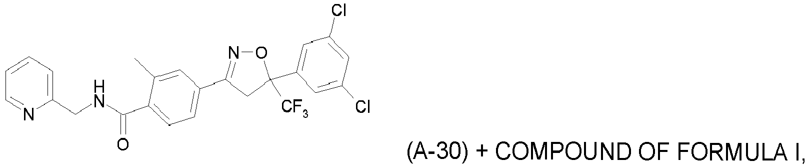 an insecticide selected from the group consisting of the compound of the formula A-31 [BYI2960 (development code)] 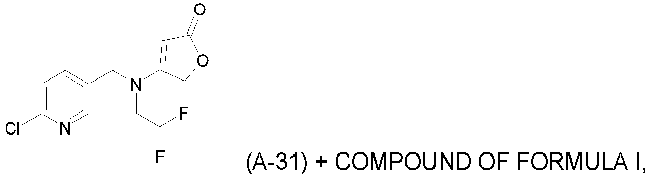 the formula A-32 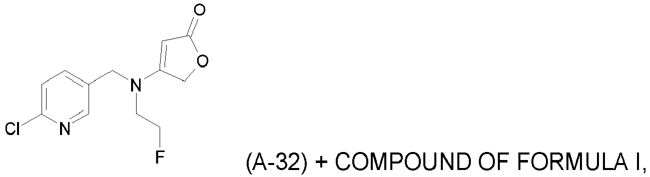 the formula A-33 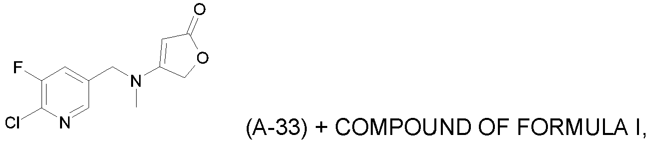 and an insecticide of the formula A-34 [SYN 876 (compound code)]

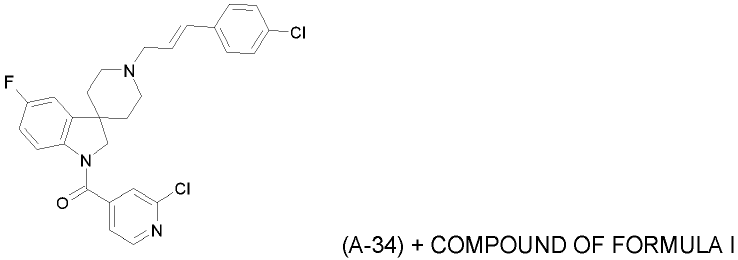

The references in brackets behind the active ingredients, e.g. [3878-19-1] refer to the Chemical Abstracts Registry number. The compounds of the formula A-1 to A-26 are described in WO 03/015518 or in WO 04/067528. The compound of the formula A-27 is described in WO 06/022225 and in WO 07/112844. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.htmL.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The compounds of formula I according to the invention can also be used in combination with one or more fungicides. In particular, in the following mixtures of the compounds of formula I with fungicides, the term COMPOUND OF FORMULA I preferably refers to a compound selected from one of the Tables 1 to 17:
COMPOUND OF FORMULA I + (E)-*N*-methyl-2-[2-(2,5-dimethylphenoxymethyl)phenyl]-2-methoxy-iminoacetamide (SSF-129), COMPOUND OF FORMULA I + 4-bromo-2-cyano-N,N-dimethyl-6-trifluoromethylbenzimidazole-1-sulphonamide,
COMPOUND OF FORMULA I + α-[*N*-(3-chloro-2,6-xylyl)-2-methoxyacetamido]-γ -butyrolactone, COMPOUND OF FORMULA I + 4-chloro-2-cyano-*N,*N-dimethyl-5-p-tolylimidazole-1-sulfonamide (IKF-916, cyamidazosulfamid), COMPOUND OF FORMULA I + 3-5-dichloro-*N*-(3-chloro-1-ethyl-1-methyl-2-oxopropyl)-4-methylbenzamide (RH-7281, zoxamide), COMPOUND OF FORMULA I + *N*-allyl-4,5,-dimethyl-2-trimethylsilylthiophene-3-carboxamide (MON65500), COMPOUND OF FORMULA I + *N*-(1-cyano-1,2-dimethylpropyl)-2-(2,4-dichlorophenoxy)propionamide (AC382042), COMPOUND OF FORMULA I + *N*-(2-methoxy-5-pyridyl)-cyclopropane carboxamide, COMPOUND OF FORMULA I + acibenzolar, COMPOUND OF FORMULA I + alanycarb, COMPOUND OF FORMULA I + aldimorph, COMPOUND OF FORMULA I + ametoctradin, COMPOUND OF FORMULA I + amisulbrom, COMPOUND OF FORMULA I + anilazine, COMPOUND OF FORMULA I + azaconazole, COMPOUND OF FORMULA I + azoxystrobin, COMPOUND OF FORMULA I + benalaxyl, COMPOUND OF FORMULA I + benalaxyl-M, COMPOUND OF FORMULA I + benomyl, COMPOUND OF FORMULA I + benthiavalicarb, COMPOUND OF FORMULA I + benzodiflupyr, COMPOUND OF FORMULA I + benzovindiflupyr, COMPOUND OF FORMULA I + biloxazol, COMPOUND OF FORMULA I + bitertanol, COMPOUND OF FORMULA I + bixafen, COMPOUND OF FORMULA I + blasticidin S, COMPOUND OF FORMULA I + boscalid, COMPOUND OF FORMULA I + bromuconazole, COMPOUND OF FORMULA I + bupirimate, COMPOUND OF FORMULA I + captafol, COMPOUND OF FORMULA I + captan, COMPOUND OF FORMULA I + carbendazim, COMPOUND OF FORMULA I + carbendazim chlorhydrate, COMPOUND OF FORMULA I + carboxin, COMPOUND OF FORMULA I + carpropamid, carvone, COMPOUND OF FORMULA I + CGA41396, COMPOUND OF FORMULA I + CGA41397, COMPOUND OF FORMULA I + chinomethionate, COMPOUND OF FORMULA I + chlazafenone, COMPOUND OF FORMULA I + chlorodincarb, COMPOUND OF FORMULA I + chlorothalonil, COMPOUND OF FORMULA I + chlorozolinate, COMPOUND OF FORMULA I + clozylacon, COMPOUND OF FORMULA I + copper containing compounds such as copper oxychloride, copper oxyquinolate, copper sulphate, copper tallate and Bordeaux mixture, COMPOUND OF FORMULA I + coumoxystrobin, COMPOUND OF FORMULA I + cyazofamid, COMPOUND OF FORMULA I + cyflufenamid, COMPOUND OF FORMULA I + cymoxanil, COMPOUND OF FORMULA I + cyproconazole, COMPOUND OF FORMULA I + cyprodinil, COMPOUND OF FORMULA I + debacarb, COMPOUND OF FORMULA I + di-2-pyridyl disulphide 1,1'-dioxide, COMPOUND OF FORMULA I + dicloaminostrobin, COMPOUND OF FORMULA I + diclofenoxystrobin, COMPOUND OF FORMULA I + dichlofluanid, COMPOUND OF FORMULA I + diclomezine, COMPOUND OF FORMULA I + dicloran, COMPOUND OF FORMULA I + diethofencarb, COMPOUND OF FORMULA I + difenoconazole, COMPOUND OF FORMULA I + difenzoquat, COMPOUND OF FORMULA I + diflumetorim, COMPOUND OF FORMULA I + O,O-di-iso-propyl-S-benzyl thiophosphate, COMPOUND OF FORMULA I + dimefluazole, COMPOUND OF FORMULA I + dimetconazole, COMPOUND OF FORMULA I + dimethomorph, COMPOUND OF FORMULA I + dimethirimol, COMPOUND OF FORMULA I + dimoxystrobin, COMPOUND OF FORMULA I + diniconazole, COMPOUND OF FORMULA I + dinocap, COMPOUND OF FORMULA I + dithianon, COMPOUND OF FORMULA 1+ dodecyl dimethyl ammonium chloride, COMPOUND OF FORMULA I + dodemorph, COMPOUND OF FORMULA I + dodine, COMPOUND OF FORMULA I + doguadine, COMPOUND OF FORMULA I + edifenphos, COMPOUND OF FORMULA I + enoxastrobin, COMPOUND OF FORMULA I + epoxiconazole, COMPOUND OF FORMULA I + ethirimol, COMPOUND OF FORMULA I + ethyl(*Z*)-*N*-benzyl-*N*([methyl(methyl-thioethylideneaminooxycarbonyl)amino]thio)-β -alaninate, COMPOUND OF FORMULA I + etridiazole, COMPOUND OF FORMULA I + famoxadone, COMPOUND OF FORMULA I + fenamidone (RPA407213), COMPOUND OF FORMULA I + fenaminstrobin, COMPOUND OF FORMULA I + fenarimol, COMPOUND OF FORMULA I + fenbuconazole, COMPOUND OF FORMULA I + fenfuram, COMPOUND OF FORMULA I + fenhexamid (KBR2738), COMPOUND OF FORMULA I + fenoxanil, COMPOUND OF FORMULA I + fenoxystrobin, COMPOUND OF FORMULA I + fenpiclonil, COMPOUND OF FORMULA I + fenpropidin, COMPOUND OF FORMULA I + fenpropimorph, COMPOUND OF FORMULA I + fenpyrazamine, COMPOUND OF FORMULA I + fenpyrazamine/ipfenpyrazolone, COMPOUND OF FORMULA I + fentin acetate, COMPOUND OF FORMULA I + fentin hydroxide, COMPOUND OF FORMULA I + ferbam, COMPOUND OF FORMULA I + ferimzone, COMPOUND OF FORMULA I + fluazinam, COMPOUND OF FORMULA I + fludioxonil, COMPOUND OF FORMULA I + flufenoxystrobin, COMPOUND OF FORMULA I + flumetover, COMPOUND OF FORMULA I + flumorph, COMPOUND OF FORMULA I + fluopicolide, COMPOUND OF FORMULA I + fluopyram, COMPOUND OF FORMULA I + fluoxastrobin, COMPOUND OF FORMULA I + fluoroimide, COMPOUND OF FORMULA I + fluquinconazole, COMPOUND OF FORMULA I + flusilazole, COMPOUND OF FORMULA I + flutianil, COMPOUND OF FORMULA I + flutolanil, COMPOUND OF FORMULA I + flutriafol, COMPOUND OF FORMULA I + fluxapyroxad, COMPOUND OF FORMULA I + folpet, COMPOUND OF FORMULA I + fosetyl, COMPOUND OF FORMULA I + fosetyl-aluminium, COMPOUND OF FORMULA I + fuberidazole, COMPOUND OF FORMULA I + furalaxyl, COMPOUND OF FORMULA I + furametpyr, COMPOUND OF FORMULA I + guazatine, COMPOUND OF FORMULA I + hexaconazole, COMPOUND OF FORMULA I + hydroxyisoxazole, COMPOUND OF FORMULA I + hymexazole, COMPOUND OF FORMULA I + imazalil, COMPOUND OF FORMULA I + imibenconazole, COMPOUND OF FORMULA I + iminoctadine, COMPOUND OF FORMULA I + iminoctadine triacetate, COMPOUND OF FORMULA I + ipconazole, COMPOUND OF FORMULA I + iprobenfos, COMPOUND OF FORMULA I + iprodione, COMPOUND OF FORMULA I + iprovalicarb (SZX0722), COMPOUND OF FORMULA I + isofetamid, COMPOUND OF FORMULA I + isopropanyl butyl carbamate, COMPOUND OF FORMULA I + isoprothiolane, COMPOUND OF FORMULA I + isopyrazam, COMPOUND OF FORMULA I + isotianil, COMPOUND OF FORMULA I + kasugamycin, COMPOUND OF FORMULA I + kresoxim-methyl, COMPOUND OF FORMULA I + LY186054, COMPOUND OF FORMULA I + LY211795, COMPOUND OF FORMULA I + LY248908, COMPOUND OF FORMULA I + mancozeb, COMPOUND OF FORMULA I + mandipropamid, COMPOUND OF FORMULA I + maneb, COMPOUND OF FORMULA I + mefenoxam, COMPOUND OF FORMULA I + mepanipyrim, COMPOUND OF FORMULA I + mepronil, COMPOUND OF FORMULA I + meptyldinocap, COMPOUND OF FORMULA I + metalaxyl, COMPOUND OF FORMULA I + metconazole, COMPOUND OF FORMULA I + metiram, COMPOUND OF FORMULA I + metiram-zinc, COMPOUND OF FORMULA I + metominostrobin, COMPOUND OF FORMULA I + metrafenone, COMPOUND OF FORMULA I + myclobutanil, COMPOUND OF FORMULA I + neoasozin, COMPOUND OF FORMULA I + nickel dimethyldithiocarbamate, COMPOUND OF FORMULA I + nicobifen, COMPOUND OF FORMULA I + nitrothal-isopropyl, COMPOUND OF FORMULA I + nuarimol, COMPOUND OF FORMULA I + ofurace, COMPOUND OF FORMULA I + organomercury compounds, COMPOUND OF FORMULA I + orysastrobin, COMPOUND OF FORMULA I + oxadixyl, COMPOUND OF FORMULA I + oxasulfuron, COMPOUND OF FORMULA I + oxolinic acid, COMPOUND OF FORMULA I + oxpoconazole, COMPOUND OF FORMULA I + oxycarboxin, COMPOUND OF FORMULA I + pefurazoate, COMPOUND OF FORMULA I + penconazole, COMPOUND OF FORMULA I + pencycuron, COMPOUND OF FORMULA I + penflufen, COMPOUND OF FORMULA I + penthiopyrad, COMPOUND OF FORMULA I + phenazin oxide, COMPOUND OF FORMULA I + phosetyl-Al, COMPOUND OF FORMULA I + phosphorus acids, COMPOUND OF FORMULA I + phthalide, COMPOUND OF FORMULA I + picoxystrobin (ZA1963), COMPOUND OF FORMULA I + polyoxin D, COMPOUND OF FORMULA I + polyram, COMPOUND OF FORMULA I + probenazole, COMPOUND OF FORMULA I + prochloraz, COMPOUND OF FORMULA I + procymidone, COMPOUND OF FORMULA I + propamocarb, COMPOUND OF FORMULA I + propiconazole, COMPOUND OF FORMULA I + propineb, COMPOUND OF FORMULA I + propionic acid, COMPOUND OF FORMULA I + proquinazid, COMPOUND OF FORMULA I + prothioconazole, COMPOUND OF FORMULA I + pyraclostrobin, COMPOUND OF FORMULA I + pyraoxystrobin, COMPOUND OF FORMULA I + pyrazophos, COMPOUND OF FORMULA I + pyribencarb, COMPOUND OF FORMULA I + pyrifenox, COMPOUND OF FORMULA I + pyrimethanil, COMPOUND OF FORMULA I + pyrisoxazole, COMPOUND OF FORMULA I + pyroquilon, COMPOUND OF FORMULA I + pyroxyfur, COMPOUND OF FORMULA I + pyrrolnitrin, COMPOUND OF FORMULA I + quaternary ammonium compounds, COMPOUND OF FORMULA I + quinomethionate, COMPOUND OF FORMULA I + quinoxyfen, COMPOUND OF FORMULA I + quintozene, COMPOUND OF FORMULA I + sedaxane, COMPOUND OF FORMULA I + sipconazole (F-155), COMPOUND OF FORMULA I + sodium pentachlorophenate, COMPOUND OF FORMULA 1+ spiroxamine, COMPOUND OF FORMULA I + streptomycin, COMPOUND OF FORMULA I + sulphur, COMPOUND OF FORMULA I + tebuconazole, COMPOUND OF FORMULA I + tecloftalam, COMPOUND OF FORMULA I + tecnazene, COMPOUND OF FORMULA I + terbufloquin, COMPOUND OF FORMULA I + tetraconazole, COMPOUND OF FORMULA I + thiabendazole, COMPOUND OF FORMULA I + thifluzamid, COMPOUND OF FORMULA I + 2-(thiocyanomethylthio)benzothiazole, COMPOUND OF FORMULA I + thiophanate-methyl, COMPOUND OF FORMULA I + thiram, COMPOUND OF FORMULA I + tiadinil, COMPOUND OF FORMULA I + timibenconazole, COMPOUND OF FORMULA I + tolclofos-methyl, COMPOUND OF FORMULA I + tolprocarb, COMPOUND OF FORMULA I + tolylfluanid, COMPOUND OF FORMULA I + triadimefon, COMPOUND OF FORMULA I + triadimenol, COMPOUND OF FORMULA I + triazbutil, COMPOUND OF FORMULA I + triazoxide, COMPOUND OF FORMULA I + triclopyricarb, COMPOUND OF FORMULA I + tricyclazole, COMPOUND OF FORMULA I + tridemorph, COMPOUND OF FORMULA I + trifloxystrobin, COMPOUND OF FORMULA I + triforine, COMPOUND OF FORMULA I + triflumizole, COMPOUND OF FORMULA I + triticonazole, COMPOUND OF FORMULA I + validamycin A, COMPOUND OF FORMULA I + valiphenal, COMPOUND OF FORMULA I + vapam, COMPOUND OF FORMULA I + vinclozolin, COMPOUND OF FORMULA I + zineb and COMPOUND OF FORMULA I + ziram.

The compounds of formula I may be mixed with soil, peat or other rooting media for the protection of plants against seed-borne, soil-borne or foliar fungal diseases.

The compounds of formula I according to the invention can also be used in combination with one or more other synergists. In particular, the following mixtures of the COMPOUND OF FORMULA I, where this term preferably refers to a compound selected from one of the Tables 1 to 17, are important:
COMPOUND OF FORMULA I + piperonyl butoxide, COMPOUND OF FORMULA I + sesamex, COMPOUND OF FORMULA I + safroxan and COMPOUND OF FORMULA I + dodecyl imidazole.

The compounds of formula I according to the invention can also be used in combination with one or more other herbicides. In particular, the following mixtures of the COMPOUND OF FORMULA I, where this term preferably refers to a compound selected from one of the Tables 1 to 17, are important:
COMPOUND OF FORMULA I + acetochlor, COMPOUND OF FORMULA I + acifluorfen, COMPOUND OF FORMULA I + acifluorfen-sodium, COMPOUND OF FORMULA I + aclonifen, COMPOUND OF FORMULA I + acrolein, COMPOUND OF FORMULA I + alachlor, COMPOUND OF FORMULA I + alloxydim, COMPOUND OF FORMULA I + allyl alcohol, COMPOUND OF FORMULA I + ametryn, COMPOUND OF FORMULA I + amicarbazone, COMPOUND OF FORMULA I + amidosulfuron, COMPOUND OF FORMULA I + aminocyclopyrachlor, COMPOUND OF FORMULA I + aminopyralid, COMPOUND OF FORMULA I + amitrole, COMPOUND OF FORMULA I + ammonium sulfamate, COMPOUND OF FORMULA I + anilofos, COMPOUND OF FORMULA I + asulam, COMPOUND OF FORMULA I + atraton, COMPOUND OF FORMULA I + atrazine, COMPOUND OF FORMULA I + azimsulfuron, COMPOUND OF FORMULA I + BCPC, COMPOUND OF FORMULA I + beflubutamid, COMPOUND OF FORMULA I + benazolin, COMPOUND OF FORMULA I +bencarbazone, COMPOUND OF FORMULA I + benfluralin, COMPOUND OF FORMULA I + benfuresate, COMPOUND OF FORMULA I + bensulfuron, COMPOUND OF FORMULA I + bensulfuron-methyl, COMPOUND OF FORMULA I + bensulide, COMPOUND OF FORMULA I + bentazone, COMPOUND OF FORMULA I + benzfendizone, COMPOUND OF FORMULA I + benzobicyclon, COMPOUND OF FORMULA I + benzofenap, COMPOUND OF FORMULA I + bicyclopyrone, COMPOUND OF FORMULA I + bifenox, COMPOUND OF FORMULA I + bilanafos, COMPOUND OF FORMULA I + bispyribac, COMPOUND OF FORMULA I + bispyribac-sodium, COMPOUND OF FORMULA I + borax, COMPOUND OF FORMULA I + bromacil, COMPOUND OF FORMULA I + bromobutide, COMPOUND OF FORMULA I + bromoxynil, COMPOUND OF FORMULA I + butachlor, COMPOUND OF FORMULA I + butafenacil, COMPOUND OF FORMULA I + butamifos, COMPOUND OF FORMULA I + butralin, COMPOUND OF FORMULA I + butroxydim, COMPOUND OF FORMULA I + butylate, COMPOUND OF FORMULA I + cacodylic acid, COMPOUND OF FORMULA I + calcium chlorate, COMPOUND OF FORMULA I + cafenstrole, COMPOUND OF FORMULA I + carbetamide, COMPOUND OF FORMULA I + carfentrazone, COMPOUND OF FORMULA I + carfentrazone-ethyl, COMPOUND OF FORMULA I + CDEA, COMPOUND OF FORMULA I + CEPC, COMPOUND OF FORMULA I + chlorflurenol, COMPOUND OF FORMULA I + chlorflurenol-methyl, COMPOUND OF FORMULA I + chloridazon, COMPOUND OF FORMULA I + chlorimuron, COMPOUND OF FORMULA I + chlorimuron-ethyl, COMPOUND OF FORMULA I + chloroacetic acid, COMPOUND OF FORMULA I + chlorotoluron, COMPOUND OF FORMULA I + chlorpropham, COMPOUND OF FORMULA I + chlorsulfuron, COMPOUND OF FORMULA I + chlorthal, COMPOUND OF FORMULA I + chlorthal-dimethyl, COMPOUND OF FORMULA I + cinidon-ethyl, COMPOUND OF FORMULA I + cinmethylin, COMPOUND OF FORMULA I + cinosulfuron, COMPOUND OF FORMULA I + cisanilide, COMPOUND OF FORMULA I + clacyfos, COMPOUND OF FORMULA I + clethodim, COMPOUND OF FORMULA I + clodinafop, COMPOUND OF FORMULA I + clodinafop-propargyl, COMPOUND OF FORMULA I + clomazone, COMPOUND OF FORMULA I + clomeprop, COMPOUND OF FORMULA I + clopyralid, COMPOUND OF FORMULA I + cloransulam, COMPOUND OF FORMULA I + cloransulammethyl, COMPOUND OF FORMULA I + CMA, COMPOUND OF FORMULA I + 4-CPB, COMPOUND OF FORMULA I + CPMF, COMPOUND OF FORMULA I + 4-CPP, COMPOUND OF FORMULA I + CPPC, COMPOUND OF FORMULA I + cresol, COMPOUND OF FORMULA I + cumyluron, COMPOUND OF FORMULA I + cyanamide, COMPOUND OF FORMULA I + cyanazine, COMPOUND OF FORMULA I + cycloate, COMPOUND OF FORMULA I + cyclosulfamuron, COMPOUND OF FORMULA I + cycloxydim, COMPOUND OF FORMULA I + cyhalofop, COMPOUND OF FORMULA I + cyhalofop-butyl, COMPOUND OF FORMULA I + 2,4-D, COMPOUND OF FORMULA I + 3,4-DA, COMPOUND OF FORMULA I + daimuron, COMPOUND OF FORMULA I + dalapon, COMPOUND OF FORMULA I + dazomet, COMPOUND OF FORMULA I + 2,4-DB, COMPOUND OF FORMULA I + 3,4-DB, COMPOUND OF FORMULA I + 2,4-DEB, COMPOUND OF FORMULA I + desmedipham, COMPOUND OF FORMULA I + dicamba, COMPOUND OF FORMULA I + dichlobenil, COMPOUND OF FORMULA I + ortho-dichlorobenzene, COMPOUND OF FORMULA I + para-dichlorobenzene, COMPOUND OF FORMULA I + dichlorprop, COMPOUND OF FORMULA I + dichlorprop-P, COMPOUND OF FORMULA I + diclofop, COMPOUND OF FORMULA I + diclofop-methyl, COMPOUND OF FORMULA I + diclosulam, COMPOUND OF FORMULA I + difenzoquat, COMPOUND OF FORMULA I + difenzoquat metilsulfate, COMPOUND OF FORMULA I + diflufenican, COMPOUND OF FORMULA I + diflufenzopyr, COMPOUND OF FORMULA I + dimefuron, COMPOUND OF FORMULA I + dimepiperate, COMPOUND OF FORMULA I + dimethachlor, COMPOUND OF FORMULA I + dimethametryn, COMPOUND OF FORMULA I + dimethenamid, COMPOUND OF FORMULA I + dimethenamid-P, COMPOUND OF FORMULA I + dimethipin, COMPOUND OF FORMULA I + dimethylarsinic acid, COMPOUND OF FORMULA I + dinitramine, COMPOUND OF FORMULA I + dinoterb, COMPOUND OF FORMULA I + diphenamid, COMPOUND OF FORMULA I + diquat, COMPOUND OF FORMULA I + diquat dibromide, COMPOUND OF FORMULA I + dithiopyr, COMPOUND OF FORMULA I + diuron, COMPOUND OF FORMULA I + DNOC, COMPOUND OF FORMULA I + 3,4-DP, COMPOUND OF FORMULA I + DSMA, COMPOUND OF FORMULA I + EBEP, COMPOUND OF FORMULA I + endothal, COMPOUND OF FORMULA I + EPTC, COMPOUND OF FORMULA I + esprocarb, COMPOUND OF FORMULA I + ethalfluralin, COMPOUND OF FORMULA I + ethametsulfuron, COMPOUND OF FORMULA I + ethametsulfuron-methyl, COMPOUND OF FORMULA I + ethofumesate, COMPOUND OF FORMULA I + ethoxyfen, COMPOUND OF FORMULA I + ethoxysulfuron, COMPOUND OF FORMULA I + etobenzanid, COMPOUND OF FORMULA I + fenoxaprop-P, COMPOUND OF FORMULA I + fenoxaprop-P-ethyl, COMPOUND OF FORMULA I + fenoxasulfone, COMPOUND OF FORMULA I + fentrazamide, COMPOUND OF FORMULA I + ferrous sulfate, COMPOUND OF FORMULA I + flamprop-M, COMPOUND OF FORMULA I + flazasulfuron, COMPOUND OF FORMULA I + florasulam, COMPOUND OF FORMULA I + fluazifop, COMPOUND OF FORMULA I + fluazifop-butyl, COMPOUND OF FORMULA I + fluazifop-P, COMPOUND OF FORMULA I + fluazifop-P-butyl, COMPOUND OF FORMULA I + flucarbazone, COMPOUND OF FORMULA I + flucarbazone-sodium, COMPOUND OF FORMULA I + flucetosulfuron, COMPOUND OF FORMULA I + fluchloralin, COMPOUND OF FORMULA I + flufenacet, COMPOUND OF FORMULA I + flufenpyr, COMPOUND OF FORMULA I + flufenpyr-ethyl, COMPOUND OF FORMULA I + flumetsulam, COMPOUND OF FORMULA I + flumiclorac, COMPOUND OF FORMULA I + flumiclorac-pentyl, COMPOUND OF FORMULA I + flumioxazin, COMPOUND OF FORMULA I + fluometuron, COMPOUND OF FORMULA I + fluoroglycofen, COMPOUND OF FORMULA I + fluoroglycofen-ethyl, COMPOUND OF FORMULA I + flupropanate, COMPOUND OF FORMULA I + flupyrsulfuron, COMPOUND OF FORMULA I + flupyrsulfuron-methyl-sodium, COMPOUND OF FORMULA I + flurenol, COMPOUND OF FORMULA I + fluridone, COMPOUND OF FORMULA I + flurochloridone, COMPOUND OF FORMULA I + fluroxypyr, COMPOUND OF FORMULA I + flurtamone, COMPOUND OF FORMULA I + fluthiacet, COMPOUND OF FORMULA I + fluthiacet-methyl, COMPOUND OF FORMULA I + fomesafen, COMPOUND OF FORMULA I + foramsulfuron, COMPOUND OF FORMULA I + fosamine, COMPOUND OF FORMULA I + glufosinate, COMPOUND OF FORMULA I + glufosinate-ammonium, COMPOUND OF FORMULA I + glyphosate, COMPOUND OF FORMULA I + halosulfuron, COMPOUND OF FORMULA I + halosulfuron-methyl, COMPOUND OF FORMULA I + haloxyfop, COMPOUND OF FORMULA I + haloxyfop-P, COMPOUND OF FORMULA I + HC-252, COMPOUND OF FORMULA I + hexazinone, COMPOUND OF FORMULA I + imazamethabenz, COMPOUND OF FORMULA I + imazamethabenz-methyl, COMPOUND OF FORMULA I + imazamox, COMPOUND OF FORMULA I + imazapic, COMPOUND OF FORMULA I + imazapyr, COMPOUND OF FORMULA I + imazaquin, COMPOUND OF FORMULA I + imazethapyr, COMPOUND OF FORMULA I + imazosulfuron, COMPOUND OF FORMULA I + indanofan, COMPOUND OF FORMULA I + indaziflam, COMPOUND OF FORMULA I + iodomethane, COMPOUND OF FORMULA I + iodosulfuron, COMPOUND OF FORMULA I + iodosulfuron-methyl-sodium, COMPOUND OF FORMULA I + iofensulfuron, COMPOUND OF FORMULA I + ioxynil, COMPOUND OF FORMULA I + isoproturon, COMPOUND OF FORMULA I + ipfencarbazone, COMPOUND OF FORMULA I + isouron, COMPOUND OF FORMULA I + isoxaben, COMPOUND OF FORMULA I + isoxachlortole, COMPOUND OF FORMULA I + isoxaflutole, COMPOUND OF FORMULA I + karbutilate, COMPOUND OF FORMULA I + lactofen, COMPOUND OF FORMULA I + lenacil, COMPOUND OF FORMULA I + linuron, COMPOUND OF FORMULA I + MAA, COMPOUND OF FORMULA I + MAMA, COMPOUND OF FORMULA I + MCPA, COMPOUND OF FORMULA I + MCPA-thioethyl, COMPOUND OF FORMULA I + MCPB, COMPOUND OF FORMULA I + mecoprop, COMPOUND OF FORMULA I + mecoprop-P, COMPOUND OF FORMULA I + mefenacet, COMPOUND OF FORMULA I + mefluidide, COMPOUND OF FORMULA I + mesosulfuron, COMPOUND OF FORMULA I + mesosulfuron-methyl, COMPOUND OF FORMULA I + mesotrione, COMPOUND OF FORMULA I + metam, COMPOUND OF FORMULA I + metamifop, COMPOUND OF FORMULA I + metamitron, COMPOUND OF FORMULA I + metazachlor, COMPOUND OF FORMULA I + metazasulfuron, COMPOUND OF FORMULA I + methabenzthiazuron, COMPOUND OF FORMULA I + methylarsonic acid, COMPOUND OF FORMULA I + methyldymron, COMPOUND OF FORMULA I + methyl isothiocyanate, COMPOUND OF FORMULA I + metiozolin, COMPOUND OF FORMULA I + metobenzuron, COMPOUND OF FORMULA I + metolachlor, COMPOUND OF FORMULA I + S-metolachlor, COMPOUND OF FORMULA I + metosulam, COMPOUND OF FORMULA I + metoxuron, COMPOUND OF FORMULA I + metribuzin, COMPOUND OF FORMULA I + metsulfuron, COMPOUND OF FORMULA I + metsulfuron-methyl, COMPOUND OF FORMULA I + MK-616, COMPOUND OF FORMULA I + molinate, COMPOUND OF FORMULA I + monolinuron, COMPOUND OF FORMULA I + MSMA, COMPOUND OF FORMULA I + naproanilide, COMPOUND OF FORMULA I + napropamide, COMPOUND OF FORMULA I + naptalam, COMPOUND OF FORMULA I + neburon, COMPOUND OF FORMULA I + nicosulfuron, COMPOUND OF FORMULA I + nonanoic acid, COMPOUND OF FORMULA I + norflurazon, COMPOUND OF FORMULA I + oleic acid (fatty acids), COMPOUND OF FORMULA I + orbencarb, COMPOUND OF FORMULA I + orthosulfamuron, COMPOUND OF FORMULA I + oryzalin, COMPOUND OF FORMULA I + oxadiargyl, COMPOUND OF FORMULA I + oxadiazon, COMPOUND OF FORMULA I + oxasulfuron, COMPOUND OF FORMULA I + oxaziclomefone, COMPOUND OF FORMULA I + oxyfluorfen, COMPOUND OF FORMULA I + paraquat, COMPOUND OF FORMULA I + paraquat dichloride, COMPOUND OF FORMULA I + pebulate, COMPOUND OF FORMULA I + pendimethalin, COMPOUND OF FORMULA I + penoxsulam, COMPOUND OF FORMULA I + pentachlorophenol, COMPOUND OF FORMULA I + pentanochlor, COMPOUND OF FORMULA I + pentoxazone, COMPOUND OF FORMULA I + pethoxamid, COMPOUND OF FORMULA I + petrolium oils, COMPOUND OF FORMULA I + phenmedipham, COMPOUND OF FORMULA I + phenmedipham-ethyl, COMPOUND OF FORMULA I + picloram, COMPOUND OF FORMULA I + picolinafen, COMPOUND OF FORMULA I + pinoxaden, COMPOUND OF FORMULA I + piperophos, COMPOUND OF FORMULA I + potassium arsenite, COMPOUND OF FORMULA I + potassium azide, COMPOUND OF FORMULA I + pretilachlor, COMPOUND OF FORMULA I + primisulfuron, COMPOUND OF FORMULA I + primisulfuron-methyl, COMPOUND OF FORMULA I + prodiamine, COMPOUND OF FORMULA I + profluazol, COMPOUND OF FORMULA I + profoxydim, COMPOUND OF FORMULA I + prometon, COMPOUND OF FORMULA I + prometryn, COMPOUND OF FORMULA I + propachlor, COMPOUND OF FORMULA I + propanil, COMPOUND OF FORMULA I + propaquizafop, COMPOUND OF FORMULA I + propazine, COMPOUND OF FORMULA I + propham, COMPOUND OF FORMULA I + propisochlor, COMPOUND OF FORMULA I + propoxycarbazone, COMPOUND OF FORMULA I + propoxycarbazone-sodium, COMPOUND OF FORMULA I + propyrisulfuron, COMPOUND OF FORMULA I + propyzamide, COMPOUND OF FORMULA I + prosulfocarb, COMPOUND OF FORMULA I + prosulfuron, COMPOUND OF FORMULA I + pyraclonil, COMPOUND OF FORMULA I + pyraflufen, COMPOUND OF FORMULA I + pyraflufenethyl, COMPOUND OF FORMULA I + pyrasulfutole, COMPOUND OF FORMULA I + pyrazolynate, COMPOUND OF FORMULA I + pyrazosulfuron, COMPOUND OF FORMULA I + pyrazosulfuron-ethyl, COMPOUND OF FORMULA I + pyrazoxyfen, COMPOUND OF FORMULA I + pyribenzoxim, COMPOUND OF FORMULA I + pyributicarb, COMPOUND OF FORMULA I + pyridafol, COMPOUND OF FORMULA I + pyridate, COMPOUND OF FORMULA I + pyriftalid, COMPOUND OF FORMULA I + pyriminobac, COMPOUND OF FORMULA I + pyriminobac-methyl, COMPOUND OF FORMULA I + pyrimisulfan, COMPOUND OF FORMULA I + pyrithiobac, COMPOUND OF FORMULA I + pyrithiobac-sodium, COMPOUND OF FORMULA I + pyroxsulam, COMPOUND OF FORMULA I + pyroxasulfone, COMPOUND OF FORMULA I + quinclorac, COMPOUND OF FORMULA I + quinmerac, COMPOUND OF FORMULA I + quinoclamine, COMPOUND OF FORMULA I + quizalofop, COMPOUND OF FORMULA I + quizalofop-P, COMPOUND OF FORMULA I + rimsulfuron, COMPOUND OF FORMULA I + saflufenacil, COMPOUND OF FORMULA I + sethoxydim, COMPOUND OF FORMULA I + siduron, COMPOUND OF FORMULA I + simazine, COMPOUND OF FORMULA I + simetryn, COMPOUND OF FORMULA I + SMA, COMPOUND OF FORMULA I + sodium arsenite, COMPOUND OF FORMULA I + sodium azide, COMPOUND OF FORMULA I + sodium chlorate, COMPOUND OF FORMULA I + sulcotrione, COMPOUND OF FORMULA I + sulfentrazone, COMPOUND OF FORMULA I + sulfometuron, COMPOUND OF FORMULA I + sulfometuron-methyl, COMPOUND OF FORMULA I + sulfosate, COMPOUND OF FORMULA I + sulfosulfuron, COMPOUND OF FORMULA I + sulfuric acid, COMPOUND OF FORMULA I + tar oils, COMPOUND OF FORMULA I + 2,3,6-TBA, COMPOUND OF FORMULA I + TCA, COMPOUND OF FORMULA I + TCA-sodium, COMPOUND OF FORMULA I + tebuthiuron, COMPOUND OF FORMULA I + tefuryltrione, COMPOUND OF FORMULA I + tembotrione, COMPOUND OF FORMULA I + tepraloxydim, COMPOUND OF FORMULA I + terbacil, COMPOUND OF FORMULA I + terbumeton, COMPOUND OF FORMULA I + terbuthylazine, COMPOUND OF FORMULA I + terbutryn, COMPOUND OF FORMULA I + thenylchlor, COMPOUND OF FORMULA I + thiazopyr, COMPOUND OF FORMULA I + thiencarbazone, COMPOUND OF FORMULA I + thiencarbazone-methyl, COMPOUND OF FORMULA I + thifensulfuron, COMPOUND OF FORMULA I + thifensulfuron-methyl, COMPOUND OF FORMULA I + thiobencarb, COMPOUND OF FORMULA I + tiocarbazil, COMPOUND OF FORMULA I + topramezone, COMPOUND OF FORMULA I + tralkoxydim, COMPOUND OF FORMULA I + triafamone, COMPOUND OF FORMULA I + tri-allate, COMPOUND OF FORMULA I + triasulfuron, COMPOUND OF FORMULA I + triaziflam, COMPOUND OF FORMULA I + tribenuron, COMPOUND OF FORMULA I + tribenuron-methyl, COMPOUND OF FORMULA I + tricamba, COMPOUND OF FORMULA I + triclopyr, COMPOUND OF FORMULA I + trietazine, COMPOUND OF FORMULA I + trifloxysulfuron, COMPOUND OF FORMULA I + trifloxysulfuron-sodium, COMPOUND OF FORMULA I + trifluralin, COMPOUND OF FORMULA I + triflusulfuron, COMPOUND OF FORMULA I + triflusulfuron-methyl, COMPOUND OF FORMULA I + trihydroxytriazine, COMPOUND OF FORMULA I + tritosulfuron, COMPOUND OF FORMULA I + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6), COMPOUND OF FORMULA I + 4-[(4,5-dihydro-3-methoxy-4-methyl-5-oxo)-1H-1,2,4-triazol-1-ylcarbonylsulfamoyl]-5-methylthiophene-3-carboxylic acid (BAY636), COMPOUND OF FORMULA I + BAY747 (CAS RN 335104-84-2), COMPOUND OF FORMULA I + topramezone (CAS RN 210631-68-8), COMPOUND OF FORMULA I + 4-hydroxy-3-[[2-[(2-methoxyethoxy)methyl]-6-(trifluoromethyl)-3-pyridinyl]carbonyl]-bicyclo[3.2.1]oct-3-en-2-one (CAS RN 352010-68-5), and COMPOUND OF FORMULA I + 4-hydroxy-3-[[2-(3-methoxypropyl)-6-(difluoromethyl)-3-pyridinyl]carbonyl]-bicyclo[3.2.1]oct-3-en-2-one, COMPOUND OF FORMULA I + N-[2-[(4,6-dimethoxy-1,3,5-triazin-2-yl)carbonyl]-6-fluorophenyl]-1,1-difluoro-N-methylmethanesulfonamide (CAS RN 874195-61-6), COMPOUND OF FORMULA I + N-[(1R,2S)-2,6-dimethyl-2,3-dihydro-1*H*-inden-1-yl]-6-(1-fluoroethyl)-1,3,5-triazine-2,4-diamine (CAS RN 950782-86-2), COMPOUND OF FORMULA I + 1-(2-chloro-6-propylimidazo[1,2-b]pyridazin-3-ylsulfonyl)-3-(4,6-dimethoxypyrimidin-2-yl)urea (CAS RN 570415-88-2), and 5-(2,6-difluoro-benzyloxymethyl)-5-methyl-3-(3-methyl-thiophen-2-yl)-4,5-dihydro-isoxazole (CAS RN 403640-27-7) and COMPOUND OF FORMULA I + ZJ0273.

The compounds of formula I according to the invention can also be used in combination with safeners. Preferably, in these mixtures, the compound of the formula I is one of those compounds listed in Tables 1 to 17 below. The following mixtures with safeners, especially, come into consideration:
COMPOUND OF FORMULA I + cloquintocet-mexyl, COMPOUND OF FORMULA I + cloquintocet acid and salts thereof, COMPOUND OF FORMULA I + cyprosulfamide, COMPOUND OF FORMULA I + fenchlorazole-ethyl, COMPOUND OF FORMULA I + fenchlorazole acid and salts thereof, COMPOUND OF FORMULA I + mefenpyr-diethyl, COMPOUND OF FORMULA I + mefenpyr diacid, COMPOUND OF FORMULA I + isoxadifen-ethyl, COMPOUND OF FORMULA I + isoxadifen acid, COMPOUND OF FORMULA I + furilazole, COMPOUND OF FORMULA I + furilazole R isomer, compound of formula (I) + N-(2-methoxybenzoyl)-4-[(methylaminocarbonyl)amino]benzenesulfonamide, COMPOUND OF FORMULA I + benoxacor, COMPOUND OF FORMULA I + dichlormid, COMPOUND OF FORMULA I + AD-67, COMPOUND OF FORMULA I + oxabetrinil, COMPOUND OF FORMULA I + cyometrinil, COMPOUND OF FORMULA I + cyometrinil Z-isomer, COMPOUND OF FORMULA I + fenclorim, COMPOUND OF FORMULA I + cyprosulfamide, COMPOUND OF FORMULA I + naphthalic anhydride, COMPOUND OF FORMULA I + flurazole, COMPOUND OF FORMULA I + CL 304,415, COMPOUND OF FORMULA I + dicyclonon, COMPOUND OF FORMULA I + fluxofenim, COMPOUND OF FORMULA I + DKA-24, COMPOUND OF FORMULA I + R-29148 and COMPOUND OF FORMULA I + PPG-1292. A safening effect can also be observed for the mixtures compound of the formula I + dymron, compound of the formula I + MCPA, compound of the formula I + mecoprop and compound of the formula I + mecoprop-P.

The mixing partners for the COMPOUND OF FORMULA I may also be in the form of esters or salts, as mentioned e.g. in The Pesticide Manual, 12th Edition (BCPC) 2000.

In the above different lists of active ingredients to be mixed with a COMPOUND OF FORMULA I, the compound of the formula I is preferably a compound of Tables 1 to 17, whereby G can be hydrogen, C(O)OEt or C(O)OiPr.

In the above-mentioned mixtures of compounds of formula I, in particular a compound selected from said Tables 1 to 17, with other insecticides, fungicides, herbicides, safeners, adjuvants and the like, the mixing ratios can vary over a large range and are, preferably 100:1 to 1:6000, especially 50:1 to 1:50, more especially 20:1 to 1:20, even more especially 10:1 1 to 1:10. Those mixing ratios are understood to include, on the one hand, ratios by weight and also, on other hand, molar ratios.

The mixtures can advantageously be used in the above-mentioned formulations (in which case "active ingredient" relates to the respective mixture of compound of formula I with the mixing partner).

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The mixtures comprising a compound of formula I selected from Tables 1 to 17 and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables 1 to 17 and the active ingredients as described above is not essential for working the present invention.

The invention is illustrated by the following preparation examples. The H-NMR data of certain compounds of this invention show line broadening at room temperature, suggesting the existence of plural conformational isomers due to, for example keto-enol tautomerism, hindered rotation, ring inversion in the piperidine moitey or nitrogen inversion at the piperidine N-OR center. Broad signals have been labeled with 'br' accordingly.

### EXAMPLE 1: Preparation of Carbonic acid cis-3-(2,5-dimethyl-phenyl)-8-methoxy-2-oxo-1-phenyl-1-aza-spiror4.51dec-3-en-4-yl ester ethyl ester (compound P1.1) and cis-3-(2,5-dimethyl-phenyl)-8-methoxy-1-phenyl-1-aza-spiro[4.5]decane-2,4-dione (compound P2.1)

### Step 1: Preparation of Carbonic acid cis-3-(2,5-dimethyl-phenyl)-8-methoxy-2-oxo-1-phenyl-1-aza-spiro[4.5]dec-3-en-4-yl ester ethyl ester (compound P1.1)

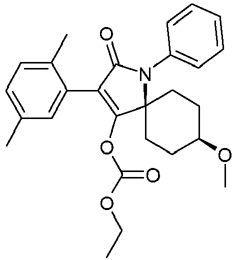

A slurry of carbonic acid *cis*-3-(2,5-dimethyl-phenyl)-8-methoxy-2-oxo-1-aza-spiro[4.5]dec-3-en-4-yl ester ethyl ester (spirotetramat, 300 mg, 0.803 mmol), triphenylbismuthane (707.5 mg, 1.607 mmol), anhydrous copper(II) acetate (223 mg, 1.205 mmol) and triethylamine (0.167 ml, 122 mg, 1.205 mmol) in dichloromethane (1.5 ml) was stirred at room temperature for five days. The reaction mixture was diluted with dichloromethane (25 ml), filtered through Hyflo (calcined diatomaceous earth) and the filtrate concentrated *in vacuo.* The residue was purified by chromatography on silica gel (ethyl acetate/cyclohexane 1:3). Yield: 194 mg of carbonic acid *cis*-3-(2,5-dimethyl-phenyl)-8-methoxy-2-oxo-1-phenyl-1-aza-spiro[4.5]dec-3-en-4-yl ester ethyl ester (compound P1.1) as a white solid, mp 114-116°C. ¹H-NMR (400 MHz, CDCl₃): δ 1.07 (t, *J*= 7.1 Hz, 3H), 1.79-1.93 (m, 6H), 2.19 (br m, 2H), 2.27 (s, 3H), 2.29 (s, 3H), 3.17 (s, 3H), 3.39 (m, 1 H), 4.01 (q, *J* = 7.1 Hz, 2H), 7.03 (m, 1 H), 7.07 (s, 1 H), 7.10 (d, *J* = 7.8 Hz, 1 H), 7.25 (m, 2H), 7.38 (m, 1 H), 7.44 (m, 2H) ppm. LC/MS (ES+): 450 (M+H)⁺; Rₜ = 1.91 min

### Step 2: Preparation of cis-3-(2,5-Dimethyl-phenyl)-8-methoxy-1-phenyl-1-aza-spiro[4.5]decane-2,4-dione (compound P2.1)

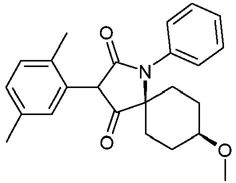

To a stirred suspension of carbonic acid *cis*-3-(2,5-dimethyl-phenyl)-8-methoxy-2-oxo-1-phenyl-1-aza-spiro[4.5]dec-3-en-4-yl ester ethyl ester (110 mg, 0.245 mmol) in dioxane (1.5 ml) and water (1.0 ml) at 5°C was added lithium hydroxide hydrate (14.7 mg, 0.612 mmol) in one portion. The reaction mixture was stirred at room temperature overnight and evaporated to dryness *in vacuo.* The residue was dissolved in water (1 ml), the aqueous phase acidified with cooling to pH 4-5 by addition of a 2N HCl solution and the product thoroughly extracted with dichloromethane (4x 2 ml). The combined organic layers were dried over sodium sulfate and concentrated *in vacuo.* Yield: 58 mg of *cis*-3-(2,5-dimethyl-phenyl)-8-methoxy-1-phenyl-1-aza-spiro[4.5]decane-2,4-dione (compound P2.1) as an amorphous white solid. ¹H-NMR (400 MHz, CDCl₃): δ 1.67-1.97 (m, 6H), 2.04-2.20 (m, 2H), 2.30 (s, 3H), 2.35 (s, 3H), 3.15 (s, 3H), 3.40 (m, 1 H), 4.53 (s, 1 H), 6.88 (s, 1 H), 7.03 (d, *J* = 7.7 Hz, 1 H), 7.12 (d, *J* = 7.7 Hz, 1 H), 7.24 (m, 2H), 7.38-7.53 (m, 3H) ppm.
LC/MS (ES+): 378 (M+H)⁺; Rₜ = 1.58 min

### EXAMPLE 2: Preparation of 3-(4-Chloro-2,6-dimethyl-phenyl)-8-methoxy-1-phenyl-1,8-diaza-spiro[4.5]decane-2,4-dione (compound P2.5)

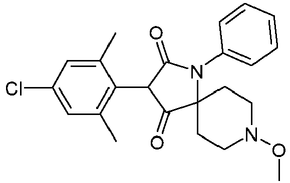

A slurry of 3-(4-chloro-2,6-dimethyl-phenyl)-8-methoxy-1,8-diaza-spiro[4.5]decane-2,4-dione [prepared in analogy to WO 2009/049851] (50 mg, 0.148 mmol), triphenylbismuthane (132 mg, 0.30 mmol), anhydrous copper(II) acetate (54.5 mg, 0.30 mmol) and triethylamine (0.041 ml, 30 mg, 0.30 mmol) in dichloromethane (0.5 ml) was stirred at room temperature for five days. The reaction mixture was diluted with dichloromethane (25 ml), filtered through Hyflo (calcined diatomaceous earth) and the filtrate concentrated *in vacuo.* The residue was purified by chromatography on silica gel (ethyl acetate/cyclohexane 1:3). Yield: 22.5 mg of 3-(4-chloro-2,6-dimethyl-phenyl)-8-methoxy-1-phenyl-1,8-diaza-spiro[4.5]decane-2,4-dione (compound P2.5) as a white solid, mp 225-227°C.
¹H-NMR (400 MHz, CD₃OD): δ 1.84 (br m, 1 H), 2.04 (br m, 1 H), 2.16 (br m, 1 H), 2.22 (s, 6H), 2.36 (br m, 1 H), 3.05 (br m, 1 H), 3.20 (br m, 2H), 3.34-3.50 (br signal, 4H), 7.12 (s, 2H), 7.24 (m, 2H), 7.40-7.53 (m, 3H) ppm.
LC/MS (ES+): 413/415 (M+H)⁺; Rₜ = 1.48 min

The spectral data (¹H-NMR, LC-MS) of this material was identical to the data set of the product obtained by applying the ring closure protocol Example 1/step 4 on substrate 4-{[2-(4-chloro-2,6-dimethyl-phenyl)-acetyl]-phenyl-amino}-1-methoxy-piperidine-4-carboxylic acid methyl ester, where 3-(4-chloro-2,6-dimethyl-phenyl)-8-methoxy-1-phenyl-1,8-diaza-spiro[4.5]decane-2,4-dione (compound P2.5) was formed as a white solid, mp 226-228°C.

### EXAMPLE 3: Preparation of Carbonic acid 3-(4-chloro-2,6-dimethyl-phenyl)-1-(4-fluorophenyl)-8-methoxy-2-oxo-1,8-diaza-spiro[4.5]dec-3-en-4-yl ester ethyl ester (compound P1.7)

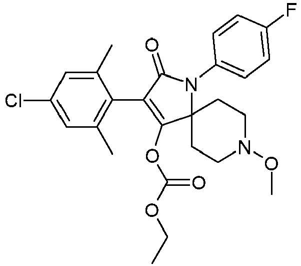

To a solution of carbonic acid 3-(4-chloro-2,6-dimethyl-phenyl)-8-methoxy-2-oxo-1,8-diaza-spiro[4.5]dec-3-en-4-yl ester ethyl ester [prepared in analogy to WO 2009/049851] (1.0 g, 2.45 mmol) and 4A molecular sieves (350 mg) in dichloromethane (15 ml) at room temperature was added (4-fluorophenyl)boronic acid (684 mg, 4.89 mmol), triethylamine (0.68 ml, 496 mg, 4.89 mmol) and anhydrous copper(II) acetate (680 mg, 3.67 mmol). The reaction slurry was stirred at room temperature for 24 hours, filtered through Hyflo (calcined diatomaceous earth) and the filtrate concentrated *in vacuo.* The residue was purified by chromatography on silica gel (ethyl acetate/cyclohexane 1:3). Yield: 270.0 mg of carbonic acid 3-(4-chloro-2,6-dimethyl-phenyl)-1-(4-fluoro-phenyl)-8-methoxy-2-oxo-1,8-diaza-spiro[4.5]dec-3-en-4-yl ester ethyl ester (compound P1.7) as an off-white solid, mp 128-130°C.
¹H-NMR (400 MHz, CDCl₃): δ 1.10 (t, *J*= 7.2 Hz, 3H), 1.82-2.39 (br signal, total 4H), 2.24 (s, 6H), 2.82-3.34 (br signal, total 4H), 3.46 (br s, 3H), 4.04 (q, *J* = 7.2 Hz, 2H), 7.06 (s, 2H), 7.13-7.24 (m, 4H) ppm.
LC/MS (ES+): 503/505 (M+H)⁺; Rₜ = 1.94 min

Compounds of the formula I from Table P1 and compounds of the formula II from Table P2 can be prepared by analogous procedures. The following LC-MS method was used to characterize the compounds:
MS: ZQ Mass Spectrometer from Waters (Single quadrupole mass spectrometer); lonisation method: Electrospray; Polarity: positive and negative ions; Capillary (kV) 3.00, Cone (V) 30.00, Extractor (V) 2.00, Source Temperature (°C) 150, Desolvation Temperature (°C) 350, Cone Gas Flow (L/Hr) 50, Desolvation Gas Flow (L/Hr) 400; Mass range: 100 to 900 Da.

LC: Acquity UPLC from Waters: solvent degasser, binary pump, heated column compartment and diode-array detector. Column: Waters UPLC HSS T3, 1.8 µm, 30 x 2.1 mm, Temp: 60°C; DAD Wavelength range (nm): 210 to 500; Solvent gradient: A = H₂O + 5% MeOH + 0.05% HCOOH, B= Acetonitril + 0.05% HCOOH.

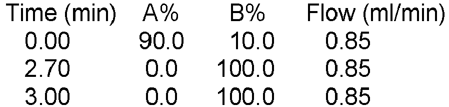

The characteristic values obtained for each compound were the retention time ("Rₜ", recorded in minutes) and the molecular ion as listed in Table P1 and Table P2.

**Table P1: Physical data of compounds of formula I:**

| Compound No. | Structures | Melting Point | MS/NMR |
|---|---|---|---|
| P1.1 | 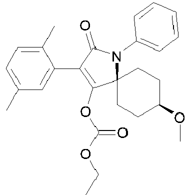 | 114-116°C | LC/MS: 450 (M+H)⁺ Rₜ = 1.91 min |
| P1.2 | 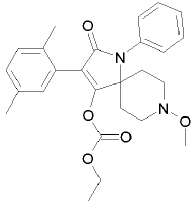 | 144-145°C | LC/MS: 451 (M+H)⁺ Rₜ = 1.83 min |
| P1.3 | 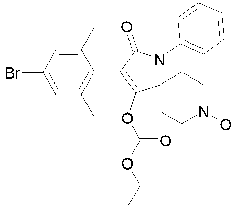 | 158-159°C | LC/MS: 529/531 (M+H)⁺ Rₜ = 1.97 min |
| P1.4 | 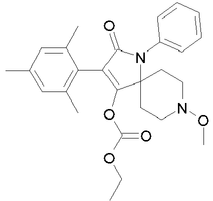 | 122-124°C | LC/MS: 465 (M+H)⁺ Rₜ = 1.91 min |
| P1.5 | 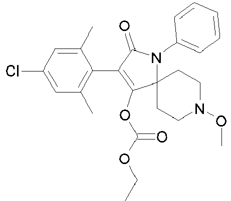 | gum | LC/MS: 485/487 (M+H)⁺ Rₜ = 1.95 min |
| P1.6 | 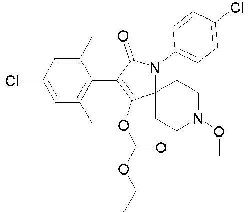 | 150-151°C | LC/MS: 519/521 (M+H)⁺ Rₜ = 2.10 min |
| P1.7 | 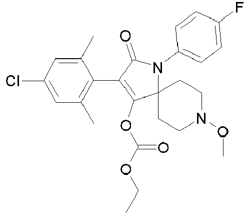 | 128-130°C | LC/MS: 503/505 (M+H)⁺ Rₜ = 1.94 min |

**Table P2: Physical data of compounds of formula II:**

| Compound No. | Structures | Melting Point | MS/NMR |
|---|---|---|---|
| P2.1 | 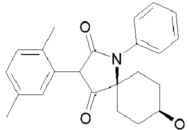 | solid | LC/MS: 378 (M+H)⁺ Rₜ = 1.58 min |
| P2.2 | 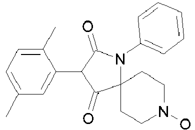 | solid | LC/MS: 379 (M+H)⁺ Rₜ = 1.41 min |
| P2.3 | 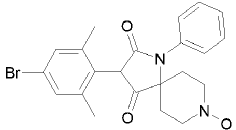 | 190°C (dec) | LC/MS: 457/459 (M+H)⁺ Rₜ = 1.52 min |
| P2.4 | 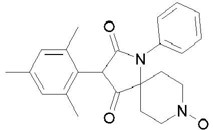 | solid | LC/MS: 393 (M+H)⁺ Rₜ = 1.44 min |
| P2.5 | 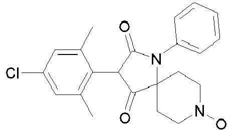 | 226-228°C | LC/MS: 413/415 (M+H)⁺ Rₜ = 1.48 min |
| P2.6 | 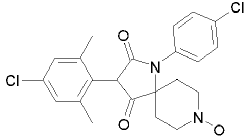 | 212°C (dec) | LC/MS: 447/449 (M+H)⁺ Rₜ = 1.67 min |
| P2.7 | 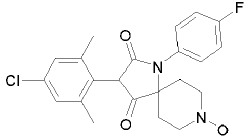 | 232°C (dec) | LC/MS: 431/433 (M+H)⁺ Rₜ = 1.53 min |

### BIOLOGICAL EXAMPLES

These examples illustrate the pesticidal/insecticidal properties of compounds of formula I.

### Example B1: Activity against Myzus persicae (green peach aphid) (mixed population, feeding/residual contact activity, preventive)

Sunflower leaf discs were placed on agar in a 24-well microtiter plate and sprayed with test solutions. After drying, the leaf discs were infested with an aphid population of mixed ages. After an incubation period of 6 days, samples were checked for mortality and special effects (e.g. phytotoxicity).

In this test, compounds listed in the tables above show good activity. In particular compounds P1.3, P1.5, P2.3, P2.4 and P2.5 showed an activity of over 80% at a concentration of 400ppm.

### Example B2: Activity against Myzus persicae (green peach aphid) (mixed population, systemic/feeding activity, curative)

Roots of pea seedlings, infested with an aphid population of mixed ages, were placed directly in the test solutions. 6 days after introduction, samples were checked for mortality and special effects on the plant.

In this test, compounds listed in the tables above showed good activity. In particular compounds P1.2, P1.3, P1.4, P1.5, P2.3, P2.4 and P2.5 showed an activity of over 80% at a concentration of 400ppm.

### Example B3: Activity against Thrips tabaci (onion Thrips)

### (mixed population, feeding/residual contact activity, preventive)

Sunflower leaf discs were placed on agar in a 24-well microtiter plate and sprayed with test solutions. After drying, the leaf discs were infested with a thrips population of mixed ages. After an incubation period of 6 days, samples were checked for mortality and special effects (e.g. phytotoxicity).

### Example B4: Activity against Tetranychus urticae (two-spotted spider mite)

### (mixed population, feeding/residual contact activity, preventive)

Bean leaf discs on agar in 24-well microtiter plates were sprayed with test solutions. After drying, the leaf discs were infested with mite populations of mixed ages. 8 days later, discs were checked for egg mortality, larval mortality, and adult mortality.

In this test, compounds listed in the tables above showed good activity. In particular compounds P1.2, P1.3, P2.3 and P2.4 showed an activity of over 80% at a concentration of 400ppm.

### Example B5: Activity against Myzus persicae (green peach aphid)

### (mixed population, feeding/residual contact activity, plant damage evaluation)

Cabbage plants infested with a mixed population of *Myzus persicae* were treated with diluted test solutions of the compounds in a spray chamber. 6 days after treatment, samples were checked for mortality and for plant damage (phytotoxicity), visual assessment being made using a 0-100% rating scale (100% = total damage to plant; 0% = no damage to plant).

In this test, compounds listed in the tables above showed good activity against *Myzus persicae* and acceptable plant compatibility. For example compounds P1.1, P1.2, P1.3, P1.5, P2.1, P2.2, P2.3, P2.4 and P2.5 showed an activity of greater or equal to 80% against *Myzus persicae* and damage to cabbage plants less or equal to 10% at a concentration of 200ppm.

## Claims

1. Compounds of the formula I 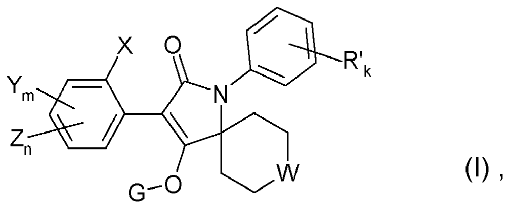 wherein
X, Y and Z independently of each other are selected from C₁₋₄alkyl, C₃₋₆cycloalkyl, C₁₋₄haloalkyl, C₁₋₄alkoxy, C₂₋₆alkenyl, C₂₋₆alkynyl, halogen, phenyl or phenyl substituted by C₁₋₄alkyl, C₁₋₄haloalkyl, halogen or cyano;
m and n, independently of each other, are 0, 1, 2 or 3 and m+n is 0, 1, 2 or 3;
G is hydrogen, a metal, ammonium, sulfonium or a latentiating group;
R' is selected from one or more of C₁₋₄alkyl, C₁₋₄haloalkyl, C₁₋₄alkoxy, halogen or cyano;
k is 0, 1, 2, 3, 4 or 5;
and W is a group selected from W¹ to W¹³: 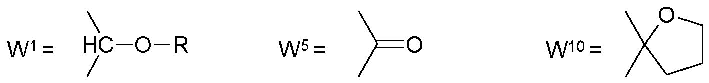 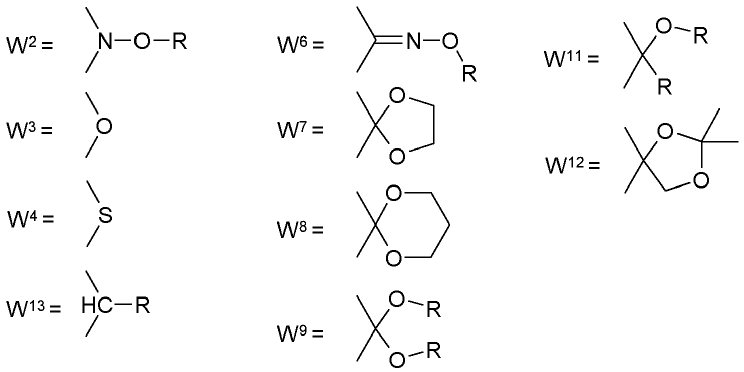 wherein R is hydrogen, C₁₋₆alkyl, C₁₋₆haloalkyl, C₁₋₆cyanoalkyl, C₂₋₆alkenyl, C₂₋₆haloalkenyl, C₃₋₆alkynyl, benzyl, C₁₋₄alkoxy(C₁₋₄)alkyl or C₁₋₄alkoxy(C₁₋₄)alkoxy(C₁₋₄)alkyl;
or an agrochemically acceptable salt or an N-oxide thereof.

2. The compound according to claim 1 wherein W is selected from W² to W¹³, preferably W² to W¹²_{.}

3. The compound according to claim 1 or 2 wherein R is selected from hydrogen, methyl, ethyl, n-propyl, iso-propyl, n-butyl, sec-butyl, tert-butyl, trifluoromethyl, allyl, propargyl, benzyl, methoxymethyl, ethoxymethyl or methoxyethyl.

4. The compound according to any one of claims 1 to 3 wherein k equals 0,1, 2, 3, 4 or 5 and when k equals 1, 2, 3, 4 or 5, then R' is selected from methyl, ethyl, n-propyl, iso-propyl, n-butyl, sec-butyl, iso-butyl, tert-butyl, fluoromethyl, difluoromethyl, difluorochloromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, methoxy, ethoxy, propoxy, i-propoxy, n-butoxy, isobutoxy, sec-butoxy and tert-butoxy, fluorine, chlorine, bromine, iodine or cyano.

5. The compound according to any of claims 1 to 4 wherein
m and n each independently equal 0 or 1;
and X, Y and Z are selected independently of each other from C₁₋₄alkyl, C₃₋₆cycloalkyl, C₁₋₄alkoxy, or halogen.

6. The compound according to any one of claims 1 to 5 wherein X, Y and Z are selected independently of each other from methyl, ethyl, cyclopropyl, methoxy, fluoro, bromo or chloro.

7. A process for the preparation of the compounds of the formula I according to any one of claims 1 to 6, wherein G is hydrogen, which comprises cyclisation of the compound of formula IV under basic conditions 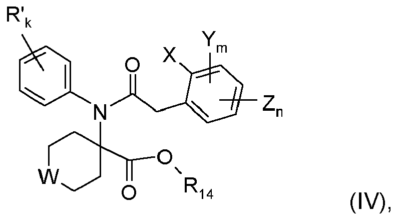 wherein W, X, Y, Z, k, m, n, and R' have the meanings assigned to them according to any one of the previous claims, and R₁₄ is C₁₋₆alkyl.

8. A pesticidal composition comprising a pesticidal effective amount of at least one compound of formula I according to any one of claims 1 to 6, and optionally comprises formulation adjuvants.

9. A pesticidal composition according to claim 8, which, in addition to comprising the compound of formula I, comprises at least one additional insecticide, acaricide, nemacitide or molluscicide.

10. A pesticidal composition according to any one of claims 8 to 9, which, in addition to comprising the compound of formula I, comprises at least one additional fungicide, herbicide, safener or plant growth regulator.

11. A method of combating and controlling pests which comprises applying to a pest, to a locus of a pest, or to a plant susceptible to attack by a pest a pesticidally effective amount of a compound of formula I according to any one of claims 1 to 6 or a pesticidal composition according to any one of claims 8 to 10.

12. Compounds of the formula IV* 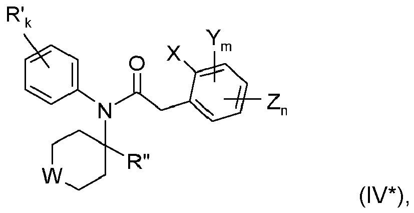 or salts thereof, wherein W, X, Y, Z, k, m, n, and R' have the meanings assigned to them according to any one of claims 1 to 6, and R" is either-C≡N or-C(O)OR₁₄, wherein R₁₄ is C₁₋₆alkyl.

13. Compounds of the formula V* 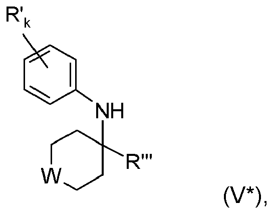 or salts thereof, wherein W, R' and k have the meanings assigned to them according to any one of claims 1 to 6, and R'" is either-C≡N, -C(O)OH or-C(O)OR₁₄, wherein R₁₄ is C₁₋₆alkyl.
